# EUROPÄISCHE PATENTANMELDUNG

(11) **EP 0 926 237 A2**
(43) Veröffentlichungstag der Anmeldung: **30.06.1999**
(21) Anmeldenummer: 98123709.2
(22) Anmeldetag: 12.12.1998
(51) Int. Cl.: C12N 15/12, A61K 48/00

(54) **Nukleinsäurekonstrukte für die Gentherapie, deren Aktivität von Inhibitoren cyclinabhängiger Kinasen beeinflusst wird**

(30) Priorität: 20.12.1997 DE 19756975
(71) Anmelder: Hoechst Marion Roussel Deutschland GmbH, 65929 Frankfurt am Main (DE)
(72) Erfinder: Eilers, Martin Prof.Dr., 35043 Marburg (DE); Buergin, Andrea, 35037 Marburg (DE); Sedlacek, Hans-Harald Prof.Dr., 35041 Marburg (DE)

(57) **Zusammenfassung**

Es werden Nukleinsäurekonstrukte für die Gentherapie von Erkrankungen offenbart, enthaltend Nukleinsäuren kodierend für ein Protein, welches das zelluläre Protein p27 inhibiert und hierdurch die durch p27 bewirkte Hemmung der Proliferation der Zelle aufhebt, Mutanten davon mit z.T. dominant interferierendem Charakter und die Verwendung von diesen Nukleinsäuren für die Prophylaxe und Therapie von Erkrankungen.

SEQUENZPROTOKOLL

(1) ALLGEMEINE INFORMATION:

(i) ANMELDER:
(A) NAME: Hoechst Marion Roussel Deutschland GmbH
(B) STRASSE: -
(C) ORT: Frankfurt
(D) BUNDESLAND: -
(E) LAND: Germany
(F) POSTLEITZAHL: 65926
(G) TELEPHON: 069-305-3005
(H) TELEFAX: 069-35-7175
(I) TELEX: -

(ii) ANMELDETITEL: Bindungspartner fuer Inhibitoren von
cyclinabhaengigen Kinasen und ihre Verwendung zur Suche
nach Inhibitoren, zur Diagnose oder zur Therapie einer
Erkrankung

(iii) ANZAHL DER SEQUENZEN: 57

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..17

(xi) SEQUENZBESCHREIBUNG: SEQ ID NO: 1:
CGGACAACTG TTGACCG 17

(2) INFORMATION ZU SEQ ID NO: 2:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 2:

TACTGTATGT ACATACAGTA 20

(2) INFORMATION ZU SEQ ID NO: 3:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 3:

GAATTGTGAG CGCTCACAAT TC 22

(2) INFORMATION ZU SEQ ID NO: 4:

(i) SEQUENZ CHARAKTERISTIKA:

(A) LÄNGE: 42 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..42

(xi) SEQUENZBESCHREIBUNG: SEQ ID NO: 4:

TCGAGTTTAC CACTCCCTAT CAGTGATAGA GAAAAGTGAA AG 42

(2) INFORMATION ZU SEQ ID NO: 5:

(i) SEQUENZ CHARAKTERISTIKA:
(A) LÄNGE: 12 Basenpaare
(B) ART: Nukleinsäure

(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..12

(xi) SEQUENZBESCHREIBUNG: SEQ ID NO: 5:

TAATGATGGG CG 12

(2) INFORMATION ZU SEQ ID NO: 6:

(i) SEQUENZ CHARAKTERISTIKA:
(A) LÄNGE: 26 Basenpaare
(B) ART: Nukleinsäure
(C) STRANGFORM: Einzel
(D) TOPOLOGIE: linear
(ii) ART DES MOLEKÜLS: DNS (genomisch)

(ix) MERKMALE:
(A) NAME/SCHLÜSSEL: exon
(B) LAGE: 1..26

(xi) SEQUENZBESCHREIBUNG: SEQ ID NO: 6:

GGAAGCAGAC CACGTGGTCT GCTTCC 26

(2) INFORMATION ZU SEQ ID NO: 7:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..17

(xi) SEQUENZBESCHREIBUNG: SEQ ID NO: 7:

AGCAGGTGTT GGGAGGC 17

(2) INFORMATION ZU SEQ ID NO: 8:

(i) SEQUENZ CHARAKTERISTIKA:
(A) LÄNGE: 41 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..41

(xi) SEQUENZBESCHREIBUNG: SEQ ID NO: 8:

GGCCGATGGG CAGATAGAGG GGGCCGATGG GCAGATAGAG G 41

(2) INFORMATION ZU SEQ ID NO: 9:

(i) SEQUENZ CHARAKTERISTIKA:
(A) LÄNGE: 1958 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..1958

(xi) SEQUENZBESCHREIBUNG: SEQ ID NO: 9:

TCGAATTGGG TACCGGGCCC CCCCTCGAGG TCGACGGTAT CGATAAGCTT GATATCGAAT 60

TCGCGGCCGC TCGAGTTACA AGATGGCGGC CCCGGGCGCT CTCTTCACCG TTCTGTAGCA 120

GCTTCGGGCT GAGCGGATGT CTCTTCTTGT CCTCAGTGTC GGACTCAGAG ACACACGGCT 180

CCCGAGTTCT GCTGATCACG AAGTTCCCGG AGGCGCTCGA CGCACCGGAA TCTCCCAGCG 240

GCCGCGACCG CCGCCTCGGC CCTGCTCGCC GCGGCGCCGG GACTCCAGCG TGATCGGCGG 300

CGGCAGTCAA GGTTCACAAA AATGGCGAAG AGAGTTGCGG AGAAGGAGTT GACTGACAGG 360

AACTGGGATG AGGAAGACGA AGTTGAAGAG ATGGGAACAT TCTCAGTGGC CAGTGAGGAA 420

GTCATGAAGA ACAGAGCCGT AAAGAAGGCA AAGCGCAGAA ACGTTGGATT TGAATCTGAT 480

AGCGGAGGAG CCTTTAAAGG TTTCAAAGGT TTGGTTGTGC CTTCTGGAGG AGGAGGGTTT 540

TCTGGATTTG GTGGCTCTGG AGGAAAGCCT CTGGAAGGAC TGACAAATGG AAACAGCACA 600

GACAATGCCA CGCCCTTCTC CAATGTAAAG ACAGCAGCAG AGCCCAAGGC AGCCTTTGGT 660

TCTTTTGCTG TGAATGGCCC TACTACCTTG GTGGATAAAG TTTCAAATCC AAAAACTAAT 720

GGGGACAGCA ATCAGCCGCC CTCCTCCGGC CCTGCTTCCA GTACCGCCTG CCCTGGGAAT 780

GCCTATCACA AGCAGCTGGC TGGCTTGAAC TGCTCCGTCC GCGATTGGAT AGTGAAGCAC 840

GTGAACACAA ACCCGCTTTG TGACCTGACT CCCATTTTTA AAGACTATGA GAGATACTTG 900

GCGACGATCG AGAAGCAGCT TGAGAATGGA GGCGGCAGCA GTTCTGAGAG CCAGACAGAC 960

AGGGCGACGG CTGGAATGGA GCCTCCTTCC CTTTTTGGTT CAACAAAACT ACAGCAAGAG 1020

TCACCATTTT CATTTCATGG CAACAAAGCG GAGGACACAT CTGAAAAGGT GGAGTTTACA 1080

GCAGAAAAGA AATCGGACGC AGCACAAGGA GCAACAAGTG CCTCGTTTAG TTTCGGCAAG 1140

AAAATTGAGA GCTCGGCTTT GGGCTCGTTA AGCTCTGGCT CCCTAACTGG GTTTTCATTC 1200

TCTGCTGGAA GCTCCAGCTT GTTTGGTAAA GATGCTGCCC AGAGTAAAGC AGCCTCTTCG 1260

CTGTTCTCTG CTAAAGCATC CGAGAGTCCG GCAGGAGGCG GCAGCAGCGA GTGCAGAGAT 1320

GGTGAAGAAG AGGAGAATGA CGAGCCACCC AAGGTAGTGG TGACCGAAGT AAAGGAAGAG 1380

GATGCTTTCT ACTCCAAAAA ATGTAAACTA TTTTACAAGA AAGACAACGA ATTTAAAGAG 1440

AAGGGTGTGG GGACCCTGCA TTTAAAACCC ACAGCAACTC AGAAGACCCA GCTCTTGGTG 1500

CGGGCAGACA CCAACCTAGG CAACATACTG CTGAATGTTC TGATCGCCCC CAACATGCCG 1560

TGCACCCGGA CAGGAAAGAA CAACGTCCTT ATCGTCTGTG TCCCCAACCC CCCACTCGAT 1620

GAGAAGCAGC CCACTCTCCC GGCCACCATG CTGATCCGGG TGAAGACGAG CGAGGATGCC 1680

GATGAATTGC ACAAGATTTT ACTGGAGAAA AAGGATGCCT GAGCACTGAG GCTGACCAAG 1740

GCATGTTGCC ACGTTGCTGC TTCCCCTCCG TCCCTAACTT AGTCACATTC TTTCCTCTTC 1800

TACTGTGACA TTCTGAGAAC TTCTAGGTAA CTTGAACTTT TGTGAGGAAG ATTAAGGCCA 1860

ATAAATCCTT TCAGTGGCGG CCGCGAATTC CTGCAGCCCG GGGGATCCAC TAGTTCTAGA 1920

GCGGCCGCCA CCGCGGTGGA GCTCCAGCTT TTGGGAGA 1958

(2) INFORMATION ZU SEQ ID NO: 10:

(i) SEQUENZ CHARAKTERISTIKA:
(A) LÄNGE: 24 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..24

(xi) SEQUENZBESCHREIBUNG: SEQ ID NO: 10:

Met Ala Lys Arg Val Ala Glu Lys Glu Leu Thr Asp Arg Asn Trp Asp
1               5                   10                  15
Glu Glu Asp Glu Val Glu Glu Met
            20

(2) INFORMATION ZU SEQ ID NO: 11:

(i) SEQUENZ CHARAKTERISTIKA:
(A) LÄNGE: 24 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..24

(xi) SEQUENZBESCHREIBUNG: SEQ ID NO: 11:

Met Ala Lys Arg Val Ala Asp Ala Gln Ile Gln Arg Glu Thr Tyr Asp
1               5                   10                  15

Ser Asn Glu Ser Asp Asp Asp Val
            20

(2) INFORMATION ZU SEQ ID NO: 12:

(i) SEQUENZ CHARAKTERISTIKA:
(A) LÄNGE: 60 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..60

(xi) SEQUENZBESCHREIBUNG: SEQ ID NO: 12:

Asn Gln Pro Pro Ser Ser Gly Pro Ala Ser Ser Thr Ala Cys Pro Gly
1               5                   10                  15

Asn Ala Tyr His Lys Gln Leu Ala Gly Leu Asn Cys Ser Val Arg Asp
            20                  25                  30

Trp Ile Val Lys His Val Asn Thr Asn Pro Leu Cys Asp Leu Thr Pro
        35                  40                  45

Ile Phe Lys Asp Tyr Glu Arg Tyr Leu Ala Thr Ile
    50                  55                  60

(2) INFORMATION ZU SEQ ID NO: 13:

(i) SEQUENZ CHARAKTERISTIKA:
(A) LÄNGE: 60 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..60

(xi) SEQUENZBESCHREIBUNG: SEQ ID NO: 13:

Asn Arg Ala Asp Gly Thr Gly Glu Ala Gln Val Asp Asn Ser Pro Thr
1               5                   10                  15

Thr Glu Ser Asn Ser Arg Leu Lys Ala Leu Asn Leu Gln Phe Lys Ala
            20                  25                  30

Lys Val Asp Asp Leu Val Leu Gly Lys Pro Leu Ala Asp Leu Arg Pro
        35                  40                  45

Leu Phe Thr Arg Tyr Glu Leu Tyr Ile Lys Asn Ile
    50                  55                  60

(2) INFORMATION ZU SEQ ID NO: 14:

(i) SEQUENZ CHARAKTERISTIKA:
(A) LÄNGE: 51 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..51

(xi) SEQUENZBESCHREIBUNG: SEQ ID NO: 14:

Ala Thr Ala Gly Met Glu Pro Pro Ser Leu Phe Gly Ser Thr Lys Leu
1               5                   10                  15

Gln Gln Glu Ser Pro Phe Ser Phe His Gly Asn Lys Ala Glu Asp Thr
            20                  25                  30

Ser Glu Lys Val Glu Phe Thr Ala Glu Lys Lys Ser Asp Ala Ala Gln
        35                  40                  45

Gly Ala Thr
    50

(2) INFORMATION ZU SEQ ID NO: 15:

(i) SEQUENZ CHARAKTERISTIKA:
(A) LÄNGE: 51 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..51

(xi) SEQUENZBESCHREIBUNG: SEQ ID NO: 15:

Ala Asn Ser Ser Thr Ser Pro Ala Pro Ser Ile Pro Ser Thr Gly Phe
1               5                   10                  15

Lys Phe Ser Leu Pro Phe Glu Gln Lys Gly Ser Gln Thr Thr Thr Asn
            20                  25                  30
Asp Ser Lys Glu Glu Ser Thr Thr Glu Ala Thr Gly Asn Glu Ser Gln
        35                  40                  45

Asp Ala Thr
    50

(2) INFORMATION ZU SEQ ID NO: 16:

(i) SEQUENZ CHARAKTERISTIKA:
(A) LÄNGE: 34 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..34

(xi) SEQUENZBESCHREIBUNG: SEQ ID NO: 16:

His Gly Asn Lys Ala Glu Asp Thr Ser Glu Lys Val Glu Phe Thr Ala
1               5                   10                  15

Glu Lys Lys Ser Asp Ala Ala Gln Gly Ala Thr Ser Ala Ser Phe Ser
            20                  25                  30

Phe Gly

(2) INFORMATION ZU SEQ ID NO: 17:

(i) SEQUENZ CHARAKTERISTIKA:
(A) LÄNGE: 34 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..34

(xi) SEQUENZBESCHREIBUNG: SEQ ID NO: 17:

Asn Gly Ser Glu Ser Lys Asp Ser Asp Lys Pro Ser Leu Pro Ser Ala
1               5                   10                  15

Val Asp Gly Glu Asn Asp Lys Lys Glu Ala Thr Lys Pro Ala Phe Ser
            20                  25                  30

Phe Gly

(2) INFORMATION ZU SEQ ID NO: 18:

(i) SEQUENZ CHARAKTERISTIKA:
(A) LÄNGE: 40 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..40

(xi) SEQUENZBESCHREIBUNG: SEQ ID NO: 18:

Leu Gly Asn Ile Leu Leu Asn Val Leu Ile Ala Pro Asn Met Pro Cys
1               5                   10                  15

Thr Arg Thr Gly Lys Asn Asn Val Leu Ile Val Cys Val Pro Asn Pro
            20                  25                  30

Pro Leu Asp Glu Lys Gln Pro Thr
        35                  40

(2) INFORMATION ZU SEQ ID NO: 19:

    (i) SEQUENZ CHARAKTERISTIKA:
        (A) LÄNGE: 40 Aminosäuren
        (B) ART: Aminosäure
        (C) STRANGFORM: Einzel
        (D) TOPOLOGIE: linear

    (ii) ART DES MOLEKÜLS: Protein

    (ix) MERKMALE:
        (A) NAME/SCHLÜSSEL: Protein
        (B) LAGE: 1..40

    (xi) SEQUENZBESCHREIBUNG: SEQ ID NO: 19:

Met Gly Asn Val Leu Leu Asn Ala Thr Val Val Asp Ser Phe Lys Tyr
1               5                   10                  15

Glu Pro Leu Ala Pro Gly Asn Asp Asn Leu Ile Lys Ala Pro Thr Val
            20                  25                  30

Ala Ala Asp Gly Lys Leu Val Thr
        35                  40

(2) INFORMATION ZU SEQ ID NO: 20:

    (i) SEQUENZ CHARAKTERISTIKA:
        (A) LÄNGE: 157 Aminosäuren
        (B) ART: Aminosäure
        (C) STRANGFORM: Einzel
        (D) TOPOLOGIE: linear

    (ii) ART DES MOLEKÜLS: Protein

    (ix) MERKMALE:
        (A) NAME/SCHLÜSSEL: Protein
        (B) LAGE: 1..157

    (xi) SEQUENZBESCHREIBUNG: SEQ ID NO: 20:

Asp Ser His Ala Asp His Asp Thr Ser Thr Glu Asn Ala Asp Glu Ser
1               5                   10                  15

Thr Thr His Pro Gln Phe Glu Pro Ile Val Ser Val Pro Glu Gln Glu
            20                  25                  30

Ile Lys Thr Leu Glu Glu Asp Glu Glu Glu Leu Phe Lys Met Arg Ala
        35                  40                  45

Lys Leu Phe Arg Phe Ala Ser Glu Asn Asp Leu Pro Glu Trp Lys Glu
    50                  55                  60

Pro Arg His Gly Asp Val Lys Leu Leu Lys His Lys Glu Lys Gly Thr
65                  70                  75                  80

Ile Arg Leu Leu Met Arg Arg Asp Lys Thr Leu Lys Ile Cys Ala Asn
                85                  90                  95

His Tyr Ile Thr Pro Met Met Glu Leu Lys Pro Asn Ala Gly Ser Asp
            100                 105                 110

Arg Ala Trp Val Trp Asn Thr His Thr Asp Phe Ala Asp Glu Cys Pro
        115                 120                 125

Lys Pro Glu Leu Leu Ala Ile Arg Phe Leu Asn Ala Glu Asn Ala Gln
    130                 135                 140

Lys Phe Lys Thr Lys Phe Glu Glu Cys Arg Lys Glu Ile
145                 150                 155

(2) INFORMATION ZU SEQ ID NO: 21:

     (i) SEQUENZ CHARAKTERISTIKA:
          (A) LÄNGE: 157 Aminosäuren
          (B) ART: Aminosäure
          (C) STRANGFORM: Einzel
          (D) TOPOLOGIE: linear

    (ii) ART DES MOLEKÜLS: Protein

    (ix) MERKMALE:
          (A) NAME/SCHLÜSSEL: Protein
          (B) LAGE: 1..157

    (xi) SEQUENZBESCHREIBUNG: SEQ ID NO: 21:

Asp Ser His Ala Asp His Asp Thr Ser Thr Glu Asn Ala Asp Glu Ser
1               5                   10                  15

Asn His Asp Pro Gln Phe Glu Pro Ile Val Ser Val Pro Glu Gln Glu
            20                  25                  30

Ile Lys Thr Leu Glu Glu Asp Glu Glu Glu Leu Phe Lys Met Arg Ala
        35                  40                  45

Lys Leu Phe Arg Phe Ala Ser Glu Asn Asp Leu Pro Glu Trp Lys Glu
    50                  55                  60

Arg Gly Thr Gly Asp Val Lys Leu Leu Lys His Lys Glu Lys Gly Thr
65                  70                  75                  80

Ile Arg Leu Leu Met Arg Arg Asp Lys Thr Leu Lys Ile Cys Ala Asn
                85                  90                  95

His Tyr Ile Thr Pro Met Met Glu Leu Lys Pro Asn Ala Gly Ser Asp
            100                 105                 110

Arg Ala Trp Val Trp Asn Thr His Thr Asp Phe Ala Asp Glu Cys Pro
        115                 120                 125

Lys Pro Glu Leu Leu Ala Ile Arg Phe Leu Asn Ala Glu Asn Ala Gln
    130                 135                 140

Lys Phe Lys Thr Lys Phe Glu Glu Cys Arg Lys Glu Ile
145                 150                 155

(2) INFORMATION ZU SEQ ID NO: 22:

     (i) SEQUENZ CHARAKTERISTIKA:
          (A) LÄNGE: 158 Aminosäuren
          (B) ART: Aminosäure
          (C) STRANGFORM: Einzel
          (D) TOPOLOGIE: linear

    (ii) ART DES MOLEKÜLS: Protein

    (ix) MERKMALE:
          (A) NAME/SCHLÜSSEL: Protein
          (B) LAGE: 1..158

    (xi) SEQUENZBESCHREIBUNG: SEQ ID NO: 22:

Ser Gln Thr Thr Thr Asn Asp Ser Lys Glu Glu Ser Thr Thr Glu Ala
1               5                   10                  15

Thr Gly Asn Glu Ser Gln Asp Ala Thr Lys Val Asp Ala Thr Pro Glu
            20                  25                  30

Glu Ser Lys Pro Ile Asn Leu Gln Asn Gly Glu Glu Asp Glu Val Ala
        35                  40                  45

Leu Phe Ser Gln Lys Ala Lys Leu Met Thr Phe Asn Ala Glu Thr Lys
    50                  55                  60

Ser Tyr Asp Ser Arg Gly Val Gly Glu Met Lys Leu Leu Lys Lys Lys
65                  70                  75                  80

Asp Asp Ser Pro Lys Val Arg Leu Leu Cys Arg Ser Asp Gly Met Gly
                85                  90                  95

Asn Val Leu Leu Asn Ala Thr Val Val Asp Ser Phe Lys Tyr Glu Pro
            100                 105                 110

Leu Ala Pro Gly Asn Asp Asn Leu Ile Lys Ala Pro Thr Val Ala Ala
        115                 120                 125

Asp Gly Lys Leu Val Thr Tyr Ile Val Phe Lys Gln Lys Leu Glu Gly
    130                 135                 140

Arg Ser Phe Thr Lys Ala Ile Glu Asp Ala Lys Lys Glu Met
145                 150                 155

(2) INFORMATION ZU SEQ ID NO: 23:

(i) SEQUENZ CHARAKTERISTIKA:
(A) LÄNGE: 160 Aminosäuren
(B) ART: Aminosäure
(C) STRANGFORM: Einzel
(D) TOPOLOGIE: linear

(ii) ART DES MOLEKÜLS: Protein

(ix) Merkmale:
(A) NAME/SCHLÜSSEL: Protein
(B) LAGE: 1..160

(xi) SEQUENZBESCHREIBUNG: SEQ ID NO: 23:

Gln Ser Lys Ala Ala Ser Ser Leu Phe Ser Ala Lys Ala Ser Glu Ser
1               5                   10                  15

Pro Ala Gly Gly Gly Ser Ser Glu Cys Arg Asp Gly Glu Glu Glu Glu
            20                  25                  30

Asn Asp Glu Pro Pro Lys Val Val Val Thr Glu Val Lys Glu Glu Asp
        35                  40                  45

Ala Phe Tyr Ser Lys Lys Cys Lys Leu Phe Tyr Lys Lys Asp Asn Glu
    50                  55                  60

Phe Lys Glu Lys Gly Val Gly Thr Leu His Leu Lys Pro Thr Ala Thr
65                  70                  75                  80

Gln Lys Thr Gln Leu Leu Val Arg Ala Asp Thr Asn Leu Gly Asn Ile
                85                  90                  95

Leu Leu Asn Val Leu Ile Ala Pro Asn Met Pro Cys Thr Arg Thr Gly
            100                 105                 110

Lys Asn Asn Val Leu Ile Val Cys Val Pro Asn Pro Leu Asp Glu Lys
        115                 120                 125

Gln Pro Thr Leu Pro Ala Thr Met Leu Ile Arg Val Lys Thr Ser Glu
    130                 135                 140

Asp Ala Asp Glu Leu His Lys Ile Leu Leu Glu Lys Lys Asp Ala Ala
145                 150                 155                 160

(2) INFORMATION ZU SEQ ID NO: 24:

(i) SEQUENZ CHARAKTERISTIKA:
(A) LÄNGE: 466 Aminosäuren

(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..466

(xi) SEQUENZBESCHREIBUNG: SEQ ID NO: 24:

Met Ala Lys Arg Val Ala Glu Lys Glu Leu Thr Asp Arg Asn Trp Asp
1               5                   10                  15

Glu Glu Asp Glu Val Glu Glu Met Gly Thr Phe Ser Val Ala Ser Glu
            20                  25                  30

Glu Val Met Lys Asn Arg Ala Val Lys Lys Ala Lys Arg Arg Asn Val
        35                  40                  45

Gly Phe Glu Ser Asp Ser Gly Gly Ala Phe Lys Gly Phe Lys Gly Leu
    50                  55                  60

Val Val Pro Ser Gly Gly Gly Gly Phe Ser Gly Phe Gly Gly Ser Gly
65                  70                  75                  80

Gly Lys Pro Leu Glu Gly Leu Thr Asn Gly Asn Ser Thr Asp Asn Ala
                85                  90                  95

Thr Pro Phe Ser Asn Val Lys Thr Ala Ala Glu Pro Lys Ala Ala Phe
            100                 105                 110

Gly Ser Phe Ala Val Asn Gly Pro Thr Thr Leu Val Asp Lys Val Ser
        115                 120                 125

Asn Pro Lys Thr Asn Gly Asp Ser Asn Gln Pro Pro Ser Ser Gly Pro
    130                 135                 140

Ala Ser Ser Thr Ala Cys Pro Gly Asn Ala Tyr His Lys Gln Leu Ala
145                 150                 155                 160

Gly Leu Asn Cys Ser Val Arg Asp Trp Ile Val Lys His Val Asn Thr
                165                 170                 175

Asn Pro Leu Cys Asp Leu Thr Pro Ile Phe Lys Asp Tyr Glu Arg Tyr
            180                 185                 190

Leu Ala Thr Ile Glu Lys Gln Leu Glu Asn Gly Gly Gly Ser Ser Ser
        195                 200                 205

Glu Ser Gln Thr Asp Arg Ala Thr Ala Gly Met Glu Pro Pro Ser Leu
    210                 215                 220

Phe Gly Ser Thr Lys Leu Gln Gln Glu Ser Pro Phe Ser Phe His Gly
225                 230                 235                 240

Asn Lys Ala Glu Asp Thr Ser Glu Lys Val Glu Phe Thr Ala Glu Lys
                245                 250                 255

Lys Ser Asp Ala Ala Gln Gly Ala Thr Ser Ala Ser Phe Ser Phe Gly
            260                 265                 270

Lys Lys Ile Glu Ser Ser Ala Leu Gly Ser Leu Ser Ser Gly Ser Leu
        275                 280                 285

Thr Gly Phe Ser Phe Ser Ala Gly Ser Ser Ser Leu Phe Gly Lys Asp
    290                 295                 300

Ala Ala Gln Ser Lys Ala Ala Ser Ser Leu Phe Ser Ala Lys Ala Ser
305                 310                 315                 320

Glu Ser Pro Ala Gly Gly Gly Ser Ser Glu Cys Arg Asp Gly Glu Glu
                325                 330                 335

Glu Glu Asn Asp Glu Pro Pro Lys Val Val Val Thr Glu Val Lys Glu
            340                 345                 350

Glu Asp Ala Phe Tyr Ser Lys Lys Cys Lys Leu Phe Tyr Lys Lys Asp
        355                 360                 365

Asn Glu Phe Lys Glu Lys Gly Val Gly Thr Leu His Leu Lys Pro Thr
    370                 375                 380

Ala Thr Gln Lys Thr Gln Leu Leu Val Arg Ala Asp Thr Asn Leu Gly
385                 390                 395                 400

Asn Ile Leu Leu Asn Val Leu Ile Ala Pro Asn Met Pro Cys Thr Arg
                405                 410                 415

Thr Gly Lys Asn Asn Val Leu Ile Val Cys Val Pro Asn Pro Pro Leu
            420                 425                 430

Asp Glu Lys Gln Pro Thr Leu Pro Ala Thr Met Leu Ile Arg Val Lys
        435                 440                 445

Thr Ser Glu Asp Ala Asp Glu Leu His Lys Ile Leu Leu Glu Lys Lys
    450                 455                 460

Asp Ala
465

(2) INFORMATION ZU SEQ ID NO: 25:

     (i) SEQUENZ CHARAKTERISTIKA:
          (A) LÄNGE: 715 Aminosäuren
          (B) ART: Aminosäure
          (C) STRANGFORM: Einzel
          (D) TOPOLOGIE: linear

    (ii) ART DES MOLEKÜLS: Protein

    (ix) MERKMALE:
          (A) NAME/SCHLÜSSEL: Protein
          (B) LAGE: 1..715

(xi) SEQUENZBESCHREIBUNG: SEQ ID NO: 25:

Met Ala Lys Arg Val Ala Asp Ala Gln Ile Gln Arg Glu Thr Tyr Asp
1               5                   10                  15

Ser Asn Glu Ser Asp Asp Asp Val Thr Pro Ser Thr Lys Val Ala Ser
            20                  25                  30

Ser Ala Val Met Asn Arg Arg Lys Ile Ala Met Pro Lys Arg Arg Met
        35                  40                  45

Ala Phe Lys Pro Phe Gly Ser Ala Lys Ser Asp Glu Thr Lys Gln Ala
    50                  55                  60

Ser Ser Phe Ser Phe Leu Asn Arg Ala Asp Gly Thr Gly Glu Ala Gln
65                  70                  75                  80

Val Asp Asn Ser Pro Thr Thr Glu Ser Asn Ser Arg Leu Lys Ala Leu
                85                  90                  95

Asn Leu Gln Phe Lys Ala Lys Val Asp Asp Leu Val Leu Gly Lys Pro
            100                 105                 110

Leu Ala Asp Leu Arg Pro Leu Phe Thr Arg Tyr Glu Leu Tyr Ile Lys
        115                 120                 125

Asn Ile Leu Glu Ala Pro Val Lys Phe Ile Glu Asn Pro Thr Gln Thr
    130                 135                 140

Lys Gly Asn Asp Ala Lys Pro Ala Lys Val Glu Asp Val Gln Lys Ser
145                 150                 155                 160

Ser Asp Ser Ser Ser Glu Asp Glu Val Lys Val Glu Gly Pro Lys Phe
                165                 170                 175

Thr Ile Asp Ala Lys Pro Pro Ile Ser Asp Ser Val Phe Ser Phe Gly
            180                 185                 190

Pro Lys Lys Glu Asn Arg Lys Lys Asp Glu Ser Asp Ser Glu Asn Asp
        195                 200                 205

Ile Glu Ile Lys Gly Pro Glu Phe Lys Phe Ser Gly Thr Val Ser Ser
    210                 215                 220

Asp Val Phe Lys Leu Asn Pro Ser Thr Asp Lys Asn Glu Lys Lys Thr
225                 230                 235                 240

Glu Thr Asn Ala Lys Pro Phe Ser Phe Ser Ser Ala Thr Ser Thr Thr
                245                 250                 255

Glu Gln Thr Lys Ser Lys Asn Pro Leu Ser Leu Thr Glu Ala Thr Lys
            260                 265                 270

Thr Asn Val Asp Asn Asn Ser Lys Ala Glu Ala Ser Phe Thr Phe Gly
        275                 280                 285

Thr Lys His Ala Ala Asp Ser Gln Asn Asn Lys Pro Ser Phe Val Phe
    290                 295                 300

Gly Gln Ala Ala Ala Lys Pro Ser Leu Glu Lys Ser Ser Phe Thr Phe
305                 310                 315                 320

Gly Ser Thr Thr Ile Glu Lys Lys Asn Asp Glu Asn Ser Thr Ser Asn
                325                 330                 335

Ser Lys Pro Glu Lys Ser Ser Asp Ser Asn Asp Ser Asn Pro Ser Phe
            340                 345                 350

Ser Phe Ser Ile Pro Ser Lys Asn Thr Pro Asp Ala Ser Lys Pro Ser
        355                 360                 365

Phe Asn Phe Gly Val Pro Asn Ser Ser Lys Asn Glu Thr Ser Lys Pro
    370                 375                 380

Val Phe Ser Phe Gly Ala Ala Thr Pro Ser Ala Lys Glu Ala Ser Gln
385                 390                 395                 400

Glu Asp Asp Asn Asn Asn Val Glu Lys Pro Ser Ser Lys Pro Ala Phe
                405                 410                 415

Asn Phe Ile Ser Asn Ala Gly Thr Glu Lys Glu Lys Glu Ser Lys Lys
            420                 425                 430

Asp Ser Lys Pro Ala Phe Ser Phe Gly Ile Ser Asn Gly Ser Glu Ser
        435                 440                 445

Lys Asp Ser Asp Lys Pro Ser Leu Pro Ser Ala Val Asp Gly Glu Asn
    450                 455                 460

Asp Lys Lys Glu Ala Thr Lys Pro Ala Phe Phe Gly Ile Asn Thr Asn
465                 470                 475                 480

Thr Thr Lys Thr Ala Asp Thr Lys Ala Pro Thr Phe Thr Phe Gly Ser
                485                 490                 495

Ser Ala Leu Ala Asp Asn Lys Glu Asp Val Lys Lys Pro Phe Ser Phe
            500                 505                 510

Gly Thr Ser Gln Pro Asn Asn Thr Pro Ser Phe Ser Phe Gly Lys Thr
        515                 520                 525

Thr Ala Asn Leu Pro Ala Asn Ser Ser Thr Ser Pro Ala Pro Ser Ile
    530                 535                 540

Pro Ser Thr Gly Phe Lys Phe Ser Leu Pro Phe Glu Gln Lys Gly Ser
545                 550                 555                 560

Gln Thr Thr Thr Asn Asp Ser Lys Glu Glu Ser Thr Thr Glu Ala Thr
                565                 570                 575

Gly Asn Glu Ser Gln Asp Ala Thr Lys Val Asp Ala Thr Pro Glu Glu
            580                 585                 590

Ser Lys Pro Ile Asn Leu Gln Asn Gly Glu Glu Asp Glu Val Ala Leu
        595                 600                 605

Phe Ser Lys Ala Lys Leu Met Thr Phe Asn Ala Glu Thr Lys Ser Tyr
    610                 615                 620

Asp Ser Arg Gly Val Gly Glu Met Lys Leu Leu Lys Lys Lys Asp Asp
625                 630                 635                 640

Pro Ser Lys Val Arg Leu Leu Cys Arg Ser Asp Gly Met Gly Asn Val
                645                 650                 655

Leu Leu Asn Ala Thr Val Val Asp Ser Phe Lys Tyr Glu Pro Leu Ala
            660                 665                 670

Pro Gly Asn Asp Asn Leu Ile Lys Ala Pro Thr Val Ala Ala Asp Gly
        675                 680                 685

Lys Thr Tyr Ile Val Lys Phe Lys Gln Lys Glu Glu Gly Arg Ser Phe
    690                 695                 700

Thr Lys Ala Ile Glu Asp Ala Lys Lys Glu Lys
705                 710                 715

(2) INFORMATION ZU SEQ ID NO: 26:

(i) SEQUENZ CHARAKTERISTIKA:
(A) LÄNGE: 335 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..335

(xi) SEQUENZBESCHREIBUNG: SEQ ID NO: 26:

Met Ala Lys Arg Val Ala Glu Lys Glu Leu Thr Asp Arg Asn Trp Asp
1               5                   10                  15

Glu Glu Asp Glu Val Glu Glu Met Gly Thr Phe Ser Val Ala Ser Glu
            20                  25                  30

Glu Val Met Lys Asn Arg Ala Val Lys Lys Ala Lys Arg Arg Asn Val
        35                  40                  45

Gly Phe Glu Ser Asp Ser Gly Gly Ala Phe Lys Gly Phe Lys Gly Leu
    50                  55                  60

Val Val Pro Ser Gly Gly Gly Gly Phe Ser Gly Phe Gly Gly Ser Gly
65                  70                  75                  80

Gly Lys Pro Leu Glu Gly Leu Thr Asn Gly Asn Ser Thr Asp Asn Ala
                85                  90                  95

Thr Pro Phe Ser Asn Val Lys Thr Ala Ala Glu Pro Lys Ala Ala Phe
            100                 105                 110

Gly Ser Phe Ala Val Asn Gly Pro Phe Thr Ala Glu Lys Lys Ser Asp
        115                 120                 125

Ala Ala Gln Gly Ala Thr Ser Ala Ser Phe Ser Phe Gly Lys Lys Ile
    130                 135                 140

Glu Ser Ser Ala Leu Gly Ser Leu Ser Ser Gly Ser Leu Thr Gly Phe
145                 150                 155                 160

Ser Phe Ser Ala Gly Ser Ser Ser Leu Phe Gly Lys Asp Ala Ala Gln
                165                 170                 175

Ser Lys Ala Ala Ser Ser Leu Phe Ser Ala Lys Ala Ser Glu Ser Pro
            180                 185                 190

Ala Gly Gly Gly Ser Ser Glu Cys Arg Asp Gly Glu Glu Glu Glu Asn
        195                 200                 205

Asp Glu Pro Pro Lys Val Val Val Thr Glu Val Lys Glu Glu Asp Ala
    210                 215                 220

Phe Tyr Ser Lys Lys Cys Lys Leu Phe Tyr Lys Lys Asp Asn Glu Phe
225                 230                 235                 240

Lys Glu Lys Gly Val Gly Thr Leu His Leu Lys Pro Thr Ala Thr Gln
                245                 250                 255

Lys Thr Gln Leu Leu Val Arg Ala Asp Thr Asn Leu Gly Asn Ile Leu
            260                 265                 270

Leu Asn Val Leu Ile Ala Pro Asn Met Pro Cys Thr Arg Thr Gly Lys
        275                 280                 285

Asn Asn Val Leu Ile Val Cys Val Pro Asn Pro Pro Leu Asp Glu Lys
    290                 295                 300

Gln Pro Thr Leu Pro Ala Thr Met Leu Ile Arg Val Lys Thr Ser Glu
305                 310                 315                 320

Asp Ala Asp Glu Leu His Lys Ile Leu Leu Glu Lys Lys Asp Ala
                325                 330                 335

(2) INFORMATION ZU SEQ ID NO: 27:

    (i) SEQUENZ CHARAKTERISTIKA:
        (A) LÄNGE: 310 Aminosäuren
        (B) ART: Aminosäure
        (C) STRANGFORM: Einzel
        (D) TOPOLOGIE: linear

   (ii) ART DES MOLEKÜLS: Protein

   (ix) MERKMALE:
        (A) NAME/SCHLÜSSEL: Protein
        (B) LAGE: 1..310

(xi) SEQUENZBESCHREIBUNG: SEQ ID NO: 27:

Met Ala Lys Arg Val Ala Glu Lys Glu Leu Thr Asp Arg Asn Trp Asp
1               5                   10                  15

Glu Glu Asp Glu Val Glu Glu Met Gly Thr Phe Ser Val Ala Ser Glu
            20                  25                  30

Glu Val Met Lys Asn Arg Ala Val Lys Lys Ala Lys Arg Arg Asn Val
        35                  40                  45

Gly Phe Glu Ser Asp Ser Gly Gly Ala Phe Lys Gly Phe Lys Gly Leu
    50                  55                  60

Val Val Pro Ser Gly Gly Gly Gly Phe Ser Gly Phe Gly Gly Ser Gly
65                  70                  75                  80

Gly Lys Pro Leu Glu Gly Leu Thr Asn Gly Asn Ser Thr Asp Asn Ala
                85                  90                  95

Thr Pro Phe Ser Asn Val Lys Thr Ala Ala Glu Pro Lys Ala Ala Phe
            100                 105                 110

Gly Ser Phe Ala Val Asn Gly Pro Thr Thr Leu Val Asp Lys Val Ser
        115                 120                 125

Asn Pro Lys Thr Asn Gly Asp Ser Asn Gln Pro Pro Ser Ser Gly Pro
    130                 135                 140

Ala Ser Ser Thr Ala Cys Pro Gly Asn Ala Tyr His Lys Gln Leu Ala
145                 150                 155                 160

Gly Leu Asn Cys Ser Val Arg Asp Trp Ile Val Lys His Val Asn Ile
                165                 170                 175

Asn Pro Leu Cys Asp Leu Thr Pro Ile Phe Lys Asp Tyr Glu Arg Tyr
            180                 185                 190

Leu Ala Thr Ile Glu Lys Gln Leu Glu Asn Gly Gly Gly Ser Ser Ser
        195                 200                 205

Glu Ser Gln Thr Asp Arg Ala Thr Ala Gly Met Glu Pro Pro Ser Leu
    210                 215                 220

Phe Gly Ser Thr Lys Leu Gln Gln Glu Ser Pro Phe Ser Phe His Gly
225                 230                 235                 240

Asn Lys Ala Glu Asp Thr Ser Glu Lys Val Glu Phe Thr Ala Glu Lys
                245                 250                 255

Lys Ser Asp Ala Ala Gln Gly Ala Thr Ser Ala Ser Phe Ser Phe Gly
            260                 265                 270

Lys Lys Ile Glu Ser Ser Ala Leu Gly Ser Leu Ser Ser Gly Ser Leu
        275                 280                 285

Thr Gly Phe Ser Phe Ser Ala Gly Ser Ser Ser Leu Phe Gly Lys Asp
    290                 295                 300

Ala Ala Glu Lys Glu Leu
305                 310

(2) INFORMATION ZU SEQ ID NO: 28:

(i) SEQUENZ CHARAKTERISTIKA:
(A) LÄNGE: 132 Aminosäuren
(B) ART: Aminosäure
(C) STRANGFORM: Einzel
(D) TOPOLOGIE: linear

(ii) ART DES MOLEKÜLS: Protein

(ix) MERKMALE:
(A) NAME/SCHLÜSSEL: Protein
(B) LAGE: 1..132

(xi) SEQUENZBESCHREIBUNG: SEQ ID NO: 28:

Thr Thr Leu Val Asp Lys Val Ser Asn Pro Lys Thr Asn Gly Asp Ser
1               5                   10                  15

Asn Gln Pro Pro Ser Ser Gly Pro Ala Ser Ser Thr Ala Cys Pro Gly
            20                  25                  30

Asn Ala Tyr His Lys Gln Leu Ala Gly Leu Asn Cys Ser Val Arg Asp
        35                  40                  45

Trp Ile Val Lys His Val Asn Thr Asn Pro Leu Cys Asp Leu Thr Pro
    50                  55                  60

Ile Phe Lys Asp Tyr Glu Arg Tyr Leu Ala Thr Ile Glu Lys Gln Leu
65                  70                  75                  80

Glu Asn Gly Gly Gly Ser Ser Ser Glu Ser Gln Thr Asp Arg Ala Thr
                85                  90                  95

Ala Gly Met Glu Pro Pro Ser Leu Phe Gly Ser Thr Lys Leu Gln Gln
            100                 105                 110

Glu Ser Pro Phe Ser Phe His Gly Asn Lys Ala Glu Asp Thr Ser Glu
        115                 120                 125

Lys Val Glu Phe
    130

(2) INFORMATION ZU SEQ ID NO: 29:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS

(ix) MERKMALE:
(A) NAME/SCHLÜSSEL: exon
(B) LAGE: 1..22

(xi) SEQUENZBESCHREIBUNG: SEQ ID NO: 29:

AGAAAGCAAA GCGCAGAAAT GT 22

(2) INFORMATION ZU SEQ ID NO: 30:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 30:

CAAATCCAGA AAAGCGTCCT C 21

(2) INFORMATION ZU SEQ ID NO: 31:

(i) SEQUENZ CHARAKTERISTIKA:
(A) LÄNGE: 24 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..24

(xi) SEQUENZBESCHREIBUNG: SEQ ID NO: 31:

TGAAGAATAG AGCCATAAAG AAAG 24

(2) INFORMATION ZU SEQ ID NO: 32:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 32:

AGAAAAGCGT CCTCCTCCAG A 21

(2) INFORMATION ZU SEQ ID NO: 33:

(i) SEQUENZ CHARAKTERISTIKA:
(A) LÄNGE: 24 Basenpaare
(B) ART: Nukleinsäure
(C) STRANGFORM: Einzel
(D) TOPOLOGIE: linear

(ii) ART DES MOLEKÜLS: DNS

(ix) MERKMALE:
(A) NAME/SCHLÜSSEL: exon
(B) LAGE: 1..24

(xi) SEQUENZBESCHREIBUNG: SEQ ID NO: 33:

TGAAGAATAG AGCCATAAAG AAAG 24

(2) INFORMATION ZU SEQ ID NO: 34:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 34:

AGCGCCACTA ACCAAATCCA GA 22

(2) INFORMATION ZU SEQ ID NO: 35:

(i) SEQUENZ CHARAKTERISTIKA:
(A) LÄNGE: 22 Basenpaare

(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 35:

AGAAAGCAAA GCGCAGAAAT GT 22

(2) INFORMATION ZU SEQ ID NO: 36:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 36:

GAAAAGCGTC CTCCTCCAGA AG 22

(2) INFORMATION ZU SEQ ID NO: 37:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 37:

GAAAGCAAAG CGCAGAAATG TT 22

(2) INFORMATION ZU SEQ ID NO: 38:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 38:

CTCCAGCGCC ACTACCAAAT C 21

(2) INFORMATION ZU SEQ ID NO: 39:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..20

(xi) SEQUENZBESCHREIBUNG: SEQ ID NO: 39:

CCCGCACGGA GCAGTTCAAG 20

(2) INFORMATION ZU SEQ ID NO: 40:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 40:

GCAGCGGCAG ATCCCAAGGT AG 22

(2) INFORMATION ZU SEQ ID NO: 41:

(i) SEQUENZ CHARAKTERISTIKA:
(A) LÄNGE: 17 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..17

(xi) SEQUENZBESCHREIBUNG: SEQ ID NO: 41:

GGCATCCTTT TTCTCCA 17

(2) INFORMATION ZU SEQ ID NO: 42:

(i) SEQUENZ CHARAKTERISTIKA:
(A) LÄNGE: 22 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..22

(xi) SEQUENZBESCHREIBUNG: SEQ ID NO: 42:

CGTTCTTATC GTCTCTGTGT TC 22

(2) INFORMATION ZU SEQ ID NO: 43:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..19

(xi) SEQUENZBESCHREIBUNG: SEQ ID NO: 43:

GCATCCTTTT TCTCCAGTA 19

(2) INFORMATION ZU SEQ ID NO: 44:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..18

(xi) SEQUENZBESCHREIBUNG: SEQ ID NO: 44:

TGTTCCAAAT CCACCAAT 18

(2) INFORMATION ZU SEQ ID NO: 45:

(i) SEQUENZ CHARAKTERISTIKA:
(A) LÄNGE: 19 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..19

(xi) SEQUENZBESCHREIBUNG: SEQ ID NO: 45:

GTCTGCATCC TCGCTGGTT 19

(2) INFORMATION ZU SEQ ID NO: 46:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 46:

CGTTCTTATC GTCTGTGTTC C 21

(2) INFORMATION ZU SEQ ID NO: 47:

(i) SEQUENZ CHARAKTERISTIKA:
(A) LÄNGE: 18 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..18

(xi) SEQUENZBESCHREIBUNG: SEQ ID NO: 47:

TTTTACCCGA ATCAACAT 18

(2) INFORMATION ZU SEQ ID NO: 48:

(i) SEQUENZ CHARAKTERISTIKA:
(A) LÄNGE: 21 Basenpaare
(B) ART: Nukleinsäure
(C) STRANGFORM: Einzel
(D) TOPOLOGIE: linear

(ii) ART DES MOLEKÜLS: DNS (genomisch)

(ix) MERKMALE:
(A) NAME/SCHLÜSSEL: exon
(B) LAGE: 1..21

(xi) SEQUENZBESCHREIBUNG: SEQ ID NO: 48:

GTACGCGAAC AGGGAAGAAT A 21

(2) ANGABEN ZU SEQ ID NO: 49:

(i) SEQUENZKENNZEICHEN:
(A) LÄNGE: 212 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:

(A) NAME/SCHLÜSSEL: Protein
(B) LAGE:1..212

(xi) SEQUENZBESCHREIBUNG: SEQ ID NO: 49:

Arg Val Ser Asn Gly Ser Pro Ser Leu Glu Arg Met Asp Ala Arg Gln
1               5                   10                  15

Ala Asp His Pro Lys Pro Ser Ala Cys Arg Asn Leu Phe Gly Pro Val
            20                  25                  30

Asn His Gly Glu Leu Thr Arg Asp Leu Glu Lys His Cys Arg Asp Met
        35                  40                  45

Glu Glu Ala Ser Gln Arg Lys Trp Asn Phe Asp Phe Gln Asn His Lys
    50                  55                  60

Pro Leu Glu Gly Arg Tyr Glu Trp Gln Glu Val Glu Arg Gly Ser Leu
65                  70                  75                  80

Pro Glu Phe Tyr Tyr Arg Pro Pro Arg Pro Pro Lys Ser Ala Cys Lys
                85                  90                  95

Val Leu Ala Gln Glu Ser Gln Asp Val Ser Gly Ser Arg Gln Ala Val
            100                 105                 110

Pro Leu Ile Gly Ser Gln Ala Asn Ser Glu Asp Arg His Leu Val Asp
        115                 120                 125

Gln Met Pro Asp Ser Ser Asp Asn Gln Ala Gly Leu Ala Glu Gln Cys
    130                 135                 140

Pro Gly Met Arg Lys Arg Pro Ala Ala Glu Asp Ser Ser Ser Gln Ile
145                 150                 155                 160

Lys Arg Ala Asn Arg Thr Glu Glu Asn Val Ser Asp Gly Ser Pro Asn
                165                 170                 175

Ala Gly Thr Val Glu Gln Thr Pro Lys Lys Pro Gly Leu Arg Arg Gln
            180                 185                 190

Thr Arg Val Asp Leu Gln Pro Ser Phe Arg Ala Asn Phe Leu Phe Met
        195                 200                 205

Ile Phe Ile Lys
    210

(2) ANGABEN ZU SEQ ID NO: 50:

(i) SEQUENZKENNZEICHEN:
(A) LÄNGE: 183 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..183

(xi) SEQUENZBESCHREIBUNG: SEQ ID NO: 50:

Met Asp Ala Arg Gln Ala Asp His Pro Lys Pro Ser Ala Cys Arg Asn
1               5                   10                  15

Leu Phe Gly Pro Val Asn His Gly Glu Leu Thr Arg Asp Leu Glu Lys
            20                  25                  30

His Cys Arg Asp Met Glu Glu Ala Ser Gln Arg Lys Trp Asn Phe Asp
        35                  40                  45

Phe Gln Asn His Lys Pro Leu Glu Gly Arg Tyr Glu Trp Gln Glu Val
    50                  55                  60

Glu Arg Gly Ser Leu Pro Glu Phe Tyr Tyr Arg Pro Pro Cys Pro Pro
65                  70                  75                  80

Lys Ser Ala Cys Lys Val Leu Ala Gln Glu Ser Gln Asp Val Ser Gly
                85                  90                  95

Ser Arg Gln Ala Val Pro Leu Ile Gly Ser Gln Ala Asn Ser Glu Asp
            100                 105                 110

Arg His Leu Val Asp Gln Met Pro Asp Ser Ser Asp Asn Gln Ala Gly
        115                 120                 125

Leu Ala Glu Gln Cys Pro Gly Met Arg Lys Arg Pro Ala Ala Glu Asp
    130                 135                 140

Ser Ser Ser Gln Ile Lys Arg Ala Asn Arg Thr Glu Glu Asn Val Ser
145                 150                 155                 160

Asp Gly Ser Pro Asn Ala Gly Thr Val Glu Gln Thr Pro Lys Lys Pro
                165                 170                 175

Gly Leu Arg Arg Gln Thr Arg
            180

(2) ANGABEN ZU SEQ ID NO: 51:

(i) SEQUENZKENNZEICHEN:
(A) LÄNGE: 199 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..199

(xi) SEQUENZBESCHREIBUNG: SEQ ID NO: 51:

Arg Val Ser Asn Gly Ser Pro Ser Pro Glu Arg Met Asp Ala Arg Gln
1               5                   10                  15

Ala Asp His Pro Lys Pro Ser Ala Cys Arg Asn Leu Phe Gly Pro Val
            20                  25                  30

Asn His Gly Glu Leu Thr Arg Asp Leu Glu Lys His Cys Arg Asp Met
        35                  40                  45

Glu Glu Ala Ser Gln His Lys Trp Asn Phe Asp Phe Gln Asn His Arg
    50                  55                  60

Pro Leu Glu Gly Arg Tyr Glu Trp Gln Glu Val Glu Arg Gly Ser Leu
65                  70                  75                  80

Pro Glu Phe Tyr Tyr Arg Pro Pro Arg Pro Pro Lys Ser Ala Cys Lys
                85                  90                  95

Val Leu Ala Gln Glu Ser Gln Asp Val Ser Gly Ser Arg Gln Ala Val
            100                 105                 110

Pro Leu Ile Gly Ser Gln Ala Asn Ser Glu Asp Arg His Leu Val Asp
        115                 120                 125

Gln Met Pro Asp Ser Ser Asp Asn Gln Ala Gly Leu Ala Glu Gln Cys
    130                 135                 140

Pro Gly Met Arg Lys Arg Pro Ala Ala Glu Asp Ser Ser Ser Gln Ile
145                 150                 155                 160

Lys Arg Ala Asn Arg Thr Glu Glu Asn Val Ser Asp Gly Ser Pro Asn
                165                 170                 175

Ala Gly Thr Val Glu Gln Thr Pro Lys Lys Pro Gly Pro Arg Arg Gln
            180                 185                 190

Thr Arg Val Asp Leu Gln Pro
        195

(2) ANGABEN ZU SEQ ID NO: 52:

(i) SEQUENZKENNZEICHEN:
(A) LÄNGE: 199 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..199

(xi) SEQUENZBESCHREIBUNG: SEQ ID NO: 52:

Arg Val Ser Asn Gly Ser Pro Ser Leu Glu Arg Met Asp Ala Arg Gln
1               5                   10                  15

Ala Asp His Pro Lys Pro Ser Ala Cys Arg Asn Leu Phe Gly Pro Val
            20                  25                  30

Asn His Gly Glu Leu Thr Arg Asp Leu Glu Lys His Cys Arg Asp Met
        35                  40                  45

Glu Glu Ala Ser Gln Arg Lys Trp Asn Phe Asp Phe Gln Asn His Lys
    50                  55                  60

Pro Leu Glu Gly Arg Tyr Glu Trp Gln Glu Val Glu Arg Gly Ser Leu
65                  70                  75                  80

Pro Glu Phe Tyr Tyr Gly Pro Pro Arg Pro Pro Lys Ser Ala Cys Lys
                85                  90                  95

Val Leu Ala Gln Glu Ser Gln Asp Val Gly Gly Ser Arg Gln Ala Val
            100                 105                 110

Pro Leu Ile Gly Ser Gln Ala Asn Ser Glu Asp Arg His Leu Val Asp
        115                 120                 125

Gln Met Pro Asp Ser Ser Asp Asn Gln Ala Gly Leu Ala Glu Gln Cys
    130                 135                 140

Pro Gly Met Arg Lys Arg Pro Ala Ala Glu Asp Ser Ser Ser Gln Ile
145                 150                 155                 160

Lys Arg Ala Asn Arg Thr Glu Glu Asn Val Ser Asp Gly Ser Pro Asn
                165                 170                 175

Ala Gly Thr Val Glu Gln Thr Pro Lys Lys Pro Gly Leu Arg Arg Gln
            180                 185                 190

Thr Arg Val Asp Leu Gln Pro
        195

(2) ANGABEN ZU SEQ ID NO: 53:

(i) SEQUENZKENNZEICHEN:
(A) LÄNGE: 199 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..199

(xi) SEQUENZBESCHREIBUNG: SEQ ID NO: 53:

Val Ser Asn Gly Ser Pro Ser Leu Glu Arg Met Asp Ala Arg Gln Ala
1               5                   10                  15

Asp His Pro Lys Pro Ser Ala Cys Arg Asn Leu Phe Gly Pro Val Asn
            20                  25                  30

His Gly Glu Leu Thr Arg Asp Leu Glu Lys His Cys Arg Asp Met Glu
        35                  40                  45

Glu Ala Ser Gln Arg Lys Trp Asn Phe Asp Phe Gln Asn His Lys Pro
    50                  55                  60

Leu Glu Gly Arg Tyr Glu Trp Gln Glu Val Glu Arg Gly Ser Leu Pro
65                  70                  75                  80

Glu Phe Tyr Tyr Gly Pro Pro Arg Pro Pro Lys Ser Ala Cys Lys Val
                85                  90                  95

Leu Ala Gln Glu Ser Gln Asp Val Ser Gly Ser Arg Gln Ala Val Pro
            100                 105                 110

Leu Ile Gly Ser Gln Ala Asn Ser Glu Asp Arg His Leu Val Asp Gln
        115                 120                 125

Met Pro Asp Ser Ser Asp Asn Gln Ala Gly Leu Ala Glu Gln Cys Pro
    130                 135                 140

Gly Met Arg Lys Arg Pro Ala Ala Glu Asp Ser Ser Ser Gln Ile Lys
145                 150                 155                 160

Arg Ala Asn Arg Thr Glu Glu Asn Val Ser Asp Gly Ser Pro Asn Ala
                165                 170                 175

Gly Thr Val Glu Gln Thr Pro Lys Lys Pro Gly Leu Arg Arg Gln Thr
            180                 185                 190

Arg Val Asp Leu Gln Pro Ser
        195

(2) ANGABEN ZU SEQ ID NO: 54:

(i) SEQUENZKENNZEICHEN:
(A) LÄNGE: 220 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..220

(xi) SEQUENZBESCHREIBUNG: SEQ ID NO: 54:

Asn Val Arg Val Ser Asn Gly Ser Pro Ser Leu Glu Arg Met Asp Ala
1               5                   10                  15

Arg Gln Ala Asp His Pro Pro Ser Ala Cys Arg Asn Leu Phe Gly Pro
            20                  25                  30

Val Asn His Gly Glu Leu Thr Arg Asp Leu Glu Lys His Cys Arg Asp
        35                  40                  45

Met Glu Glu Ala Ser Gln Arg Lys Trp Asn Phe Asp Phe Gln Asn His
    50                  55                  60

Lys Pro Leu Glu Gly Arg Tyr Glu Trp Gln Glu Val Glu Arg Gly Ser
65                  70                  75                  80

Leu Pro Glu Phe Tyr Tyr Arg Pro Pro Arg Pro Pro Lys Ser Ala Cys
                85                  90                  95

Lys Val Pro Ala Gln Glu Ser Gln Asp Val Ser Gly Ser Arg Gln Ala
            100                 105                 110

Val Pro Leu Ile Gly Ser Gln Ala Asn Ser Glu Asp Arg His Leu Val
        115                 120                 125

Asp Gln Met Pro Asp Ser Ser Asp Asn Gln Ala Gly Leu Ala Glu Gln
    130                 135                 140

Cys Pro Gly Met Arg Lys Arg Pro Ala Ala Glu Asp Ser Ser Ser Gln
145                 150                 155                 160

Ile Lys Arg Ala Asn Arg Thr Glu Glu Asn Val Ser Asp Gly Ser Pro
                165                 170                 175

Asn Ala Gly Thr Val Glu Gln Thr Pro Lys Lys Pro Gly Leu Arg Arg
            180                 185                 190

Gln Thr Arg Val Asp Leu Gln Pro Ser Phe Arg Ala Asn Phe Leu Phe
        195                 200                 205

Met Ile Phe Ile Ile Lys Val Ile Lys Lys Ile Ser
    210                 215                 220

(2) ANGABEN ZU SEQ ID NO: 55:

    (i) SEQUENZKENNZEICHEN:
        (A) LÄNGE: 183 Aminosäuren
        (B) ART: Aminosäure
        (C) STRANGFORM: Einzelstrang
        (D) TOPOLOGIE: linear

    (ii) ART DES MOLEKÜLS: Protein

    (ix) MERKMAL:
        (A) NAME/SCHLÜSSEL: Protein
        (B) LAGE:1..183

    (xi) SEQUENZBESCHREIBUNG: SEQ ID NO: 55:

Met Asp Ala Arg Gln Ala Asp His Pro Lys Pro Ser Ala Cys Arg Asn
1               5                   10                  15

Leu Phe Gly Pro Val Asn His Gly Glu Leu Thr Arg Asp Leu Glu Lys
            20                  25                  30

His Cys Gln Asp Met Glu Glu Ala Ser Gln Arg Lys Trp Asn Phe Asp
        35                  40                  45

Phe Gln Asn His Lys Pro Leu Glu Gly Arg Tyr Glu Trp Gln Glu Val
    50                  55                  60

Glu Arg Gly Ser Leu Pro Glu Phe Tyr Tyr Arg Pro Pro Arg Pro Pro
65                  70                  75                  80

Lys Ser Ala Cys Lys Val Leu Ala Gln Glu Ser Gln Asp Val Ser Gly
                85                  90                  95

Ser Arg Gln Ala Val Pro Leu Ile Gly Ser Gln Ala Asn Ser Glu Asp
            100                 105                 110

Arg His Leu Val Asp Gln Met Pro Asp Ser Ser Asp Asn Gln Ala Gly
        115                 120                 125

Leu Ala Glu Gln Cys Pro Gly Met Arg Lys Arg Pro Ala Ala Glu Asp
    130                 135                 140

Ser Ser Ser Gln Ile Lys Arg Ala Asn Arg Thr Glu Glu Asn Val Ser
145                 150                 155                 160

Asp Gly Ser Pro Asn Ala Gly Thr Val Glu Gln Thr Pro Lys Lys Pro
                165                 170                 175

Gly Leu Arg Arg Gln Thr Arg
            180

(2) ANGABEN ZU SEQ ID NO: 56:

    (i) SEQUENZKENNZEICHEN:
        (A) LÄNGE: 138 Aminosäuren
        (B) ART: Aminosäure
        (C) STRANGFORM: Einzelstrang
        (D) TOPOLOGIE: linear

    (ii) ART DES MOLEKÜLS: Protein

    (ix) MERKMAL:
        (A) NAME/SCHLÜSSEL: Protein
        (B) LAGE:1..138

    (xi) SEQUENZBESCHREIBUNG: SEQ ID NO: 56:

Met Asp Ala Arg Gln Ala Asp His Pro Lys Pro Ser Ala Cys Arg Asn
1               5                   10                  15

Leu Phe Gly Pro Val Asn His Gly Glu Leu Thr Arg Asp Leu Glu Lys
            20                  25                  30

His Cys Arg Asp Met Glu Glu Ala Ser Gln Arg Lys Trp Asn Phe Asp
        35                  40                  45

Phe Gln Asn His Lys Pro Leu Glu Gly Arg Tyr Glu Trp Gln Glu Val
    50                  55                  60

Glu Arg Gly Ser Leu Pro Glu Phe Tyr Tyr Gly Pro Pro Arg Pro Pro
65                  70                  75                  80

Lys Ser Ala Cys Lys Val Leu Ala Gln Glu Ser Gln Asp Val Ser Gly
                85                  90                  95

Ser Arg Gln Ala Val Pro Leu Ile Gly Ser Gln Ala Asn Ser Glu Asp
            100                 105                 110

Arg His Leu Val Asp Gln Met Pro Asp Ser Ser Asp Ser Gln Ala Gly
        115                 120                 125

Leu Ala Glu Gln Cys Pro Gly Met Arg Lys
    130                 135

(2) ANGABEN ZU SEQ ID NO: 57:

(i) SEQUENZKENNZEICHEN:
(A) LÄNGE: 197 Aminosäuren
(B) ART: Aminosäure
(C) STRANGFORM: Einzelstrang
(D) TOPOLOGIE: linear

(ii) ART DES MOLEKÜLS: Protein

(ix) MERKMAL:
(A) NAME/SCHLÜSSEL: Protein
(B) LAGE:1..197

(xi) SEQUENZBESCHREIBUNG: SEQ ID NO: 57:

Met Ser Asn Val Arg Val Ser Asn Gly Ser Pro Ser Leu Glu Arg Met
1               5                   10                  15

Asp Ala Arg Gln Ala Asp His Pro Lys Pro Ser Ala Cys Arg Asn Leu
            20                  25                  30

Phe Gly Pro Val Asn His Glu Glu Leu Thr Arg Asp Leu Glu Lys His
        35                  40                  45

Cys Arg Asp Met Glu Glu Ala Ser Gln Arg Lys Trp Asn Phe Asp Phe
    50                  55                  60

Gln Asn His Lys Pro Leu Glu Gly Arg Tyr Glu Trp Gln Glu Val Glu
65                  70                  75                  80

Arg Gly Ser Leu Pro Glu Phe Tyr Tyr Arg Pro Pro Arg Pro Pro Lys
                85                  90                  95

Ser Ala Cys Lys Val Leu Ala Gln Glu Ser Gln Asp Val Ser Gly Ser
            100                 105                 110

Arg Gln Ala Val Pro Leu Ile Gly Ser Gln Ala Asn Ser Glu Asp Arg
        115                 120                 125

His Leu Val Asp Gln Met Pro Asp Ser Ser Asp Asn Gln Ala Gly Leu
    130                 135                 140

Ala Glu Gln Cys Pro Gly Met Arg Lys Arg Pro Ala Ala Glu Asp Ser
145                 150                 155                 160

Ser Ser Gln Asn Lys Arg Ala Asn Arg Thr Glu Glu Asn Val Ser Asp
                165                 170                 175

Gly Ser Pro Asn Ala Gly Thr Val Glu Gln Thr Pro Lys Lys Pro Gly
            180                 185                 190

Leu Arg Arg Gln Thr
        195

## Beschreibung

Die vorliegende Anmeldung betrifft ein Protein, welches das zelluläre Protein p27 inhibiert und hierdurch die durch p27 bewirkte Hemmung der Proliferation der Zelle aufhebt, Mutanten davon mit z.T. dominant interferierenden Charakter, die entsprechenden dafür kodierenden Nukleinsäuren und die Verwendung von Protein und Nukleinsäuren für die Prophylaxe und Therapie von Erkrankungen. Desweiteren werden Nukleinsäurekonstrukte für die Gentherapie von Erkrankungen offenbart, enthaltend o.g. Nukleinsäuren.

### A) Einführung

Der Zellzyklus eukaryotischer Zellen wird gesteuert durch cyclinabhängige Kinasen ("cyclin-dependent kinases" = cdk's); dies sind Kinasen, die für ihre Aktivität eine regulatorische Untereinheit ("Cyclin") benötigen. Verschiedene Prozesse im Zellzyklus (wie zum Beispiel der Replikation, Eintritt in die Mitose) werden durch verschiedene cdk's gesteuert (Morgan, Nature 374, 131 (1995)). Hierbei ist die Aktivität cyclinabhängiger Kinasen hochreguliert. Dabei gibt es interne Steuerungsmechanismen, die zum Beispiel den Eintritt in die Mitose verhindern, so lange die DNA nicht komplett repliziert ist. Die Steuerung durch externe Faktoren, wie zum Beispiel Wachstumsfaktoren, findet ausschließlich vor dem Beginn der DNA-Replikation statt, Replikation wird durch aktive Cyclin E/cdk2 Komplexe initiiert.

Neben der Menge an Cyclin an der Kinase-Untereinheit, wird die Aktivität cyclinabhängiger Kinasen durch kleine Inhibitorproteine reguliert (Sherr und Roberts, Gene Dev 9 , 1149 (1995)). Beispielsweise sind für Cyclin E/cdk2 zwei Inhibitoren, die nach ihrer Größe als p21 und als p27 bezeichnet werden, entscheidend. In Zellen, die hohe Mengen an p27 exprimieren, ist die Cyclin E/cdk2 Kinase normalerweise inaktiv und der Eintritt in die DNA-Replikation ist blockiert.

In vielen humanen Tumoren sind positive Zellzyklusregulatoren überexprimiert oder zumindest konstitutiv exprimiert (Sherr, Science 274, 1672 (1996)), negative Regulatoren aber häufig mutiert oder schwach exprimiert (Fero et al., Cell 85, 733 (1996)). Es gibt spezifische Korrelationen: beispielsweise findet man in vielen Nackentumoren die Überexpression des Cyclin D1-Genes. Daher besteht die Hoffnung, daß cyclinabhängige Kinasen und ihre Funktion Zielstrukturen bei der Suche nach neuen, selektiv wirksamen, antiproliferativ wirkenden Substanzen sein könnten.

Das Gen für das p27 Protein ist seit einigen Jahren bekannt (K. Polyak et al. Cell 78, 59-66 (1994)) und in der Genbank zugänglich [murines p27; Zugangsnummer (Accession Number) K 09968; humanes p27: K 10906]. Trotz intensiver Suche wurden bisher Mutationen im p27 Gen in menschlichen Tumoren nicht gefunden. Dies ist um so überraschender, weil Mäuse, in denen das Gen für p27 inaktiviert worden ist, einen Phänotyp mit multiplen Dysplasien und erhöhter Tumorinzidenz zeigen (Fero et al., Cell 85, 733 (1996), Kiyokawa et al., Cell 85, 721 (1996)). Statt dessen wird offensichtlich die Funktion von p27 im wesentlichen posttranskriptionell reguliert.

So wird p27 durch Proteolyse abgebaut; dies geschieht auch zu Beginn der DNA-Replikation von normalen Zellen. Die Fähigkeit, p27 proteolytisch abzubauen, ist in vielen Tumoren deutlich gegenüber dem Normalgewebe erhöht und dieses scheint direkt mit einer ungünstigen Prognose (Loda et al., Nature Med. 3, 231 (1997)) zu korrelieren.

Es muß aber noch weitere Mechanismen geben, die zur Inaktivierung von p27 führen können. Zum Beispiel wird nach der Transformation von Zellen durch das Myc-Onkogen p27 erst funktionell inaktiviert (d.h., es bindet nicht mehr an Cyclin/cdk Komplexe), aber erst viel später abgebaut. Bislang ist unerklärlich, warum bei einer Reihe von zum Beispiel Brusttumoren hohe Mengen von p27 Proteinen exprimiert werden, und diese Tumoren trotzdem sehr schnell wachsen. Die Mechanismen, die p27 in diesen Tumoren inaktivieren, sind bislang unbekannt (Fredersdorf et al., Proc. Natl. Acad. Sci. USA 94, 6380 (1997)).

Es besteht demgemäß ein großes Interesse der Fachwelt, Mechanismen bzw. Stoffe aufzufinden, die für die Inaktivierung von p27 in Tumoren verantwortlich sind. Mit der vorliegenden Erfindung wurde dieses Problem gelöst. Das in der Anmeldung beschriebene Protein p163 kann p27 binden, die Funktion von p27 inhibieren und p27 im Zytoplasma der Proteolyse zuführen.

### B) Allgemeine Beschreibung der Erfindung

### 1) Das Protein p163 und seine Nukleinsäuresequenz

Gegenstand der Erfindung ist ein neues Protein, genannt p163, welches p27 bindet, hierdurch in seiner Funktion inhibiert und in das Zytoplasma der Proteolyse zuführt. Die Aminosäuresequenz dieses Proteins wurde ermittelt.

Das Protein p163 bindet spezifisch an p27 und nicht an andere Proteine wie Myc, Max, Bcl-6 oder Prothymosin-α.

Gegenstand der Erfindung ist desweiteren eine Nukleotidsequenz, welche für das Protein p163 kodiert. Diese Nukleotidsequenz wurde für das Mäuse-p163 Protein und durch Sequenzvergleich auch für das humane p163 Protein ermittelt.

### 2) Dominant negative p163 Analoga

Gegenstand dieser Erfindung sind desweiteren Nukleotidsequenzen, welche für Teilpeptide von p163 und/oder für p163 analoge, dominant negativ wirkende Proteine kodieren, d.h. die die Bindung des natürlichen Proteins p163 an das Protein p27 inhibieren, ohne die Bindung des p27 Proteins an Cyclin E/cdk2 und die hierdurch bedingte Inhibition von Cyclin E/cdk2 wesentlich zu beeinträchtigen.

Gegenstand der Erfindung ist desweiteren die Verwendung von Nukleinsäuresequenzen, die für dominant negative Mutanten des p163 Proteins oder Teilsequenzen hiervon kodieren, wobei die Verwendung beinhaltet die Einführung dieser Nukleinsäuresequenzen in eine Zielzelle und die Inhibition der Bindung zwischen dem zielzelleigenen p163 Protein und dem zielzelleigenen p27 Protein, so daß die Proliferation der Zielzelle durch das freiwerdende zielzelleigene p27 Protein gehemmt wird.

### 3) Peptide, die p163 hemmen

Gegenstand dieser Erfindung sind jedoch auch Nukleotidsequenzen, welche für Proteine oder Peptide kodieren, die die Funktion von p163 hemmen. Hierzu gehören Proteine oder Peptide, die an die Bindestelle des p163 Proteins für das p27 Protein oder an die Bindestelle des p163 Proteins für das Ran (Loeb et al. Mol. Cell. Biol. 4, 209-222 (1993)) binden und hierdurch das zellinterne p163 Protein hemmen.

Zu diesen Proteinen gehören Peptide, die der Bindestelle des p27 Proteins oder des Ran Proteins für das p163 Protein entsprechen. Zu diesen Proteinen gehören des weiteren Antikörper oder Antikörperspaltprodukte, wie F(ab)₂ Fab, Fv und s.c. Fv mit Spezifität gegen das Protein p163, insbesondere gegen dessen Bindestelle für das p27 Protein.

Gegenstand der Erfindung ist des weiteren die Verwendung von Nukleinsäuresequenzen kodierend für derartige inhibierende Proteine oder Peptide, wobei die Verwendung beinhaltet die Einführung dieser Nukleinsäuresequenzen in eine Zielzelle und die Inhibition der Bindung zwischen dem zielzelleigenen p163 Protein und dem zielzelleigenen p27 Protein oder dem zielzelleigenen Ran Protein, so daß die Proliferation der Zielzelle durch das freiwerdende zielzelleigene p27 Protein gehemmt wird.

### 4) Verwendung von p163 zur Stimulation der Zellteilung

Gegenstand dieser Erfindung ist des weiteren die Verwendung der Nukleotidsequenz kodierend für das p163 Protein oder das p163 Protein selbst zur Hemmung des zellinternen p27 Proteins und damit zur Stimulation der Zellteilung einer Zielzelle.

### 5) Inhibition der Transkription und Translation von p163

Gegenstand der Erfindung sind jedoch auch Nukleinsäuresequenzen, welche die Transkription und/oder Translation der zielzelleigenen Nukleinsäuresequenz kodierend für das p163 Protein hemmen und hierdurch zu einem Freiwerden des p27 Proteins und damit zu einer Inhibition der Proliferation der Zielzelle führen. Derartige erfindungsgemäße Nukleinsäuresequenzen können beispielsweise antisense (Triple-)DNA, antisense RNA und/oder Ribozyme darstellen.

### 6) Testsysteme zur Findung von p163 Inhibitoren

Gegenstand der Erfindung ist des weiteren die Verwendung der Nukleinsäuresequenz kodierend für das p163 Protein oder des p163 Proteins oder Teilsequenzen hiervon für Testsysteme, in denen nach Inhibitoren der Bindung zwischen dem P163 Protein und dem p27 Protein oder dem Ran Protein gesucht werden und die Verwendung der in diesen Testsystemen gefundenen Inhibitoren des p163 Proteins für die Inhibition der Proliferation einer Zielzelle.

### 7) Nachweis von p163 zur Diagnose einer Erkrankung

Gegenstand der Erfindung ist des weiteren die Verwendung von Nukleinsäurekonstrukten kodierend für das p163 Protein oder für Teilsequenzen des p163 Proteins oder von Nukleinsäuresequenzen oder Proteinsequenzen, welche an diese Nukleinsäuresequenzen für das p163 Protein binden oder von Proteinen, welche an das p163 Protein binden zum Nachweis von Nukleinsäuren kodierend für p163 oder zum Nachweis des p163 Proteins in einer Zelle oder einem Gewebe oder einer Körperflüssigkeit zum Zwecke der Ermittlung des Proliferationszustandes einer Zelle oder eines Gewebes oder der Diagnose eines Krankheitszustandes.

### 8) Expressionssysteme enthaltend Bindesequenzen für p27 oder p163/p27 Proteinkomplexe

Gegenstand der Erfindung ist des weiteren ein Expressionssystem, das eine Nukleinsäuresequenz enthält, deren Expression durch das p27 Protein gesteuert wird und welches im einfachsten Fall folgende Komponenten enthält:
Komponente a)
   ― mindestens eine Aktivierungssequenz (Promotor Nr. I)
Komponente b)
   ― mindestens ein Gen für einen Transkriptionsfaktor bestehend aus einem Fusionsprotein enthaltend
      - Komponente b₁) mindestens eine Aktivierungsdomäne eines Transkriptionsfaktors
      - Komponente b₂)
         1)mindestens eine Sequenz oder mindestens eine Teilsequenz des p163 Proteins, an welcher das p27 Protein bindet, oder
         2) mindestens einen Antikörper oder mindestens ein Antikörperfragment wie Fab, Fv oder scFv, welches an die Bindestelle des Proteins p27 für das p163 Protein bindet, oder
         3) mindestens einen Antikörper oder mindestens ein Antikörperfragment wie Fab, Fv oder scFv, welches an die Bindestelle des Proteins p163 für das p27 Protein bindet, oder
         4) mindestens einen Antikörper oder mindestens ein Antikörpertragment wie Fab, Fv oder scFv, welches an den Komplex aus p163 und p27 bindet.
      - Komponente b₃) mindestens eine DNA-Bindedomäne eines Transkriptionsfaktors
Komponente c)
   ― mindestens eine Aktivierungssequenz (Promotor Nr. II), welche durch Bindung des Transkriptionsfaktors, kodiert von der Komponente b) aktiviert wird
Komponente d)
   ― mindestens ein Effektorgen.

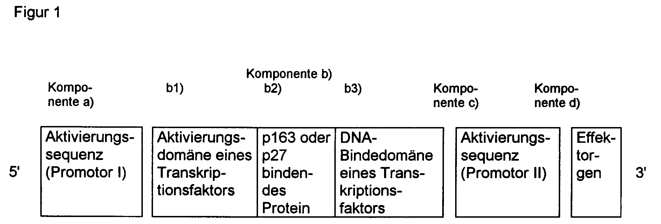

Die Anordnung der einzelnen Komponenten ist beispielhaft in Figur 1 wiedergegeben. Voraussetzung für die erfindungsgemäße Funktionsfähigkeit des Expressionssystems ist, daß die Komponente b₂1), b₂2) oder b₂3) derartig zwischen oder an die Komponenten b₁) und b₃) gefügt ist, daß die Bindung des p27 Proteins oder des p163 Proteins an das Expressionsprodukt der Komponente b₂) die Funktionsfähigkeit der Aktivierungsdomäne [Komponente b₁)] und/oder der DNA-Bindedomäne [Komponente b₃)] inhibiert. Diese Inhibition führt in Zellen, in welchen freies p27 oder freies p163 vorliegt, zu einer Inhibition der Expression des Effektorgenes. In Zellen, in welchen das p27 Protein und/oder das p163 Protein komplexiert ist, so daß es nicht mehr mit der Komponente b₂) interagieren kann oder aber nicht mehr oder nur gering vorhanden ist, fehlt diese Inhibition, so daß der Transkriptionsfaktor [Komponente b)] ungehindert die Aktivierungssequenz [Komponente c)] aktivieren und damit die Transkription des Effektorgenes in die Wege leiten kann.

Eine Besonderheit stellt die Komponente b₂4) dar. Durch Bindung von p163/p27 Komplexen an die Komponente b₂4) in sich teilenden Zellen wird der Transkriptionsfaktor [Komponente b)] inhibiert, in ruhenden Zellen (d.h. bei freiem p27) ist dagegen der Transkriptionsfaktor [Komponente b)] frei und voll funktionsfähig.

Die Transkription des Effektorgenes wird eingeleitet durch die Aktivierung der Aktivierungssequenz [Komponente a)], welche eine Expression des Genes für den Transkriptionsfaktor Komponente b) zur Folge hat. Der Transkriptionsfaktor [Komponente b)] wiederum bindet an die Aktivierungssequenz [Komponente c)], welche eine Expression des Effektorgenes [Komponente d)] induziert.

In einer besonderen Ausführungsform dieser Erfindung ist die Komponente a) gleich der Komponente c). In dieser besonderen Ausführungsform führt eine geringe Aktivierung der Aktivierungssequenz [Promotor I, Komponente a)] zu einer Expression des Transkriptionsfaktors [Komponente b)], welcher sowohl die Aktivierungssequenz [Promotor I, Komponente a)] als auch die Aktivierungssequenz [Promotor II, Komponente c)] aktiviert und hierdurch die Expression sowohl des Genes für das Effektorgen [Komponente d)] induziert als auch die Expression des Transkriptionsfaktors [Komponente b)] verstärkt, wodurch wiederum die Expression des Effektorgenes [Komponente d)] verstärkt wird.

Dieses Expressionssystem kann erweitert werden
― durch die Aneinanderreihung mehrerer gleicher oder unterschiedlicher Sequenzen für Effektorgene [Komponenten d), d'), d'')], welche jeweils miteinander durch gleiche oder unterschiedliche IRES-Sequenzen oder durch Aktivierungssequenzen [Komponenten c') und c'')] verbunden sind.
― durch die Aneinanderreihung mehrerer gleicher oder unterschiedlicher Gene für Transkriptionsfaktoren [Komponente b)], die jeweils miteinander durch gleiche oder unterschiedliche IRES-Sequenzen oder Aktivierungssequenzen [Komponente a) oder Komponente c)] verbunden sind.

Bei einer Aneinanderreihung von Genen für unterschiedliche Transkriptionsfaktoren sind die Aktivierungssequenzen so auszuwählen, daß sie Nukleotidsequenzen enthalten, an welche der Transkriptionsfaktor [Komponente b)] binden kann.

Durch die erfindungsgemäßen Nukleinsäurekonstrukte kann ein Effektorgen [Komponente d)] je nach Wahl der Aktivierungssequenz [Komponenten a) oder c)] unspezifisch, zellspezifisch oder virusspezifisch oder unter bestimmten metabolischen Bedingungen oder auch zellzyklusspezifisch exprimiert werden. Bei dem Effektorgen handelt es sich um ein Gen, das seinerseits für einen pharmakologisch aktiven Wirkstoff oder aber für ein Enzym kodiert, welches eine inaktive Vorstufe eines Pharmakons in ein aktives Pharmakon spaltet.

Beispielsweise kann des Effektorgen so ausgewählt sein, daß dieser Wirkstoff oder dieses Enzym als Fusionsprotein mit einem Liganden exprimiert wird und dieser Ligand an die Oberfläche von Zellen, zum Beispiel Endothel- oder Tumorzellen oder Leukozyten, bindet.

Die aufgeführten erfindungsgemäßen Nukleinsäurekonstrukte bestehen bevorzugterweise aus DNS. Unter dem Begriff "Nukleinsäurekonstrukte" werden künstliche Gebilde aus Nukleinsäure verstanden, die in den Zielzellen transkribiert werden können. Sie sind bevorzugt in einen Vektor eingefügt, wobei Plasmidvektoren oder virale Vektoren besonders bevorzugt sind.

Das Nukleinsäurekonstrukt, ggf. eingefügt in einen Vektor, wird einem Patienten zur Prophylaxe oder Therapie einer Erkrankung verabreicht. Die Verabreichung kann peroral, lokal oder per Injektion oder Infusion erfolgen.

Gegenstand der vorliegenden Erfindung sind auch Zellen von Säugern, die ein erfindungsgemäßes Nukleinsäurekonstrukt enthalten. In besonders bevorzugter Ausführungsform werden die Nukleinsäurekonstrukte in Zelllinien eingebracht, die dann nach Transfektion als Träger des erfindungsgemäßen Expressionssystems zur Expression des Effektorgenes verwendet werden können. Derartige Zellen können zur Bereitstellung eine Heilmittels für Patienten benutzt werden. Alternativ können die Zellen oder Zelllinien, wie z.B. Tumor-, Immun- oder Endothelzellen, in welche die erfindungsgemäße Nukleinsäurekonstrukte eingebracht wurden, in Patienten lokal verabreicht oder parenteral zum Beispiel intravenös, intraarteriell in eine Körperhöhle, in ein Organ oder subkutan injiziert werden.

Eine bevorzugte Verwendung des erfindungsgemäßen Nukleinsäurekonstruktes besteht somit in der Prophylaxe oder Behandlung einer Erkrankung, wobei die Erfindung die in vitro Einführung eines Nukleinsäurekonstruktes in eine Zielzelle, die unspezifische, virus- oder zielzellspezifische, metabolisch spezifische und/oder zellzyklusspezifische Expression des Heilmittels in der Zielzelle und die lokale oder parenterale Verabreichung der Zielzelle an den Patienten oder aber die lokale oder parenterale Verabreichung des Nukleinsäurekonstruktes an den Patienten zu in vivo Einführung eines Nukleinsäurekonstruktes in die Zielzelle umfaßt.

Die erfindungsgemäßen Nukleinsäurekonstrukte kommen in dieser Form nicht in der Natur vor, d.h. das Effektorgen für den Wirkstoff oder für ein Enzym oder für ein Ligand-Wirkstoff- oder Ligand-Enzym-Fusionsprotein ist nicht natürlicherweise kombiniert mit Nukleinsäuresequenzen, wie sie das erfindungsgemäße Nukleinsäurekonstrukt enthält.

Bevorzugte Effektorgene [Komponente d)], welche in ein erfindungsgemäßes Expressionssystem eingebaut werden, kodieren für einen pharmakologisch aktiven Wirkstoff. Dieses sind Proteine und Glykoproteine, ausgewählt aus der Gruppe enthaltend Cytokine, Wachstumsfaktoren, Rezeptoren für Cytokine oder Wachstumsfaktoren, Antikörper oder Antikörperfragmente, antiproliferativ oder zytostatisch wirkende Proteine, apoptotisch oder antiapoptotisch wirkende Proteine, Tumorantigene, Angiogeneseinhibitoren, Thrombose-induzierende Proteine, Gerinnungshemmer, fibrinolytisch wirkende Proteine, Blutplasmaproteine, Komplement-aktivierende Proteine, Hüllsubstanzen von Viren und Bakterien, Hormone, kreislaufwirksame Peptide, Neuropeptide, Enzyme, Mediatoren, natürlicherweise vorkommende, nicht veränderte Regulatorproteine und Ribozyme oder auf die Genexpression inhibierend wirkende (antisense) Nukleotidsequenzen.

Bevorzugterweise handelt es sich bei dem Transgen um ein Effektorgen, das für ein Ribozym kodiert, welches die mRNA inaktiviert, welche kodiert für ein Protein ausgewählt aus der Gruppe enthaltend Zellzykluskontrollproteine, insbesondere Cyclin A, Cyclin B, Cyclin D1, Cyclin E, E2F1-5, cdc2, cdc25C, p163 oder DP1 oder Virusproteine oder Cytokine oder Wachstumsfaktoren oder deren Rezeptoren.

In einer weiteren Ausführungsform kann des Effektorgen für ein Ligand-Wirkstoffusionsprotein kodieren, wobei der Ligand ein Antikörper, ein Antikörperfragment, ein Cytokin, ein Wachstumsfaktor, ein Adhäsionsmolekül oder ein Peptidhormon sein kann und der Wirkstoff ein wie oben beschriebener pharmakologisch aktiver Wirkstoff oder ein Enzym. Beispielsweise kann das Effektorgen ein Ligand-Enzymfusionsprotein kodieren, wobei das Enzym eine Vorstufe eines Pharmakons in ein Pharmakon spaltet und der Ligand an eine Zelloberfläche bindet, bevorzugt an Endothelzellen oder Tumorzellen.

### C) Nähere Beschreibung der Erfindung

### 1) Charakterisierung des p163 Proteins und der das p163 Protein kodierenden Nukleinsäuresequenz

Mit Hilfe der "two-hybrid-Technologie" (Fields und Song, Nature 340, 245 (1989)) wurde das p 163 Proteins als ein neues Partnerprotein von p27 entdeckt. Hierzu wurde die gesamte kodierende Sequenz des murinen p27 Proteins mit Hilfe von PCR in den Hefevektor pGBT10 (Clontech) in-frame mit der DNA-bindenden Domäne des Transkriptionsfaktors GAL4 kloniert. Der Hefestamm Hf7c (Fa. Clontech Heidelberg, Matchmaker Two Hybrid, K 1605-1) wurde mit diesem Vektor transformiert, tryptophan-auxotrophe Kolonien isoliert und die Expression der korrekten Fusionsproteine im Western Blot mit Hilfe spezifischer Antikörper gegen GAL4 und p27 nachgewiesen.

Dieser Stamm wurde in einer zweiten Transformation mit einer VP16-getaggten cDNA-Bibliothek aus Mausembryonen transformiert (Vojtek et al., Cell 74, 205 (1993)). 400 Kolonien, die histidin-auxotroph in Gegenwart von 15 nM Aminotriazol waren, wurden weiter analysiert. 70 dieser Klone wurden sequenziert; sie kodierten alle für verschiedene D-Typ Cycline; das sind bekannte Interaktionspartner von p27. Mit Hilfe von Southern Blots wurde gezeigt, daß 390 der 400 resistenten Klone für D-Cycline kodierten. Zwei der verbleibenden Klone ("Nummer 163") kodierten für das gleiche Protein: sie wurden komplett sequenziert.

Aus einer aus Balb/c-3T3 Zellen hergestellten λZAP-cDNA Bibliothek wurden mehrere Klone identifiziert, die homolog zu p163 waren. Die Sequenzanalyse zeigt einen offenen Leserahmen; dieser wird auch in der VP16-Chimäre aus der cDNA-Bibliothek benutzt. Beide Originalklone kodieren für Aminosäure 121 bis 252 dieses Leserahmens. Die Klone p163 wurden mit β-Galaktosidase als Reportergen in Hefe näher charakterisiert:

Wie in Tabelle 1 dargestellt, interagiert das kodierte Protein in Hefe spezifisch mit p27, nicht mit anderen Proteinen wie Myc, Max, Bcl-6 oder Prothymosin-α. Tabelle 1 zeigt auch eine erste Eingrenzung der Domänen von p27, in denen die Interaktion mit p163 stattfindet. Diese Daten weisen darauf hin, daß p163 an die gleichen Domänen von p27 wie cyclinabhängige Kinasen (Russo et al., Nature 382, 325 (1996)) bindet. Dies ist ein Hinweis, daß p163 und cyclinabhängige Kinasen um die Bindung an p27 kompetieren. Zum anderen konnte die Domäne von p163, die mit p27 interagiert, auf die Aminosäure 121 bis 252 eingegrenzt werden.

Zur Bestätigung dieser Ergebnisse aus diesem Hefetestsystem wurde rekombinant exprimiertes p163 [als Fusionsprotein mit Glutathiontransferase (GST) exprimiert] an eine Säule gebunden und dann versucht, ³⁵S- markiertes p27 Protein-affinitätschromatographisch an dieser Säule zu reinigen. Durch die Verwendung des Fusionsproteins p163 mit einer Glutathiontransferase (GST) war es möglich, eine einheitliche Matrix für diese Experimente einzusetzen (sogenannte "GST-pulldowns"; Hateboer et al., Proc. Natl. Acad. Sci. USA 90, 8489 (1993)).

Für diese Untersuchungen wurde der Leserahmen aus dem Hefeklon in einen pGEX-Vektor (Pharmacia, Freiburg pGEX-2T; Best. Nr. 27-4801-01) umgesetzt, der für ein chimäres Protein mit Glutathion-S-Transferase (GST) unter der Kontrolle des IPTG-induzierbaren TAC-Promotors in E.coli kodiert (Smith and Johnson, Gene 67: 31, 1988). Chimäre Proteine wurden aus induzierten E.coli-Kulturen durch Affinitätschromatographie an Glutathion-Agarose gereinigt (Smith and Johnson, 1988) und anschließend gegen 100 mM Tris-HCl, Ph7,5, 100 mM KCl, 20 mM EDTA, 10% glycerol, 0,1 mM PMSF dialysiert. Als Kontrolle wurde Glutathion-S-Transferase nach dem gleichen Protokoll gereinigt und dialysiert: die gereinigten Proteine wurden bei -80°C gelagert.

Jeweils 10 µg der beiden Proteine wurden anschließend mit 10 µg BSA und 20 mg gequollener GST-Agarose für zwei Stunden bei 4°C inkubiert; daraufhin wurde die Agarose abzentrifugiert und zweimal mit Dialyse-Puffer gewaschen. Als Kontrolle für die Bindung der Proteine wurde ein Aliquot der Agarose direkt in Probenpuffer aufgekocht und die gebundenen Proteine nach einer SDS-Gelelektrophorese nachgewiesen. ³⁵S-Methionin-markiertes p27 wurde durch in vitro Translation (nach Angaben des Herstellers: Promega, Mannheim; TNT coupled retc. lys. systems; L 4610) hergestellt und zwei Stunden in einem Gesamtvolumen von 200 µl in Dialysepuffer mit 0.1% NP-40 bei 4°C mit beladener Agarose inkubiert. Die Agarose wurde viermal mit Dialysepuffer/0,1% NP-40 gewaschen und anschließend in SDS-Probenpuffer aufgekocht. Gebundene Proteine wurden durch SDS-Gelelektrophorese und Fluorographie nachgewiesen.

In Tabelle 2 sind die Ergebnisse dieser Versuche dargestellt. Sie belegen, daß rekombinantes, in vitro synthetisiertes, ³⁵S-markiertes p27 selektiv an eine Säule, die mit einem chimären GST-p163 Fusionsprotein beladen ist, nicht aber an eine Säule, die mit der gleichen Menge GST beladen ist, bindet. Tabelle 2 belegt auch, daß p27 aus einem Zellextrakt ineffizient an GST-p163 bindet; in diesem Extrakt ist p27 an Komplexe cyclinabhängiger Kinasen gebunden. Eine kurze Hitzebehandlung setzt p27 aus diesen Komplexen frei und erlaubt dann die Bindung an p163. Dies belegt, daß p163 und cyclinabhängige Kinasen um die Bindung an p27 kompetieren.

Die erfindungsgemäße Nukleotidsequenz, welche für das murine Protein p163 kodiert, ist in Tabelle 3 dargestellt. Diese Sequenz hat ein Homolog in der Datenbank; es handelt sich um das Nukleoprotein (Nup)2 Gen der Hefe. Nup2 ist ein Protein der Kernmembran, das an der Bildung von Kernprotein und am Transport von Proteinen in und aus dem Kern beteiligt ist. Nup2 ist in der Hefe wohl vor allem am Export beteiligt.

Mehrere funktionelle Domänen sind konserviert, obwohl insgesamt die Homologie nicht hoch ist (Tabelle 4). So ist die Bindestelle für ein regulatorisches Protein (Ran, ein GTP-bindendes Protein), das den Kerntransport reguliert, zwischen Ran binding protein (RBP-1), NuP-2 und p163 konserviert (Tabelle 5), wie auch kurze Pentapeptidsequenzen, denen strukturelle Funktion zugesprochen wird (Tabelle 6).

Um nachzuweisen, daß es sich bei p163 zweifellos um ein Nukleoporin handelt, wurde ein polyklonales Antiserum gegen das Protein [Histidin-p163 (aa 121-252)] erzeugt und affinitätsgereinigt; dieses Antiserum erkennt fraglos ein Nukleoporin, was in der Immunfluoreszens an der typischen Kernporenfärbung ("dots" auf der Oberfläche und die Kernperipherie ist gefärbt) erkennbar ist.

Zum Nachweis einer intrazellulären Assoziation zwischen p163 oder Nup2 und p27 wurden beide Proteine in HeLa-Zellen transient mit CMV-Expressionsvektoren exprimiert, 48 Stunden später die Zellen lysiert und auf eine mögliche Interaktion beider Proteine durch Immunpräzipitation/Western Blots untersucht (Peukert et al., EMBO J. 16, 5672 (1997)). Die Assoziation zwischen p27 und Nup2 wurde mit je einem polyklonalen Antikörper gegen Nup2 und gegen murines p27 nachgewiesen. Das Protein p27 wurde nur dann gut präzipitiert, wenn p163 exprimiert wurde (siehe Tabelle 7). Dies ist ein Nachweis, daß Nup2 und p27 in HeLa-Zellen in vivo miteinander assoziieren können.

Durch Sequenzvergleich und Datensuche mit humanen ESTs wurde die Nukleinsäuresequenz für das humane p163 Protein identifiziert. Für die Ran Bindedomäne liegt sie in der Sequenz AA161285 in den Positionen 173 ≥ 575, für die p27 Bindedomäne in der Sequenz R62312 in den Positionen 318 ≥ 486, für andere Teilabschnitte von p163 in der Sequenz THC199124 in den Positionen 348 ≥ 489.

Um genauer zu definieren, mit welchen Aminosäuren von p27 die Interaktion von Nup2-p163 stattfindet, wurde in Hefe nach Mutanten gesucht, die nicht mehr mit p163 interagieren. Dazu wurde die cDNA, kodierend für p27 einer fehlerinduzierenden PCR-Reaktion (PCR-Bedingungen gemäß "PCR-technology, Principles and Applications for DNA amplifications"; H.A. Erlich, Stockton press, NY 1989) unterzogen und die resultierenden Klone, die zufällige Fehler enthalten, auf eine Interaktion mit Nup2 und, als Kontrolle, mit Cyclin D1 getestet. Die (vom Standard abweichenden) Bedingungen dieser PCR waren: 40 Zyklen in Gegenwart von 1U GIBCO-Taq Polymerase und von 0.1 mM MnCl₂.

Das erhaltene cDNA-Fragment wurde mit einem pGBT10-Vektor, der mit BamHI und EcoRI linearisiert und gereinigt worden war, in den Hefestamm HF7c-p163/Nup2 cotransformiert. Transformierte Kolonien wurden durch Selektion aus -Leu-Trp Mangelmedium selektioniert. 960 einzelne Klone wurden isoliert und auf selektives Medium (-Leu-His-Trp) plattiert. Mit Klonen, die auf diesem selektiven Medium nicht wuchsen, wurde ein β-Galaktosidase Test als zweiter Interaktionstest durchgeführt. Negative Klone wurden identifiziert und aus ihnen das pGBT10-p27 Plasmid isoliert. Die gewonnenen Plasmide wurden erstens mit einem Hefe-Expressionsplasmid retransformiert, der p163/Nup2 als Chimäre mit einer transaktivierenden Domäne exprimiert, und zweitens als Kontrolle mit einem Plasmid, das Cyclin D1 als Chimäre mit einer transaktivierenden Domäne exprimiert. Drei Klone wurden erhalten, die selektiv nicht mehr mit Nup2, wohl aber mit Cyclin D1 interagieren. Diese Plasmide wurden zusammen mit einigen Kontrollen sequenziert.

Eine Auswahl der erhaltenen Phänotype ist in Tabelle 13 zusammengefaßt. In diesen Versuchen zeigt sich eine Interaktion als Wachstum in der Abwesenheit von Histidin(-Trp-Leu-His, "selektiv"), während die Kontrolle (-Trp-Leu; "nicht selektiv") zeigt, daß beide Proteine in Hefe exprimiert wurden. Aus 1000 Klonen wurden 3 gefunden, die spezifisch nicht mehr mit p163-Nup2, wohl aber mit Cyclin D1 interagieren. Die Sequenzen sind in Tabelle 14 gezeigt. Alle drei Klone, nicht aber eine Reihe von Kontrollklonen, tragen die gleiche Mutation in der Aminosäure Arginin 90 (mutiert zu Glycin). Die Sequenzen zeigen auch, daß die Klone unabhängig entstanden sind, denn sie tragen weitere, unterschiedliche Mutationen. Damit ist Arginin 90 eindeutig als zentrale Aminosäure in der Wechselwirkung von p27 mit Nup2 definiert. Die Mutanten stehen für eine Validierung der biologischen Relevanz dieser Interaktion zur Verfügung.

### 2) Nukleotidsequenzen für dominant negativ wirkende Analoga des p163 Proteins

Durch die oben beschriebenen Funktionsanalysen sind zwei Domänen des p163 Proteins bekannt, welche für seine Funktion entscheidend sind.

Zum einen ist dies die Domäne (Aminosäuren 121-252) zur Bindung an p27. Zum anderen ist dies die Domäne (Aminosäuren 307-467) zur Bindung an Ran, einem GTP-bindenden Protein, das Transportfunktionen reguliert.

Gegenstand der Erfindung sind somit Nukleinsäuresequenzen für das Protein p163 oder für Teile dieses Proteins, wobei diese Nukleinsäuresequenzen Mutationen in der die p27 bindenden und/oder der die Ran bindenden Domäne aufweisen, so daß die Bindung des exprimierten mutierten Proteins entweder an p27 oder an Ran drastisch verringert ist. Eingeführt in eine Zelle inhibieren derartige Analoga von p163 das funktionsfähige zelleigene p163. Hierdurch entsteht freies p27, was zur Inhibition der Zellteilung führt. Beispiele für derartige dominant negative Mutanten von p163 sind in den Tabellen 8 und 9 dargestellt. Tabelle 8 zeigt p163 mit einer Deletion der p27 bindenden Domäne, Tabelle 9 zeigt p163 ohne die Ran-bindende Domäne.

Gegenstand der Erfindung ist somit die Einführung von Nukleinsäurekonstrukten kodierend für dominant negative p163 Analoga in eine Zelle mit dem Ziel, die Proliferation dieser Zelle zu inhibieren. Diese Einführung in die Zelle kann in in vitro aber auch in vivo erfolgen.

Zur bestmöglichen und/oder gesteuerten Expression des erfindungsgemäßen Nukleinsäurekonstruktes ist dieses vorzugsweise mit einer Aktivierungssequenz verbunden. Beispiele für derartige Aktivierungssequenzen sind im Abschnitt 8.3. aufgeführt. Zur Lokalisation im Kern ist der Nukleinsäuresequenz kodierend für p163 ein nukleäres Lokalisationssignal (NLS) anzufügen. Derartige NLS sind dem Fachmann bekannt.

Das Nukleinsäurekonstrukt wird in die Zelle als nackte DNA oder mit Hilfe eines nichtviralen Vektors oder mit Hilfe und eingefügt in einen viralen Vektor nach der dem Fachmann bekannten Verfahren eingebracht.

### 3) Nukleotidsequenzen für Proteine oder Peptide, die an die Bindedomäne von p163 binden

Durch die vorgelegten Untersuchungen konnte die p27 Bindedomäne des p163 Proteins auf die Aminosäuren 121-252 und die Ran Bindedomäne auf die Aminosäuren 307-467 festgelegt werden.

Gegenstand der Erfindung sind somit Nukleinsäurekonstrukte, die für Peptide kodieren, welche entweder an die Bindedomäne des p163 für das p27 binden und hierdurch die Bindung von p163 an p27 inhibieren oder an die Bindedomäne des p163 für das Ran binden und hierdurch die Bindung von p163 an Ran inhibieren. Durch diese Inhibition entsteht freies p27, welches durch Bindung beispielsweise an Cyclin E/cdk2 die Zellproliferation inhibiert. Ein Beispiel für ein derartig inhibierendes Peptid sind Aminosäuresequenzen, welche mit der p163 bindenden Domäne des p27 Proteins (Aminosäuren 69-178) oder des Ran korrespondieren.

Ein weiteres Beispiel sind Antikörper oder Antikörperfragmente wie F(ab)₂, Fab, Fv oder s.c. Fv, welche an p163 binden, im besonderen an die p27 Bindedomäne oder an die Ran Bindestelle von p163. Derartige Antikörper können beispielsweise durch Immunisierung von Tieren mit p163 oder der p27-bindenen Domäne oder Ran-bindenden Domäne von p163 gewonnen werden. Diese Domänen sind in Tabelle 5 (Ran-bindende Domäne) oder in Tabelle 10 (p27-bindende Domäne) dargestellt.

Gegenstand der Erfindung ist somit die Einführung von Nukleinsäurekonstrukten kodierend für an p163 bindende Proteine in eine Zelle mit dem Ziel, die Proliferation dieser Zelle zu inhibieren. Diese Einführung in die Zelle kann in in vitro aber auch in vivo erfolgen.

Zur bestmöglichen und/oder gesteuerten Expression des erfindungsgemäßen Nukleinsäurekonstruktes ist dieses vorzugsweise mit einer Aktivierungssequenz verbunden. Beispiele für derartige Aktivierungssequenzen sind im Abschnitt 8.3. aufgeführt. Zur Lokalisation im Kern ist der Nukleinsäuresequenz kodierend für das erfindungsgemäße Nukleinsäurekonstrukt ein nukleäres Lokalisationssignal (NLS) anzufügen. Derartige NLS sind dem Fachmann bekannt.

Das Nukleinsäurekonstrukt wird in die Zelle als nackte DNA oder mit Hilfe eines nichtviralen Vektors oder mit Hilfe und eingefügt in einen viralen Vektor nach der dem Fachmann bekannten Verfahren eingebracht.

### 4) Nukleotidsequenzen für p163 zur Stimulation der Zellteilung

Die Einführung der Nukleotidsequenz für p163 oder des Proteins p163 in eine Zelle führt zur Inhibition des intrazellulären p27. Hierdurch werden Cyclin E/cdk2 Komplexe (die durch p27 inhibiert werden) frei, die die Zellteilung initiieren.

Gegenstand der Erfindung ist somit die Einführung von Nukleinsäurekonstrukten kodierend für p163 in eine Zelle mit dem Ziel, die Proliferation dieser Zelle zu fördern. Diese Einführung in die Zelle kann in in vitro aber auch in vivo erfolgen.

Zur bestmöglichen und/oder gesteuerten Expression des erfindungsgemäßen Nukleinsäurekonstruktes ist dieses vorzugsweise mit einer Aktivierungssequenz verbunden. Beispiele für derartige Aktivierungssequenzen sind im Abschnitt 8.3. aufgeführt. Zur Lokalisation im Kern ist der Nukleinsäuresequenz kodierend für p163 ein nukleäres Lokalisationssignal (NLS) anzufügen. Derartige NLS sind dem Fachmann bekannt.

Das Nukleinsäurekonstrukt wird in die Zelle als nackte DNA oder mit Hilfe eines nichtviralen Vektors oder mit Hilfe und eingefügt in einen viralen Vektor nach der dem Fachmann bekannten Verfahren eingebracht.

Durch Einführung eines Nukleinsäurekonstruktes kodierend für p163 können beispielsweise Zellen in der Zellkultur oder Zellen in vivo, z.B. bei mangelhafter Zellproliferaton, zur Proliferation angeregt werden.

### 5) Nukleotidsequenzen zur Hemmung der Transkription und/oder Translation des p163 Proteins

Die Kenntnis der Nukleinsäuresequenz des p163 Proteins bietet die Möglichkeit, Oligonukleotide herzustellen mit Hilfe derer die Transkription oder Translation von p163 spezifisch inhibiert werden kann. Zu diesen Antisense-Nukleinsäuren gehören Oligonukleotide, antisense (Triplex-)DNA, Ribozyme oder antisense RNA. Die Methode der Herstellung von Triplex-DNA-Oligonukleotiden ist ausführlich von Frank-Kamenetskii und Mirkin, Ann. Rev. Biochem. 64, 65 (1995) und die Methode der antisense RNA-Oligonukleotidherstellung von Neckers et al., Crit. Rev. Oncogen. 3, 175 (1992); Carter und Lemoine, Br. J. Cancer 67, 869 (1993); Mukhdopadhyay und Roth, Crit. Rev. Oncogen. 7, 151 (1996) und Mercola und Cohen, Cancer Gene Ther. 2 , 47 (1995) beschrieben worden.

Bevorzugt im Sinne der Erfindung sind Triplex-DNA oder antisense RNA-Oligonukleotide zu verwenden, welche mit Nukleotidsequenzen des p163 hybridisieren, welche für Teilbereiche der Bindedomäne (Gesamtbereich Aminosäure 121-252) für das p27 Protein oder für Teilbereiche der Bindedomäne (Gesamtbereich Aminosäure 307-467) für das Ran Protein kodieren.

Zu den Nukleotidsequenzen, welche die Transkription und/oder die Translation von p163 hemmen, gehören desweiteren Ribozyme spezifisch für Nukleotidsequenzbereiche des p163 Proteins.

Die Methode der Herstellung von Ribozymen wurde ausführlich von Burke, Nucl. Acids Mol. Biol 8, 105 (1994), Christoffersen und Marr, J. Med. Chem 38, 2023 (1995) und Scott et al., Science 274, 2065 (1996) beschrieben. Bevorzugt im Sinne der Erfindung sind Ribozyme, die mit Nukleotidsequenzen des p163 hybridisieren, die im Bereich der Nukleotidsequenz für die Bindedomäne (Aminosäuren 121-252) für das p27 Protein oder für die Bindedomäne (Aminosäure 307-467) für das Ran Protein liegen.

Beispiele für durch Ribozyme spaltbare Nukleotidsequenzen des p163 Proteins sind in Tabelle 11 aufgeführt.

Triplex-DNA, antisense RNA oder Ribozyme werden als Oligonukleotide [hergestellt und gegen den Abbau durch DNAsen bzw. RNAsen geschützt nach den dem Fachmann bekannten Methoden (siehe die oben zitierte Literatur)] entweder in vitro zu den Zielzellen gegeben oder in vivo durch Injektion oder lokale Applikation verabreicht.

Gegenstand der Erfindung ist jedoch auch die Einführung von Nukleinsäurekonstrukten in eine Zelle mit dem Ziel, daß sich in der Zelle durch Transkription die antisense RNS oder Ribozyme entsprechend den erfindungsgemäßen Oligonukleotide entstehen, um die Proliferation dieser Zelle zu inhibieren. Die Einführung in die Zelle kann in vitro aber auch in vivo erfolgen.

Zur bestmöglichen und/oder gesteuerten Expression des erfindungsgemäßen Nukleinsäurekonstruktes ist dieses vorzugsweise mit einer Aktivierungssequenz verbunden. Beispiele für derartige Aktivierungssequenzen sind im Abschnitt 8.3. aufgeführt.

Das Nukleinsäurekonstrukt wird in die Zelle als nackte DNA oder mit Hilfe eines nichtviralen Vektors oder mit Hilfe und eingefügt in einen viralen Vektor nach der dem Fachmann bekannten Verfahren eingebracht.

### 6) Testsysteme zur Auffindung von Inhibitoren der Funktion von p163

Die Entdeckung des p163 Proteins und seiner Wechselwirkung mit dem p27 Protein ermöglicht die Suche nach Inhibitoren dieser Wechselwirkung. Hierfür sind Testsysteme vonnöten, bei welchen die Bindung zwischen dem p27 Protein und dem p163 Protein gehemmt wird und diese Hemmung durch einen Indikator angezeigt wird.

Zahlreiche Methoden sind bekannt. Ein Beispiel für diese Methode ist der "Two Hybrid Screen Assay" (siehe hierzu Abschnitt C.1. und Tabelle 1).

Alternativ kann jedoch auch beispielsweise ein Affinitätssystem zum Screening verwendet werden, bei welchem p163 [bevorzugt die p27 bindenden Domäne (Aminosäure 121-252)] an eine feste Phase gebunden ist, mit der Prüfsubstanz inkubiert wird und die Bindung der Prüfsubstanz durch Hemmung der Bindung eines markierten Bindungspartners für die p27 bindende Domäne des p163 Proteins ermittelt wird. Dieser markierte Bindungspartner kann p27 sein oder ein an die p27 bindende Domäne des p163 Proteins spezifisch bindender Antikörper.

In gleicher Weise wie für Inhibitoren der Bindung zwischen p163 und p27 können auch Testsysteme für Inhibitoren der Bindung zwischen p163 und Ran aufgebaut und für das Screening genutzt werden. Gegenstand der Erfindung ist somit die Verwendung von Nukleinsäuresequenzen kodierend für p163 oder Teilproteinen von p163 oder die Verwendung des p163 Proteins oder Teilproteinen hiervon für die Suche nach Inhibitoren für p163.

### 7) Verwendung der Nukleinsäuresequenz für p163 oder der Proteinsequenz p163 zur Diagnose des Proliferationsstadiums einer Zelle oder eines Krankheitszustandes

Wie in der Einführung Abschnitt A) dargestellt, zeichnen sich eine Reihe von Tumoren durch eine erhöhte intrazelluläre Konzentration von p27 aus. Aufgrund der Tatsache, daß diese Tumoren proliferieren ist anzunehmen, daß die Funktion dieses erhöhten p27 gehemmt ist. Entsprechend dieser Erfindung ist p163 der spezifische Inhibitor von p27. Der Nachweis dieses Inhibitors in einer Zelle erlaubt eine Aussage über den Proliferationszustand der Zelle. Diese Aussage kann z.B. zur Beurteilung der Malignität einer Tumorzelle oder eines Tumorgewebes von großer Wichtigkeit sein. Durch Zelltod wird das Protein p163 freigesetzt. Der Nachweis von p163 in einer Körperflüssigkeit erlaubt also die Aussage über proliferative und/oder mit Zelltod einhergehende, beispielsweise entzündliche Prozesse in einem Körper. Der Nachweis des Proteins p163 kann durch spezifische Bindung an markierte Substanzen erfolgen. Zu diesen spezifisch bindenden Substanzen gehören das p27 Protein, das Ran Protein und Antikörper, beispielsweise erzeugt durch Immunisierung von Tieren mit dem Protein p163 oder mit Teilsequenzen dieses Proteins.

Der Nachweis der p163-mRNA in einer Zelle oder einem Gewebe kann jedoch auch erfolgen durch Hybridisierung der das Protein p163 kodierenden Nukleinsäuresequenz mit der korrespondierenden mRNA. Eine Erhöhung der Sensitivität dieses Nachweises ist durch die dem Fachmann geläufige "Polymerase Chain Reaction" (PCR)-Technologie möglich, bei welcher wenige Kopien einer nachzuweisenden Nukleinsäuresequenz mit Hilfe sogenannter Primer-Paare vermehrt werden können.

Beispiele für Primer-Paare zur Vermehrung und zum Nachweis der Nukleinsäuresequenz kodierend für p163 sind in den Tabellen 12a, b und c dargestellt.

Der Nachweis der RNA kodierend für p163 ist jedoch auch möglich beispielsweise mit der von Gussoni et al., Nature Biotechnol. 14, 1012 (1996) publizierten Fluoreszenz in situ Hybridisierung.

Gegenstand der Erfindung ist somit der Nachweis einer Nukleinsäuresequenz kodierend für p163 oder des Proteins p163 für die Ermittlung des Proliferationszustandes einer Zelle oder eines Gewebes oder dem Nachweis von proliferativen oder mit Zelltod einhergehenden Veränderungen in einem lebenden Säuger.

### 8) Besonderheiten der Expressionssysteme, die durch p163, p27 oder den Komplex aus p163/p27 gesteuert werden

### 8.1. die Komponente b)

### 8.1.1. Bindesequenz für p163, p27 oder den p163/p27 Proteinkomplex [Komponente b₂)]

In einer bevorzugten Ausführungsform der Erfindung enthält das Expressionssystem mit seiner Komponente b₂) mindestens eine Nukleinsäuresequenz für das p163 Protein oder für denjenigen Teil des p163 Proteins, welcher an das Protein p27 bindet.

In einer weiteren bevorzugten Ausführungsform enthält das Expressionssystem mit seiner Komponente b₂) mindestens eine Nukleinsäuresequenz für einen Antikörper oder einen Teil eines Antikörpers mit den Bindesequenzen (VH und VL)
― für die Bindestelle des Proteins p27 für das p163 Protein, oder
― für die Bindestelle des Proteins p163 für das p27 Protein.

In diesen Ausführungsformen bindet z.B. in der Zelle vorhandenes freies p27 Protein oder freies p163 Protein an das Expressionsprodukt der Komponente b₂) und inhibiert damit den Transkriptionsfaktor kodiert von Komponente b) und blockiert damit das Expressionssystem. Ist jedoch beispielsweise in einer Zelle das Protein p27 mit dem Protein p163 komplexiert, so bleibt das Expressionsprodukt der Komponente b₂) und damit der Transkriptionsfaktor [Komponente b)] frei und das Expressionssystem ist funktionsfähig.

Diese erfindungsgemäßen Expressionssysteme sind somit in sich teilenden Zellen (Anwesenheit von p163/p27 Komplexen) funktionsfähig und in ruhenden Zellen (freies p27) inhibiert.

In einer weiteren besonderen Ausführungsform enthält das Expressionssystem mit seiner Komponente b₂) mindestens eine Nukleinsäuresequenz für einen Antikörper oder einen Teil eines Antikörpers mit den Bindesequenzen (VH und VL) für den Komplex aus p27 und p163. In dieser Ausführungsform binden Komplexe von p27 und p163 an das Expressionsprodukt von Komponente b₂) und blockieren damit das Expressionssystem, während freies p27 oder p163 nicht an das Expressionsprodukt der Komponente b₂) bindet.

Dieses erfindungsgemäße Expressionssystem ist somit in sich teilenden Zellen (Anwesenheit von p163/p27 Komplexen) blockiert und in ruhenden Zellen (freies p27) funktionsfähig.

Bei Wahl eines Antikörpers für Komponene b₂) sind bevorzugt die epitopbindenen Teile des Antikörpers FV_{L} FV_{H} einzusetzen, bei murinem Ursprung in humanisierter Form. Die Humanisierung erfolgt in der von Winter et al. (Nature 349, 293 (1991) und Hoogenbooms et al. (Rev. Tr. Transfus. Hemobiol. 36, 19 (1993)) dargestellten Weise. Die Antikörperfragmente werden entsprechend dem Stand der Technik hergestellt, beispielsweise in der von Winter et al., Nature 349, 293 (1991), Hoogenboom et al., Rev. Tr. Transfus. Hemobiol. 36, 19 (1993), Girol. Mol. Immunol 28, 1379 (1991) oder Huston et al., Int. Rev. Immunol. 10, 195 (1993) beschriebenen Weise. Eine detaillierte Beschreibung der Herstellung von Antikörpern, Antikörperfragmenten und rekombinanten Antikörperfragmenten erfolgte in der Patentanmeldung DE 96 49 645.4.

Rekombinante Antikörperfragmente werden direkt aus existierenden Hybridomen hergestellt oder werden mit Hilfe der "phage display"-Technologie aus Bibliotheken muriner bzw. humaner Antikörperfragmente isoliert (Winter et al., Annu. Rev. Immunol. 12, 433 (1994)). Diese Antikörperfragmente werden dann auf genetischer Ebene direkt für die Kopplung mit den Komponenten b₁) und b₃) eingesetzt.

Zur Herstellung von rekombinanten Antikörperfragmenten aus Hybridomen wird die genetische Information, die für die antigenbindenden Domänen (V_{H}, V_{L}) der Antikörper kodiert, durch Isolierung der mRNA, die reverse Transkription der RNA in cDNA und die anschließende Amplifikation mittels Polymerasekettenreaktion und Oligonukleotiden komplementär zu den 5'- bzw. 3'-Enden der variablen Fragmente gewonnen. Die so erhaltenen DNA-Fragmente kodierend für die V_{H}- und V_{L}-Fragmente werden dann in bakterielle Expressionsvektoren kloniert und so können z.B. Fv-Fragmente, einzelkettige Fv-Fragmenten (scFv) oder Fab-Fragmente exprimiert werden.

Neue Antikörperfragmente können mittels der "phage display"-Technologie auch direkt aus Antikörperbibliotheken (Immunbibliotheken, naive Bibliotheken) murinen oder humanen Ursprungs isoliert werden. Beim "phage display" von Antikörperfragmenten werden die antigenbindenden Domänen als Fusionsproteine mit dem Gen des Hüllproteins g3P filamentöser Bakteriophagen entweder in das Phagengenom oder in Phagemid-Vektoren in Form von scFv-Fragmentgenen oder als Fab-Fragmentgene kloniert. Antigen-bindende Phagen werden an antigenbeladenen Plastikgefäßen (panning), an antigenkonjugierten, paramagnetischen "beads" oder durch Bindung an Zellobeflächen selektioniert.

Immunbibliotheken werden hergestellt durch PCR-Amplifikation der Gene der variablen Antikörperfragmente aus B-Lymphozyten immunisierter Tiere oder Patienten. Dazu werden Kombinationen von Oligonukleotiden, die spezifisch sind für murine oder humane Immunglobuline bzw. für die humanen Immunglobulin-Genfamilien verwendet.

Unter Verwendung nichtimmunisierter Spender als Quelle der Immunglobulingene lassen sich naive Bibliotheken herstellen. Alternativ können Immunglobulin-Keimbahngene zur Herstellung semisynthetischer Antikörperrepertoires eingesetzt werden, wobei die Komplementarität-bestimmende Region 3 der variablen Fragmente durch PCR mit Hilfe degenerierter Primer ergänzt wird. Diese sogenannten "single pot"-Bibliotheken haben gegenüber Immunbibliotheken den Vorteil, daß Antikörperfragmente gegen eine Vielzahl von Antigenen aus einer einzigen Bibliothek isoliert werden können.

Die Affinität von Antikörperfragmenten kann mittels der "phage display"-Technologie weiter erhöht werden, wobei neue Bibliotheken von bereits existierenden Antikörperfragmenten durch zufällige, kodonbasierende oder gezielte Mutagenese, durch "chain shuffling" einzelner Domänen mit Fragmenten aus naiven Repertoires oder unter Zuhilfenahme von bakteriellen Mutatorstämmen hergestellt werden und durch Reselektion unter stringenten Bedingungen Antikörperfragmente mit verbesserten Eigenschaften isoliert werden. Zusätzlich können murine Antikörperfragmente durch stufenweisen Austausch einer der variablen Domänen gegen ein humanes Repertoire und anschließende Selektion mit dem ursprünglichen Antigen ("guided selection") humanisiert werden. Alternativ erfolgt die Humanisierung muriner Antikörper durch zielgerichteten Austausch der hypervariablen Regionen humaner Antikörper durch die korrespondierenden Regionen des originalen murinen Antikörpers.

### 8.1.2. Die Aktivierungsdomäne [Komponente b₁)] und die DNA-Bindedomäne [Komponente b₃)]

Im Sinne der Erfindung können alle verfügbaren Gene von Aktivierungsdomänen und DNA-Bindedomänen eines Transkriptionsfaktors für die Komponente b) verwendet werden. Beispiele hierfür, deren Beschreibung die Erfindung jedoch nicht einschränken soll, sind:
- Aktivierungdomänen [Komponente b₁)]
   mindestens eine Sequenz
   - der cDNA für die saure Transaktivierungsdomäne (TAD) von HSV1 -VP16 (Aminosäuren 406 bis 488; Triezenberg et al., Genes Developm. 2: 718 (1988); Triezenberg, Curr. Opin. Gen. Developm. 5: 190 (1995) oder Aminosäuren 413 bis 490; Regier et al., Proc. Natl. Acad. Sci. USA 90, 883 (1993)) oder
   - der Aktivierungsdomäne von Oct-2 (Aminosäuren 438 bis 479; Tanaka et al., Mol. Cell. Biol. 14: 6046 (1994) oder Aminosäuren 3 bis 154; Das et al., Nature 374: 657 (1995)) oder
   - der Aktivierungsdomäne von SP1 (Aminosäuren 340 bis 485; Courey und Tijan, Cell 55, 887 (1988)) oder
   - der Aktivierungsdomäne von NFY (Aminosäuren 1 bis 233; Li et al., J. Biol. Chem. 267, 8984 (1992); van Hujisduijnen et al., EMBO J. 9, 3119 (1990); Sinha et al., J. Biol. Chem. 92, 1624 (1995); Coustry et al. J. Biol. Chem. 270, 468 (1995)) oder
   - der Aktivierungsdomäne von ITF2 (Aminosäuren 2 bis 452; Seipel et al., EMBO J 13, 4961 (1992)) oder
   - der Aktivierungsdomäne von c-Myc (Aminosäuren 1 bis 262; Eilers et al.) oder
   - der Aktivierungsdomäne von CTF (Aminosäuren 399 bis 499; Mermod et al., Cell 58, 741 (1989); Das und Herr, Nature 374, 657 (1995))
- DNA-Bindedomänen [Komponente b₃)]
   mindestens eine Sequenz
   - der cDNA für die DNA-Bindedomäne des Gal4-Proteins (Aminosäuren 1 bis 147; Chasman und Kornberg, Mol. Cell. Biol. 10: 2916 (1990)) oder
   - des LexA-Proteins (Aminosäuren 1 bis 81; Kim et al., Science 255: 203 (1992) oder das ganze LexA-Protein (Aminosäuren 1 bis 202; Brent et al., Cell 43: 729 (1985)) oder
   - des lac-Repressor (lac I) Proteins (Brown et al., Cell 49: 603 (1987); Fuerst et al., PNAS USA 86: 2549 (1989)) oder
   - des Tetracyclin-Repressor(tet R)-Proteins (Gossen et al., PNAS USA 89; 5547 (1992); Dingermann et al., EMBO J. 11: 1487 (1992)) oder
   - des ZFHD1-Proteins (Pomerantz et al., Science 267: 93 (1995)).

Vorteilhaft im Sinne der Erfindung ist, an das 3' Ende der DNA-Bindedomäne ein nukleares Lokalisationssignal (NLS) anzufügen.

### 8.2. Die durch die Komponente b) aktivierbare Aktivierungssequenz Promotoreinheit II [Komponente c)]

Die Auswahl dieser Aktivierungssequenz richtet sich nach der Wahl der DNA-Bindedomäne [Komponente b₃)] in dem Gen für einen Transkriptionsfaktor [Komponente b)].

Für die unter 8.1.2. aufgeführten Beispiele für die DNA-Bindedomänen bestehen wiederum beispielhaft folgende Möglichkeiten:

### 8.2.1. Möglichkeit A)

― eine Aktivierungssequenz
   mit mindestens einer Bindesequenz [Nukleotidsequenz: 5'-CGGACAACTGTT-GACCG-3', SEQ ID NO.: 1] für das Gal4-Protein (Chasman und Kornberg, Mol. Cell Biol. 10, 2916 (1990)) und (an deren 3' Ende)
   - der basale Promotor von SV40
      (Nukleinsäuren 48 bis 5191; Tooze (ed), DNA Tumor Viruses (Cold Spring Harbor New York, New York; Cold Spring Harbor Laboratory) oder
   - der Promotor von c-fos (Das et al., Nature 374, 657 (1995)) oder
   - der U2 sn RNA-Promotor oder
   - der Promotor von HSV TK (Papavassiliou et al., J. Biol. Chem. 265, 9402 (1990); Park et al., Molec. Endocrinol 7, 319 (1993)) angefügt ist.

### 8.2.2. Möglichkeit B)

― eine Aktivierungssequenz
   - mit mindestens einer Bindesequenz [Nukleotidsequenz 5'-TACTGTATGTACA-TACAGTA-3', SEQ ID NO.: 2] für das LexA Protein [LexA-Operator; Brent et al., Nature 612, 312 (1984)] und (an deren 3' Ende)
   - der basale Promotor von SV40
      (Nukleinsäuren 48 bis 5191; Tooze (ed), DNA Tumor Viruses (Cold Spring Harbor New York, New York; Cold Spring Harbor Laboratory) oder ein anderer Promotor (siehe Möglichkeit A) angefügt ist

### 8.2.3. Möglichkeit C)

― eine Aktivierungssequenz
   - mit mindestens einer Lac-Operator-Bindesequenz (Nukleotidsequenz: 5'-GAATTGTGAGCGCTCACAATTC-3', SEQ ID NO.: 3) für das lac I Repressorprotein (Fuerst et al., PNAS USA 86, 2549 (1989); Simons et al., PNAS USA 81, 1624 (1984)) und (an deren 3' Ende)
   - der basale Promotor von SV40 (Nukleinsäuren 48 bis 5191; Tooze (ed) DNA Tumor Viruses (Cold Spring Harbor New Yor, N.Y., Cold Spring Harbor Laboratory) oder ein anderer Promotor (siehe Möglichkeit A) angefügt ist.

### 8.2.4. Möglichkeit D)

― eine Aktivierungssequenz
   - mit mindestens einer Tetrazyklin-Operator-(tet O)-Bindesequenz (Nukleotidsequenz: 5'-TCGAGTTTACCACTCCCTATCAGTGATAGAGAAAAG-TGAAAG-3', SEQ ID NO.: 4) für das Tetracyclin-Repressor-(tet R)-Protein und (an deren 3' Ende)
   - der basale Promotor von SV40 (Nukleinsäuren 48 bis 5191; Tooze (ed.) DNA Tumor Viruses (Cold Spring Harbor New York, N.Y., Cold Spring Harbor Laboratory) oder ein anderer Promotor (siehe Möglichkeit A) angefügt ist

### 8.2.5. Möglichkeit E)

― eine Aktivierungssequenz
   - mit mindestens einer Bindesequenz [Nukleotidsequenz 5'-TAATGATGGGCG-3', SEQ ID NO.: 5] für das ZFHD-1 Protein (Pomerantz et al., Science 267, 93 (1995)) und (an dessen 3' Ende)
   - der basale Promotor von SV40 (Nukleinsäuren 48 bis 5191; Tooze (ed.), DNA Tumor Viruses (Cold Spring Harbor New York, New York, Cold Spring Harbor Laboratory) oder einen anderen Promotor (siehe Möglichkeit A) angefügt ist.

### 8.3. Die Aktivierungssequenz I [Komponente a)]

Im Sinne der Erfindung sind als Aktivierungssequenzen Nukleotidsequenzen zu verwenden, welche nach Bindung von Transkriptionsfaktoren die Transkription eines am 3'-Ende benachbart gelegenen Genes aktivieren. Die Wahl der Aktivierungssequenz richtet sich nach der zu behandelnden Erkrankung und der zu transduzierenen Zielzelle. So kann die Aktivierungssequenz [Komponente a)] uneingeschränkt, zielzellspezifisch, unter bestimmten metabolischen Bedingungen, zellzyklusspezifisch oder virusspezifisch aktivierbar sein. Eine detaillierte Beschreibung dieser Promotorsequenzen erfolgte bereits in den Patentanmeldungen EP95930524.4, EP95931933.6, EP95931204.2, EP95931205.9 EP97101507.8, EP97102547.3, DE196.39103.2 und DE196.51443.6. Zu den auszuwählenden Promotorsequenzen gehören beispielsweise:

### 8.3.1. uneingeschränkt aktivierbare Promotoren und Aktivatorsequenzen, wie beispielsweise

― der Promotor der RNA-Polymerase III
― der Promotor der RNA-Polymerase II
― der CMV-Promotor und -Enhancer
― der SV40 Promotor

### 8.3.2. virale Promotor- und Aktivatorsequenzen, wie beispielsweise

― HBV
― HCV
― HSV
― HPV
― EBV
― HTLV
― HIV

Bei Verwendung des HIV-Promotors ist die gesamte LTR-Sequenz einschließlich der TAR-Sequenz [Position -453 bis -80, Rosen et al., Cell 41, 813 (1985)] als virusspezifischer Promotor einzusetzen.

### 8.3.3. Metabolisch aktivierbare Promotor- und Enhancersequenzen, wie beispielsweise der durch Hypoxie induzierbare Enhancer.

### 8.3.4. Zellzyklusspezifisch aktivierbare Promotoren

Solche sind beispielsweise der Promotor des cdc25C Gens, des Cyclin A Gens, des cdc2 Gens, des B-myb Gens, des DHFR-Gens, des E2F-1 Gens des cdc25B Genes, oder aber Bindesequenzen für während der Zellproliferation auftretende oder aktivierte Transkriptionsfaktoren. Zu diesen Bindesequenzen gehören beispielsweise Bindesequenzen für c-myc-Proteine. Zu diesen Bindesequenzen sind Monomere oder Multimere der als Myc E-Box bezeichneten Nukleotidsequenz [5'-GGAAGCAGACCACGTGGTCTGCTTCC-3', SEQ ID NO.: 6; Blackwood and Eisenmann, Science 251: 1211 (1991)] zu zählen.

### 8.3.5. Tetrazyklin aktivierbare Promotoren, wie beispielsweise der Tetrazyklin-Operator in Kombination mit einem entsprechenden Repressor.

### 8.3.6. Chimäre Promotoren

Ein chimärer Promotor stellt die Kombination einer stromaufwärts gelegenen zellspezifisch, metabolisch oder virusspezifisch aktivierbaren Aktivatorsequenz mit einem stromabwärts gelegenen Promotormodul dar, welches die Nukleotidsequenz CDE-CHR oder E2FBS-CHR enthält, an welche suppressive Proteine binden, die hierdurch die Aktivierung der stromaufwärts gelegenen Aktivatorsequenz in G₀- und G₁-Phase des Zellzyklus hemmen können (PCT/GB9417366.3; Lucibello et al., EMBO J. 14, 12 (1994)).

### 8.3.7. Zellspezifisch aktivierbare Promotoren

Hierzu zählen bevorzugt Promotoren oder Aktivatorsequenzen aus Promotoren oder Enhancern von solchen Genen, die für Proteine bevorzugt gebildet in ausgewählten Zellen kodieren.

Zum Beispiel sind im Sinne der Erfindung in folgenden Zellen Promotoren für folgende Proteine bevorzugt zu verwenden:

### 8.3.7.1. Promotor- und Aktivatorsequenzen aktiviert in Endothelzellen

― Hirn-spezifischer, endothelialer Glucose-1-Transporter
― Endoglin
― VEGF-Rezeptor-1 (flt-1)
― VEGF-Rezeptor-2 (flk-1, KDR)
― tie-1 oder tie-2
― B61-Rezeptor (Eck-Rezeptor)
― B61
― Endothelin, im speziellen Endothelin B oder Endothelin-1
― Endothelin-Rezeptoren, insbesondere der Endothelin B-Rezeptor
― Mannose-6-Phosphat-Rezeptoren
― von Willebrand Faktor
― IL-1α, IL-1β
― IL-1-Rezeptor
― Vascular Cell Adhesion Molecule (VCAM-1)
― synthetische Aktivatorsequenzen

Als Alternative zu natürlichen endothelzellspezifischen Promotoren lassen sich auch synthetische Aktivatorsequenzen verwenden, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen 5'-TTATCT-3' ist [Lee et al., Biol. Chem. 266, 16188 (1991), Dormann et al., J. Biol. Chem. 267, 1279 (1992) und Wilson et al., Mol. Cell Biol. 10, 4854 (1990)].

### 8.3.7.2. Promotoren oder Aktivatorsequenzen, aktiviert in Zellen in Nachbarschaft aktivierter Endothelzellen

― VEGF
   Die genregulatorischen Sequenzen für das VEGF-Gen sind die 5' flankierende Region, die 3' flankierende Region, das c-Src-Gen oder das v-Src-Gen
― Steroid-Hormonrezeptoren und deren Promotorelemente (Truss und Beato, Endocr. Rev 14, 459 (1993)), insbesondere der Maus-Mammatumor-Virus-Promotor

### 8.3.7.3. Promotoren oder Aktivatorsequenzen aktiviert in Muskelzellen, insbesondere glatten Muskelzellen

― Tropomyosin
― α-Actin
― α-Myosin
― Rezeptor für PDGF
― Rezeptor für FGF
― MRF-4
― Phosphofructokinase A
― Phosphoglyceratemutase
― Troponin C
― Myogenin
― Rezeptoren für Endothelin A
― Desmin
― VEGF
   Die genregulatorischen Sequenzen für das VEGF-Gen sind bereits im Abschnitt "Promotoren aktiviert in Zellen in Nachbarschaft aktivierter Endothelzellen" aufgeführt worden (s.o.)
― "artifizielle" Promotoren
   Faktoren der Helix-Loop-Helix (HLH)-Familie (MyoD, Myf-5, Myogenen, MRF4) sind als muskelspezifische Transkriptionsfaktoren beschrieben. Des weiteren gehören zu den muskelspezifischen Transkriptionsfaktoren das Zinkfingerprotein GATA-4.

Die HLH-Proteine sowie GATA-4 zeigen muskelspezifische Transkription nicht nur mit Promotoren von muskelspezifischen Genen, sondern auch im heterologen Kontext, so auch mit artifiziellen Promotoren. Derartige artifizielle Promotoren sind beispielsweise multiple Kopien der (DNA) Bindestelle für muskelspezifische HLH-Proteine wie der E-Box (Myo D) (z.B. 4x AGCAGGTGTTGGGAGGC, SEQ ID NO.: 7) oder multiple Kopien der DNA Bindestelle für GATA-4 des α-Myosin-Heavy Chain Gens (z.B. 5'-GGCCGATGGGCAGATAGAGGGGGCCGATGGGCAGATAGAGG3', SEQ ID NO.: 8)

### 8.3.7.4. Promotoren und Aktivatorsequenzen, aktiviert in Gliazellen

Hierzu zählen im besonderen die genregulatorischen Sequenzen bzw. Elemente aus Genen, die beispielsweise für folgende Proteine kodieren:
― das Schwannzell-spezifische Protein Periaxin
― Glutaminsynthetase
― das Gliazell-spezifische Protein (Glial fibrillary acid protein = GFAP)
― das Gliazellprotein S100b
― IL-6 (CNTF)
― 5-HT-Rezeptoren
― TNFα
― IL-10
― Insulin-like Growth Factor Receptor I and II
― VEGF

Die genregulatorischen Sequenzen für das VEGF-Gen sind bereits oben aufgeführt worden.

### 8.3.7.5. Promotoren und Aktivatorsequenzen aktiviert in blutbildenden Zellen

Zu solchen genregulatorischen Sequenzen gehören Promotorsequenzen für Gene eines Cytokins oder seines Rezeptors, die in blutbildenden Zellen oder in benachbarten Zellen, wie beispielsweise dem Stroma, exprimiert sind.

Hierzu gehören Promotorsequenzen für beispielsweise folgende Cytokine und ihre Rezeptoren:
― Stem Cell Factor-Receptor
― Stem Cell Factor
― IL-1α
― IL-1-Rezeptor
― IL-3
― IL-3-Rezeptor (α-subunit)
― IL-3-Rezeptor (β-subunit)
― IL-6
― IL-6-Rezeptor
― GM-CSF
― GM-CSF-Rezeptor (α-Kette)
― Interferon Regulatory Factor 1 (IRF-1)
   Der Promotor von IRF-1 wird durch IL-6 gleichermaßen aktiviert wie durch IFNγ oder IFNβ
― Erythropoietin
― Erythropoietin-Rezeptor.

### 7.3.7.6. Promotoren und Aktivatorsequenzen aktiviert in Lymphozyten und/oder Makrophagen

Hierzu gehören beispielsweise die Promotor- und Aktivatorsequenzen der Gene für Cytokine, Cytokinrezeptoren und Adhäsionsmoleküle und Rezeptoren für das Fc-Fragment von Antikörpern.

Hierzu gehören beispielsweise:
― IL-1 -Rezeptor
― IL-1α
― IL-1β
― IL-2
― IL-2-Rezeptor
― IL-3
― IL-3-Rezeptor (α-subunit)
― IL-3-Rezeptor (β-subunit)
― IL-4
― IL-4-Rezeptor
― IL-5
― IL-6
― IL-6-Rezeptor
― Interferon Regulatory Factor 1 (IRF-1)
   (Der Promotor von IRF-1 wird durch IL-6 gleichermaßen aktiviert wie durch IFNγ oder IFNβ).
― IFNγ Responsive Promotor
― IL-7
― IL-8
― IL-10
― IL-11
― IFNγ
― GM-CSF
― GM-CSF-Rezeptor (α-Kette)
― IL-13
― LIF
― Makrophagen-Colony Stimulating Factor (M-CSF)-Rezeptor
― Typ I und II Makrophagen Scavenger Rezeptoren
― MAC-1 (Leukozytenfunktionsantigen)
― LFA-1α (Leukozytenfunktionsantigen)
― p150,95 (Leukozytenfunktionsantigen)

### 8.3.7.7. Promotor- und Aktivatorsequenzen aktiviert in Synovialzellen

Hierzu gehören die Promotorsequenzen für Matrix-Metalloproteinasen (MMP), beispielsweise für:
― MMP-1 (interstitielle Kollagenase)
― MMP-3 (Stromelysin/Transin)

Hierzu gehören des weiteren die Promotorsequenzen für Tissue Inhibitors of Metalloproteinases (TIMP), beispielsweise
― TIMP-1
― TIMP-2
― TIMP-3

### 8.3.7.8. Promotoren und Aktivatorsequenzen aktiviert in Leukämiezellen

Hierzu gehören beispielsweise Promotoren für
― c-myc
― HSP-70
― bcl-1/cyclin D-1
― bcl-2
― IL-6
― IL-10
― TNFα, TNFβ
― HOX-11
― BCR-Abl
― E2A-PBX-1
― PML-RARA (Promyelocytic Leukemia - Retinoic Acid Receptor)
― c-myc
― c-myc-Proteine binden an und aktivieren Multimere der als Myc E-Box bezeichneten Nukleotidsequenz (5'-GGAAGCAGACCACGTGGTCTGCTTCC-3', SEQ ID NO.: 6)

### 8.3.7.9. Promotoren oder Aktivatorsequenzen aktiviert in Tumorzellen

Als Promotor- oder Aktivatorsequenz ist eine genregulatorische Nukleotidsequenz vorgesehen, mit der Transkriptionsfaktoren, gebildet oder aktiv in Tumorzellen, interagieren.

Im Sinne dieser Erfindung zählen zu den bevorzugten Promotoren oder Aktivatorsequenzen genregulatorische Sequenzen bzw. Elemente aus Genen, die besonders in Krebszellen oder Sarkomzellen gebildete Proteine kodieren. So wird bei kleinzelligen Bronchialkarzinomen bevorzugt der Promotor des N-CAM-Proteins, bei Ovarialkarzinomen der Promotor des "Hepatitis growth factor"-Rezeptors oder des L-Plastin und bei Pankreaskarzinomen der Promotor des L-Plastins oder des polymorphen epithelialen Mucins (PEM) verwendet.

### 8.4. Das Effektorgen [Komponente d)]

Im Sinne der Erfindung kodieren die Effektorgene [Komponente d)] für einen Wirkstoff zur Prophylaxe und/oder Therapie einer Erkrankung. Effektorgene und Promotorsequenzen sind im Hinblick auf die Art der Therapie der Erkrankung und unter Berücksichtigung der zu transduzierenden Zielzelle auszuwählen.

Beispielsweise sind bei folgenden Erkrankungen folgende Kombinationen von Promotorsequenzen und Effektorgenen zu wählen (eine detaillierte Beschreibung erfolgte bereits in den Patentanmeldungen EP95930524.4, EP95931933.6, EP95931204.2, EP95931205.9, EP97101507.8, D196.17851.7, D196.39103.2 und D196.51443.6, auf die Bezug genommen wird).

### 8.4.1. Therapie von Tumoren

### 1.1. Zielzellen:

― proliferierende Endothelzellen oder
― der Endothelzelle benachbarte Stromazellen und Muskelzellen oder
― Tumorzellen oder Leukämiezellen

### 1.2. Promotoren:

― endothelzellspezifisch und zellzyklusspezifisch oder
― zellunspezifisch oder muskelzellspezifisch und zellzyklusspezifisch oder
― tumorzellspezifisch (solide Tumoren, Leukämien) und zellzyklusspezifisch

### 1.3. Effektorgene für Inhibitoren der Zellproliferation, zum Beispiel für

― das Retinoblastomprotein (pRb=p110) oder die verwandten p107 und p130 Proteine
   Das Retinoblastomprotein (pRb/p110) und die verwandten p107 und p130 Proteine werden durch Phosphorylierung inaktiviert. Bevorzugt sind solche Gene dieser Zellzyklusinhibitoren zu verwenden, welche Mutationen für die Inaktivierungsstellen der exprimierten Proteine aufweisen, ohne daß diese hierdurch in ihrer Funktion beeinträchtigt werden. Beispiele für diese Mutationen wurden beschrieben für das p110.
   In analoger Weise wird die DNA-Sequenz für das p107 Protein oder das p130 Protein mutiert.
― das p53 Protein
   Das Protein p53 wird in der Zelle inaktiviert entweder durch Bindung an spezielle Proteine, wie beispielsweise MDM2, oder durch Oligomerisierung des p53 über das dephosphorylierte C-terminale Serin. Bevorzugt wird somit eine DNA-Sequenz für ein p53 Protein verwendet, welches C-terminal verkürzt ist um das Serin 392
― das p21 (WAF-1)
― das p16 Protein
― andere cdk-Inhibitoren
― das GADD45 Protein
― das bak Protein
― ein Bindeprotein für ein Regulatorprotein (siehe II.1.)

### 1.4. Effektorgene für Gerinnung induzierende Faktoren und Angiogeneseinhibitoren, zum Beispiel:

― Plasminogenaktivatorinhibitor-1 (PAI-1)
― PAI-2
― PAI-3
― Angiostatin
― Interferone (IFNα, IFNβ oder IFNγ)
― Platelet factor 4
― TIMP-1
― TIMP-2
― TIMP-3
― Leukemia Inhibitory Factor (LIF)
― Tissue Factor (TF) und dessen gerinnungsaktive Fragmente

### 1.5. Effektorgene für zytostatische und zytotoxische Proteine, zum Beispiel für

― Perforin
― Granzym
― IL-2
― IL-4
― IL-12
― Interferone, wie beispielsweise IFN-α, IFNβ oder IFNγ
― TNF, wie TNFα oder TNFβ
― Oncostatin M
― Sphingomyelinase
― Magainin und Magainin-Derivate

### 1.6. Effektorgene für zytostatische oder zytotoxische Antikörper und für Fusionsproteine zwischen antigenbindenden Antikörperfragmenten mit zytostatischen, zytotoxischen oder entzündungserregenden Proteinen oder Enzymen.

― Zu den zytostatischen oder zytotoxischen Antikörpern gehören solche gerichtet gegen Membranstrukturen von Endothelzellen wie sie beispielsweise von Burrows et al. (Pharmac. Ther. 64, 155 (1994)), Hughes et al., (Cancer Res 49, 6214 (1989)) und Maruyama et al., (PNAS USA 87, 5744 (1990)) beschrieben wurden. Insbesondere zählen hierzu Antikörper gegen die VEGF-Rezeptoren.
― Des weiteren gehören hierzu zytostatische oder zytotoxische Antikörper gerichtet gegen Membranstrukturen auf Tumorzellen. Derartige Antikörper wurden zum Beispiel von Sedlacek et al., Contrib. to Oncol. 32, Karger Verlag, München (1988) und Contrib. to Oncol. 43, Karger Verlag, München (1992) übersichtlich dargestellt. Weitere Beispiele stellen dar Antikörper gegen Sialyl Lewis; gegen Peptide auf Tumoren, welche von T-Zellen erkannt werden; gegen von Onkogenen exprimierte Proteine; gegen Ganglioside wie GD3, GD2, GM2, 9-0-acetyl GD3, Fucosyl GM1; gegen Blutgruppenantigene und deren Vorläufer; gegen Antigene auf dem polymorphen epithelialen Mucin; gegen Antigene auf Heat Shock Proteinen
― Des weiteren gehören hierzu gehören Antikörper gerichtet gegen Membranstrukturen von Leukämiezellen. Eine große Anzahl derartiger monoklonaler Antikörper sind bereits für diagnostische und therapeutische Verfahren beschrieben worden (Übersichten bei Kristensen, Danish Medical Bulletin 41, 52 (1994); Schranz, Therapia Hungarica 38, 3 (1990); Drexler et al., Leuk. Res. 10, 279 (1986); Naeim, Dis. Markers 7, 1 (1989); Stickney et al., Curr.. Opin. Oncol. 4, 847 (1992); Drexler et al. Blut 57, 327 (1988); Freedman et al., Cancer Invest. 9, 69 (1991)). Je nach Typ der leukämie sind als Liganden beispielsweise monoklonalen Antikörper oder deren antigenbindende Antikörperfragmente gerichtet gegen folgende Membranantigene geeignet:

| Zellen | Membranantigen |
|---|---|
| AML | CD13 |
| | CD15 |
| | CD33 |
| | CAMAL |
| | Sialosyl-Le |
| B-CLL | CD5 |
| | CD1c |
| | CD23 |
| | Idiotypen und Isotypen der Membranimmunglobuline |
| T-CLL | CD33 |
| | M38 |
| | IL-2-Rezeptoren |
| | T-Zell-Rezeptoren |
| ALL | CALLA |
| | CD19 |
| | Non-Hodgkin Lymphoma |

― Die Humanisierung muriner Antikörper, die Herstellung und Optimierung der Gene für Fab und rek. Fv Fragmente erfolgt entsprechend der dem Fachmann bekannten Technik (Winter et al., Nature 349, 293 (1991); Hoogenbooms et al., Rev. Tr. Transfus. Hemobiol. 36, 19 (1993); Girol. Mol. Immunol. 28, 1379 (1991) oder Huston et al., Intern. Rev. Immunol. 10, 195 (1993)). Die Fusion der rek. Fv-Fragmente mit Genen für zytostatische, zytotoxische oder entzündungserregende Proteinen oder Enzymen erfolgt gleichermaßen entsprechend dem dem Fachmann bekannten Stand der Technik.

### 1.7. Effektorgene für Fusionsproteine von Zielzell-bindenden Liganden mit zytostatischen und zytotoxischen Proteinen. Zu den Liganden gehören alle Substanzen, welche an Membranstrukturen oder Membranrezeptoren auf Endothelzellen binden. Beispielsweise gehören hierzu

― Cytokine wie beispielsweise IL-1 oder Wachstumsfaktoren oder deren Fragmente bzw. Teilsequenzen von ihnen, die an Rezeptoren exprimiert durch Endothelzellen binden, wie beispielsweise PDGF, bFGF, VEGF, TGF.
― Des weiteren gehören hierzu Adhäsionsmoleküle, welche an aktivierte und/oder proliferierende Endothelzellen binden. Hierzu gehören beispielsweise SLex, LFA-1, MAC-1, LECAM-1, VLA-4 oder Vitronectin.
― Hierzu gehören des weiteren Substanzen, welche an Membranstrukturen oder Membranrezeptoren von Tumor- oder Leukämiezellen binden. Beispielsweise gehören hierzu Hormone oder Wachstumsfaktoren oder deren Fragmente bzw. Teilsequenzen von ihnen, die an Rezeptoren exprimiert durch Leukämiezellen oder Tumorzellen binden.
   Derartige Wachstumsfaktoren wurden bereits beschrieben (Übersichten bei Cross et al., Cell 64, 271 (1991), Aulitzky et al., Drugs 48, 667 (1994), Moore, Clin. Cancer Res. 1, 3 (1995), Van Kooten et al., Leuk. Lymph. 12, 27 (1993)).
― Die Fusion der Gene dieser an die Zielzelle bindenden Liganden mit zytostatischen, zytotoxischen oder entzündungserregenden Proteinen oder Enzymen erfolgt entsprechend dem Stand der Technik mit den dem Fachmann bekannten Methoden.

### 1.8. Effektorgene für Induktoren von Entzündungen, zum Beispiel für

― IL-1
― IL-2
― RANTES (MCP-2)
― monocyte chemotactic and activating factor (MCAF)
― IL-8
― macrophage inflammatory protein-1 (MIP-1α, -β)
― neutrophil activating protein-2 (NAP-2)
― IL-3
― IL-5
― human leukemia inhibitory factor (LIF)
― IL-7
― IL-11
― IL-13
― GM-CSF
― G-CSF
― M-CSF
― Cobra venom factor (CVF) oder Teilsequenzen vom CVF, welchem dem menschlichen Komplementfaktor C3b funktionell entsprechen, d.h. welche an den Komplementfaktor B binden können und nach Spaltung durch den Faktor D eine C3 Konvertase darstellen
― der menschliche Komplementfaktor C3 oder seine Teilsequenz C3b
― Spaltprodukte des menschlichen Komplementfaktors C3, welche funktionell und strukturell dem CVF ähneln
― bakterielle Proteine, welche Komplement aktivieren oder Entzündungen auslösen, wie beispielsweise Porine von Salmonella typhi murium, "clumping" Faktoren von Staphylococcus aureus, Moduline besonders von gram-negativen Bakterien, "Major outer membrane protein" von Legionellen oder von Haemophilus influenzae Typ B oder von Klebsiellen oder M-Moleküle von Streptokokken Gruppe G.

### 1.9. Effektorgene für Enzyme für die Aktivierung von Vorstufen von Zytostatika, zum Beispiel für Enzyme, welche inaktive Vorsubstanzen (Prodrugs) in aktive Zytostatika (Drugs) verwandeln oder spalten.

Derartige Substanzen und die jeweils zugehörigen Prodrugs und Drugs sind bereits von Deonarain et al. (Br. J. Cancer 70, 786 (1994)), Mullen, Pharmac. Ther. 63, 199 (1994)) und Harris et al. (Gene Ther. 1, 170 (1994)) übersichtlich beschrieben worden. Beispielsweise ist die DNA-Sequenz eines der folgenden Enzyme zu verwenden:
― Herpes Simplex Virus thymidinkinase
― Varizella Zoster Virus Thymidinkinase
― bakterielle Nitroreduktase
― bakterielle β-Glucuronidase
― pflanzliche β-Glucuronidase aus Secale cereale
― humane β-Glucuronidase
― humane Carboxy peptidase (CB) zum Beispiel CB-A der Mastzelle, CB-B des Pankreas oder bakterielle Carboxy peptidase
― bakterielle β-Laktamase
― bakterielle Cytosine deaminase
― humane Catalase bzw. Peroxidase
― Phosphatase, im besonderen humane alkalische Phosphatase, humane saure Prostataphosphatase oder Typ 5 saure Phosphatase
― Oxidase, im besonderen humane Lysyloxidase oder humane saure D-aminooxidase
― Peroxidase, im besonderen humane Glutathion Peroxidase, humane Eosinophilen Peroxidase oder humane Schilddrüsen Peroxidase
― Galaktosidase

### 8.4.2. Therapie von Autoimmunerkrankungen und Entzündungen

### 2.1. Zielzellen:

― proliferierende Endothelzellen oder
― Makrophagen und/oder Lymphozyten oder
― Synovialzellen

### 2.2. Promotoren:

― endothelzellspezifisch und Zellzyklusspezifisch oder
― makrophagen- und/oder lymphozytenspezifisch und/oder zellzyklusspezifisch oder
― synovialzellspezifisch und/oder Zellzyklusspezifisch

### 2.3. Effektorgene für die Therapie von Allergien, zum Beispiel für

― IFNβ
― IFNγ
― IL-10
― Antikörper bzw. Antikörperfragmente gegen IL-4
― lösliche IL-4-Rezeptoren
― IL-12
― TGFβ

### 2.4. Effektorgene für die Verhinderung der Abstoßung von transplantierten Organen, zum Beispiel für

― IL-10
― TGFβ
― lösliche IL-1-Rezeptoren
― lösliche IL-2-Rezeptoren
― IL-1-Rezeptorantagonisten
― lösliche IL-6-Rezeptoren
― immunsuppressive Antikörper oder deren V_{H} und V_{L} enthaltende Fragmente oder deren über einen Linker verbundene V_{H}- und V_{L}-Fragmente. Immunsuppressive Antikörper sind beispielsweise Antikörper spezifisch für den T-Zell-Rezeptor oder seinen CD3-Komplex, gegen CD4 oder CD8 des weiteren gegen den IL-2-Rezeptor, IL-1-Rezeptor oder IL-4-Rezeptor oder gegen die Adhäsionsmoleküle CD2, LFA-1, CD28 oder CD40

### 2.5. Effektorgene für die Therapie von Antikörper-mediierten Autoimmunerkrankungen, zum Beispiel für

― TGFβ
― IFNα
― IFNβ
― IFNγ
― IL-12
― lösliche IL-4-Rezeptoren
― lösliche IL-6-Rezeptoren
― immunsuppressive Antikörper oder dessen V_{H} und V_{L}-enthaltende Fragmente

### 2.6. Effektorgene für die Therapie von Zell-mediierten Autoimmunerkrankungen, zum Beispiel für

― IL-6
― IL-9
― IL-10
― IL-13
― TNFα oder TNFβ
― einen immunsuppressiven Antikörper oder dessen V_{H}- und V_{L}-enthaltende Fragmente

### 2.7. Effektorgene für Inhibitoren der Zellproliferation, zytostatische oder zytotoxische Proteine und Enzyme für die Aktivierung von Vorstufen von Zytostatika

Beispiele für Gene kodierend für derartige Proteine sind bereits im Abschnitt "Effektorgene für die Therapie von Tumoren" aufgeführt.

In gleicher Form wie dort bereits beschrieben, können im Sinne der Erfindung Effektorgene verwendet werden, welche für Fusionsproteine aus Antikörpern bzw. Fab oder rek. Fv-Fragmenten dieser Antikörper oder anderen Liganden spezifisch für die Zielzelle und den o.a. Cytokinen, Wachstumsfaktoren, Rezeptoren, zytostatischen oder zytotoxischen Proteinen und Enzymen kodieren.

### 2.8. Effektorgene für die Therapie der Arthritis

Im Sinne der Erfindung werden Effektorgene ausgewählt, deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert.

Hierzu gehören zum Beispiel
― IL-1-Rezeptorantagonist (IL-1-RA);
   IL-1-RA inhibiert die Bindung von IL-1α, β
― löslicher IL-1-Rezeptor;
   löslicher IL-1-Rezeptor bindet und inaktiviert IL-1
― IL-6
   IL-6 erhöht die Sekretion von TIMP und Superoxiden und vermindert die Sekretion von IL-1 und TNFα durch Synovialzellen und Chondrozyten
― löslicher TNF-Rezeptor
   löslicher TNF-Rezeptor bindet und inaktiviert TNF.
― IL-4
   IL-4 inhibiert die Bildung und Sekretion von IL-1, TNFα und MMP
― IL-10
   IL-10 inhibiert die Bildung und Sekretion von IL-1, TNF∝ und MMP und erhöht die Sekretion von TIMP
― Insulin-like growth factor (IGF-1)
   IGF-1 stimuliert die Synthese von extrazellulärer Matrix.
― TGFβ, im speziellen TGFβ1 und TGFβ2
   TGFβ stimuliert die Synthese von extrazellulärer Matrix.
― Superoxiddismutase
― TIMP, im speziellen TIMP-1, TIMP-2 oder TIMP-3

### 8.4.3. Therapie der mangelhaften Bildung von Zellen des Blutes

### 3.1. Zielzellen:

― proliferierende, unreife Zellen des blutbildenden Systems oder
― Stromazellen benachbart den blutbildenden Zellen

### 3.2. Promotoren:

― spezifisch für blutbildende Zellen und/oder Zellzyklusspezifisch
― zellunspezifisch und zellzyklusspezifisch

### 3.3. Effektorgene für die Therapie der Anämie, zum Beispiel für

― Erythropoietin

### 3.4. Effektorgene für die Therapie der Leukopenie, zum Beispiel für

― G-CSF
― GM-CSF
― M-CSF

### 3.5. Effektorgene für die Therapie der Thrombozytopenie, zum Beispiel für

― IL-3
― Leukemia Inhibitory Factor (LIF)
― IL-11
― Thrombopoietin

### 8.4.4. Therapie von Schäden des Nervensystems

### 4.1. Zielzellen:

― Gliazellen oder
― proliferierende Endothelzellen

### 4.2. Promotoren:

― Gliazell-spezifisch und zellzyklusspezifisch oder
― Endothelzell-spezifisch und Zellzyklusspezifisch oder
― unspezifisch und Zellzyklusspezifisch

### 4.3. Effektorgene für neuronale Wachstumsfaktoren, zum Beispiel

― FGF
― Nerve growth factor (NGF)
   Brain-derived neurotrophic factor (BDNF)
― Neurotrophin-3 (NT-3)
― Neurotrophin-4 (NT-4)
― Ciliary neurotrophic factor (CNTF)

### 4.4. Effektorgene für Enzyme, zum Beispiel für

― Tyrosinhydroxylase
― Dopadecarboxylase

### 4.5. Effektorgene für Cytokine und deren Inhibitoren, welche die neurotoxische Wirkung von TNFα inhibieren oder neutralisieren, zum Beispiel für

― TGFβ
― lösliche TNF-Rezeptoren
― TNF-Rezeptoren neutralisieren TNFα
― IL-10
   IL-10 inhibiert die Bildung von IFNγ, TNFα, IL-2 und IL-4
― lösliche IL-1-Rezeptoren
― IL-1-Rezeptor I
― IL-1-Rezeptor II
― lösliche IL-1-Rezeptoren neutralisieren die Aktivität von IL-1
― IL-1-Rezeptor-Antagonist
― lösliche IL-6-Rezeptoren

### 8.4.5. Therapie von Störungen des Blutgerinnungs- und Blutkreislaufsystems

### 5.1. Zielzellen:

― Endothelzellen oder
― proliferierende Endothelzellen oder
― somatische Zellen in Nachbarschaft von Endothelzellen und glatte Muskelzellen oder
― Makrophagen

### 5.2. Promotoren:

― zellunspezifisch und zellzyklusspezifisch oder
― spezifisch für Endothelzellen, glatte Muskelzellen oder Makrophagen und zellzyklusspezifisch

### 5.3. Stukturgene für die Inhibition der Gerinnung oder für die Förderung der Fibrinolyse, zum Beispiel für

― Tissue Plasminogen Activator (tPA)
― Urokinase-type Plasminogen Activator (uPA)
― Hybride von tPA und uPA
― Protein C
― Hirudin
― Serin Proteinase Inhibitoren (Serpine), wie beispielsweise C-1S-Inhibitor, α1-Antitrypsin oder Antithrombin III
― Tissue Factor Pathway Inhibitor (TFPI)

### 5.4. Effektorgene für die Förderung der Gerinnung, zum Beispiel für

― F VIII
― F IX
― von Willebrand factor
― F XIII
― PAI-1
― PAI-2
― Tissue Factor and Fragmente hiervon

### 5.5. Effektorgene für Angiogenesefaktoren, zum Beispiel für

― VEGF
― FGF

### 5.6. Effektorgene für die Blutdrucksenkung, zum Beispiel für

― Kallikrein
― Endothelzell "nitric oxide synthase"

### 5.7. Effektorgene für die Inhibition der Proliferation von glatten Muskelzellen nach Verletzungen der Endothelschicht, zum Beispiel für

― ein antiproliferatives, zytostatisches oder zytotoxisches Protein oder
― ein Enzym zur Aufspaltung von Vorstufen von Zytostatika in Zytostatika wie bereits oben (unter Tumor) aufgeführt oder
― ein Fusionsprotein eines dieser Wirkstoffe mit einem Liganden, beispielsweise einem Antikörper oder Antikörperfragmenten spezifisch für Muskelzellen

### 5.8. Effektorgene für weitere Blutplasmaproteine, zum Beispiel für

― Albumin
― C1-Inaktivator
― Serum Cholinesterase
― Transferrin
― 1-Antritrypsin

### 8.4.6. Impfungen

### 6.1. Zielzellen:

― Muskelzellen oder
― Makrophagen und/oder Lymphozyten
― Endothelzellen

### 6.2. Promotoren:

― unspezifisch und zellzyklusspezifisch oder
― zielzellspezifisch und zellzyklusspezifisch

### 6.3. Effektorgene für die Prophylaxe von Infektionserkrankungen

Die Möglichkeiten, auf konventionellem Wege wirkungsvolle Impfstoffe herzustellen, sind beschränkt.

Demzufolge wurde die Technologie der DNA-Vakzine entwickelt. Diese DNA-Vakzinen werfen jedoch Fragen zur Wirksamkeitsstärke auf (Fynan et al., Int. J. Immunopharm. 17, 79 (1995); Donnelly et al., Immunol 2, 20 (1994)).

Gemäß dieser Erfindung ist mit einer größeren Wirksamkeit der DNA-Vakzine zu rechnen.

Als Wirksubstanz ist die DNA eines vorn Infektionserreger gebildeten Proteins auszuwählen, welches durch Auslösung einer Immunreaktion, d.h. durch Antikörperbindung und/oder durch zytotoxische T-Lymphozyten zur Neutralisierung und/oder zur Abtötung des Erregers führt. Derartige sogenannte Neutralisationsantigene werden als Impfantigene bereits angewandt (siehe Übersicht bei Ellis, Adv. Exp. Med. Biol. 327, 263 (1992)).

Bevorzugt im Sinne der Erfindung wird die DNA kodierend für Neutralisationsantigene folgender Erreger:
― Influenza A-Virus
― HIV
― Tollwut-Virus
― HSV (Herpes Simplex Virus)
― RSV (Respiratory Syncytial Virus)
― Parainfluenza-Virus
― Rotavirus
― VZV (Varizella Zoster Virus)
― CMV (Cytomegalo-Virus)
― Masern-Virus
― HPV (Humanes Papillomvirus)
― HBV (Hepatitis B-Virus)
― HCV (Hepatitis C-Virus)
― HDV (Hepatitis D-Virus)
― HEV (Hepatitis E-Virus)
― HAV (Hepatitis A-Virus)
― Vibrio Cholera-Antigen
― Borrelia Burgdorferi
― Helicobacter pylori
― Malaria-Antigen
― Zu derartigen Wirksubstanzen im Sinne der Erfindung gehört jedoch auch die DNA eines Antiidiotyp-Antikörpers oder seiner Antigen-bindenden Fragmente, dessen Antigenbindungsstrukturen (die "complementarity determining regions") Kopien der Protein- oder Kohlenhydratstruktur des Neutralisationsantigens des Infektionserregers darstellen.

Derartige Antiidiotyp-Antikörper können besonders Kohlenhydratantigene bei bakteriellen Infektionserregern ersetzen.

Derartige antiidiotypische Antikörper und ihre Spaltprodukte wurden von Hawkins et al. (J. Immunother. 14, 273 (1993)) und Westerink und Apicella (Springer Seminars in Immunopathol 15, 227 (1993)) übersichtlich beschrieben.

### 6.4. Effektorgene für "Tumorvakzinen"

― Hierzu gehören Antigene auf Tumorzellen. Derartige Antigene wurden zum Beispiel von Sedlacek et al., Contrib. to Oncol 32, Karger Verlag, München (1988) und Contrib. to Oncol 43, Karger Verlag, München (1992) übersichtlich dargestellt.

Weitere Beispiele stellen die Gene für folgende Proteinantigene bzw. für die variable Region (V_{L}, V_{H}) von Antiidiotypantikörpern korrespondierend für folgende Nicht-Proteinantigene dar:
― Ganglioside
― Sialyl Lewis
― Peptide auf Tumoren, welche von T-Zellen erkannt werden
― von Onkogenen exprimierte Proteine
― Blutgruppenantigene und deren Vorläufer
― Antigene auf tumorassoziiertem Mucin
― Antigene auf Heat Shock Proteinen

### 8.4.7. Die Therapie von chronischen Infektionserkrankungen

### 7.1. Zielzelle:

― Leberzelle
― Lymphozyt und/oder Makrophage
― Epithelzelle
― Endothelzelle

### 7.2. Promotoren:

― virusspezifisch oder zellspezifisch und zellzyklusspezifisch

### 7.3. Effektorgene, beispielsweise für

― ein Protein, welches zytostatische, apoptotische oder zytotoxische Wirkungen aufweist.
― ein Enzym, welches eine Vorstufe einer antiviralen oder zytotoxischen Substanz in die aktive Substanz spaltet.

### 7.4. Effektorgene für antivirale Proteine

― antiviral wirksame Cytokine und Wachstumsfaktoren. Hierzu zählen beispielsweise IFNα, IFNβ, IFN-γ, TNFβ, TNFα, IL-1 oder TGFβ
― Antikörper einer Spezifität, die das jeweilige Virus inaktiviert oder dessen V_{H} und V_{L} enthaltende Fragmente oder dessen über einen Linker verbundene V_{H} und V_{L} Fragmente herstellt wie bereits beschrieben.
   Antikörper gegen Virusantigen sind beispielsweise:
   anti HBV
   anti HCV
   anti HSV
   anti HPV
   anti HIV
   anti EBV
   anti HTLV
   anti Coxsackie Virus
   anti Hantaan Virus
― ein Rev bindendes Protein. Diese Proteine binden an die Rev-RNA und inhibieren Rev-abhängige posttranskriptionelle Stufen der Retrovirus-Genexpression. Beispiele für Rev-bindende Proteine sind:
   RBP9-27
   RBP1-8U
   RBP1-8D
   Pseudogene von RBP1-8
― Ribozyme, welche die mRNA von Genen für Zellzykluskontrollproteine oder die mRNA von Viren verdauen. Ribozyme katalytisch für HIV wurden beispielsweise von Christoffersen et al., J. Med. Chem 38, 2033 (1995) übersichtlich beschrieben.

### 7.5. Effektorgene für antibakterielle Proteine

Zu den antibakteriellen Proteinen gehören beispielsweise Antikörper, die bakterielle Toxine neutralisieren oder Bakterien opsonieren. Beispielsweise gehören hierzu Antikörper gegen
Meningokokken C oder B
E. coli
Borrelia
Pseudomonas
Helicobacter pylori
Staphylococcus aureus

### 8.5. Kombination gleicher oder unterschiedlicher Effektorgene Gegenstand der Erfindung ist des weiteren ein selbstverstärkendes ggf.

pharmakologisch kontrollierbares Expressionssystem, in welchem eine Kombination der DNA-Sequenzen von zwei gleichen oder zwei unterschiedlichen Effektorgenen [Komponente d) und d')] vorliegt. Zur Expression beider DNA-Sequenzen ist eine weitere Promotorsequenz oder vorzugsweise die cDNA einer "internal ribosome entry site" (IRES) als regulatorisches Element zwischen beiden Effektorgenen geschaltet.

Eine IRES ermöglicht die Expression zweier über eine IRES miteinander verbundener DNA-Sequenzen.

Derartige IRES wurden beispielsweise von Montford und Smith (TIG 11, 179 (1995); Kaufman et al., Nucl. Acids Res 19, 4485 (1991); Morgan et al., Nucl. Acids Res. 20, 1293 (1992); Dirks et al., Gene 128, 247 (1993); Pelletier und Sonenberg, Nature 334, 320 (1988) und Sugitomo et al., BioTechn 12, 694 (1994)) beschrieben.

So kann beispielsweise die cDNA der IRES-Sequenz des Poliovirus (Position ≤ 140 bis ≥ 630 des 5' UTR) verwendet werden.

Bevorzugt im Sinne der Erfindung sind über weitere Promotorsequenzen oder eine IRES-Sequenz Effektorgene zu verknüpfen, welche eine additive Wirkung aufweisen.

Im Sinne der Erfindung sind bevorzugte Kombinationen von Effektorgenen beispielsweise für

### 8.5.1. die Therapie von Tumoren

― gleiche oder unterschiedliche, zytostatische, apoptotische, zytotoxische oder entzündungserregende Proteine oder
― gleiche oder unterschiedliche Enzyme für die Spaltung der Vorstufe eines Zytostatikums

### 8.5.2. die Therapie von Autoimmunerkrankungen

― unterschiedliche Cytokine oder Rezeptoren mit synergistischer Wirkung zur Hemmung der zellulären und/oder humoralen Immunreaktion oder
― unterschiedliche oder gleiche TIMPs

### 8.5.3. die Therapie von mangelhafter Bildung von Zellen des Blutes

― unterschiedliche, hierarchisch aufeinanderfolgende Cytokine, wie beispielsweise IL-1, IL-3, IL-6 oder GM-CSF und Erythropoietin, G-CSF oder Thrombopoietin

### 8.5.4. die Therapie von Nervenzellschäden

― ein neuronaler Wachstumsfaktor und ein Cytokin oder der Inhibitor eines Cytokines

### 8.5.5. die Therapie von Störungen des Blutgerinnungs- und Blutkreislaufsystems

― ein Antithrombotikum und ein Fibrinolytikum (z.B. tPA oder uPA) oder
― ein zytostatisches, apoptotisches oder zytotoxisches Protein und ein Antithrombotikum oder ein Fibrinolytikum
― mehrere unterschiedliche, synergistisch wirkende Blutgerinnungsfaktoren beispielsweise F VIII und vWF oder F VIII und F IX

### 8.5.6. Impfungen

― ein Antigen und ein immunstimulierendes Cytokin, wie beispielsweise IL-1α, IL-1ß, IL-2, GM-CSF, IL-3 oder IL-4 Rezeptor
― unterschiedliche Antigene eines Infektionserregers oder unterschiedlicher Infektionserreger oder
― unterschiedliche Antigene eines Tumortyps oder unterschiedlicher Tumortypen

### 8.5.7. Therapie von viralen Infektionserkrankungen

― ein antivirales Protein und ein zytostatisches, apoptotisches oder zytotoxisches Protein
― Antikörper gegen unterschiedliche Oberflächenantigene eines Virus oder mehrere Viren

### 8.5.8. Therapie von bakteriellen Infektionserkrankungen

― Antikörper gegen unterschiedliche Oberflächenantigene und/oder Toxine eines Keimes

### 8.6. Einfügung von Signalsequenzen und Transmembrandomänen

### 8.6.1. Verstärkung der Translation

Zur Verstärkung der Translation kann am 3' Ende der Promotorsequenz und unmittelbar am 5' Ende des Startsignals (ATG) der Signal- bzw. Transmembransequenz die Nukleotidsequenz GCCACC oder GCCGCC eingefügt (Kozak, J. Cell Biol. 108, 299 (1989)) werden.

### 8.6.2. Erleichterung der Sekretion

Zur Erleichterung der Sekretion des Expressionsproduktes des Effektorgenes kann die ggf. in der DNA-Sequenz des Effektorgenes enthaltende homologe Signalsequenz ersetzt werden durch eine heterologe, die extrazelluläre Ausschleusung verbessernde Signalsequenz.

So kann beispielsweise die Signalsequenz für das Immunglobulin (DNA Position ≤ 63 bis ≥ 107; Riechmann et al., Nature 332, 323 (1988)) oder die Signalsequenz für das CEA (DNA-Position ≤ 33 bis ≥ 134; Schrewe et al., Mol. Cell Biol 10, 2738 (1990); Berling et al., Cancer Res 50, 6534 (1990)) oder die Signalsequenz des humanen Respiratory Syncytial Virus Glycoproteins (cDNA der Aminosäuren ≤ 38 bis ≥ 50 oder 48 bis 65; Lichtenstein et al., J. Gen. Virol. 77, 109 (1996)) eingefügt werden.

### 8.6.3. Verankerung des Wirkstoffes

### 3.1) Zur Verankerung des Wirkstoffes in die Zellmembran der den Wirkstoff bildenden transduzierten Zelle kann alternativ oder zusätzlich zur Signalsequenz eine Sequenz für eine Transmembrandomäne eingeführt werden.

So kann beispielsweise die Transmembransequenz des menschlichen Makrophagenkolonie-stimulierenden Faktors (DNA-Position ≤ 1485 bis ≥ 1554; Cosman et al., Behring Inst. Mitt 83, 15 (1988)) oder die DNA-Sequenz für die Signal- und Transmembranregion des menschlichen Respiratory Syncytial Virus (RSV)-Glykoproteins G (Aminosäuren 1 bis 63 oder deren Teilsequenzen, Aminosäuren 38 bis 63; Vijaya et al., Mol. Cell Biol. 8, 1709 (1988); Lichtenstein et al., J. Gen. Virol 77, 109 (1996)) oder die DNA-Sequenz für die Signal- und Transmembranregion der Influenzavirus-Neuraminidase (Aminosäuren 7 bis 35 oder die Teilsequenz Aminosäuren 7 bis 27; Brown et al., J .Virol 62, 3824 (1988)) zwischen der Promotorsequenz und der Sequenz des Effektorgens eingefügt werden.

### 3.2) Zur Verankerung des Wirkstoffes in die Zellmembran der den Wirkstoff bildenden transduzierten Zellen kann jedoch auch die Nukleotidsequenz für einen Glykophospholipid-Anker eingefügt werden.

Die Einfügung eines Glykophospholipid-Ankers erfolgt am 3' Ende der Nukleotidsequenz für das Effektorgen und kann zusätzlich zur Einfügung einer Signalsequenz erfolgen.

Glykophospholipid-Anker sind beispielsweise für das CEA, für das N-CAM und für weitere Membranproteine, wie beispielsweise Thy-1, beschrieben worden (siehe Übersicht Ferguson et al., Ann. Rev. Biochem 57, 285 (1988)).

### 3.3) Eine weitere Möglichkeit der Verankerung von Wirkstoffen an die Zellmembran entsprechend der vorliegenden Erfindung ist die Verwendung einer DNA-Sequenz für ein Ligand-Wirkstoff-Fusionsprotein. Die Spezifität des Liganden dieses Fusionsproteins ist gerichtet gegen eine Membranstruktur auf der Zellmembran der gewählten Zielzelle.

Zu den Liganden, welche an die Oberfläche von Zellen binden, gehören beispielsweise Antikörper oder Antikörperfragmente, gerichtet gegen Strukturen auf der Oberfläche von beispielsweise
― Endothelzellen. Insbesondere zählen hierzu Antikörper gegen die VEGF-Rezeptoren oder gegen Kinin-Rezeptoren
― oder von Muskelzellen, wie Antikörper gegen Actin oder Antikörper gegen Angiotensin II-Rezeptoren oder Antikörper gegen Rezeptoren für Wachstumsfaktoren, wie beispielsweise gegen EGF-Rezeptoren oder gegen PDGF-Rezeptoren oder gegen FGF-Rezeptoren oder Antikörper gegen Endothelin A-Rezeptoren
― Zu den Liganden gehören auch Antikörper oder deren Fragmente, welche gerichtet sind gegen tumorspezifische oder tumorassoziierte Antigene auf der Tumorzellmembran. Derartige Antikörper wurden bereits beschrieben.

Die murinen monoklonalen Antikörper sind bevorzugt in humanisierter Form einzusetzen. Fab und rek. Fv-Fragmente und deren Fusionsprodukte werden, wie bereits beschrieben, mit der dem Fachmann bekannten Technologie hergestellt.

Zu den Liganden gehören des weiteren alle Wirkstoffe, wie beispielsweise Cytokine oder Adhäsionsmoleküle, Wachstumsfaktoren oder deren Fragmente bzw. Teilsequenzen von ihnen, Mediatoren oder Peptidhormone, welche an Membranstrukturen oder Membranrezeptoren auf der jeweiligen ausgewählten Zelle binden. Beispielsweise gehören hierzu
― Liganden für Endothelzellen, wie IL-1, PDGF, bFGF, VEGF, TGGβ oder Kinin und Derivate oder Analoga von Kinin
― desweiteren gehören hierzu Adhäsionsmoleküle. Derartige Adhäsionsmoleküle, wie beispielsweise SLex, LFA-1, MAG-1, LeCAM-1, VLA-4 oder Vitronectin oder Derivate oder Analoga von Vitronectin, wurden bereits für Endothelzellen beschrieben (Übersichten bei Augustin-Voss et al., J. Cell Biol. 119, 483 (1992); Pauli et al., Cancer Metast. Rev. 9, 175 (1990); Honn et al., Cancer Metast. Rev. 11, 353 (1992); Varner et al., Cell Adh. Commun. 3, 367 (1995)).

### Figurenlegende:

- Figur 1:: Erfindungsgemäßes Nukleinsäurekonstrukt

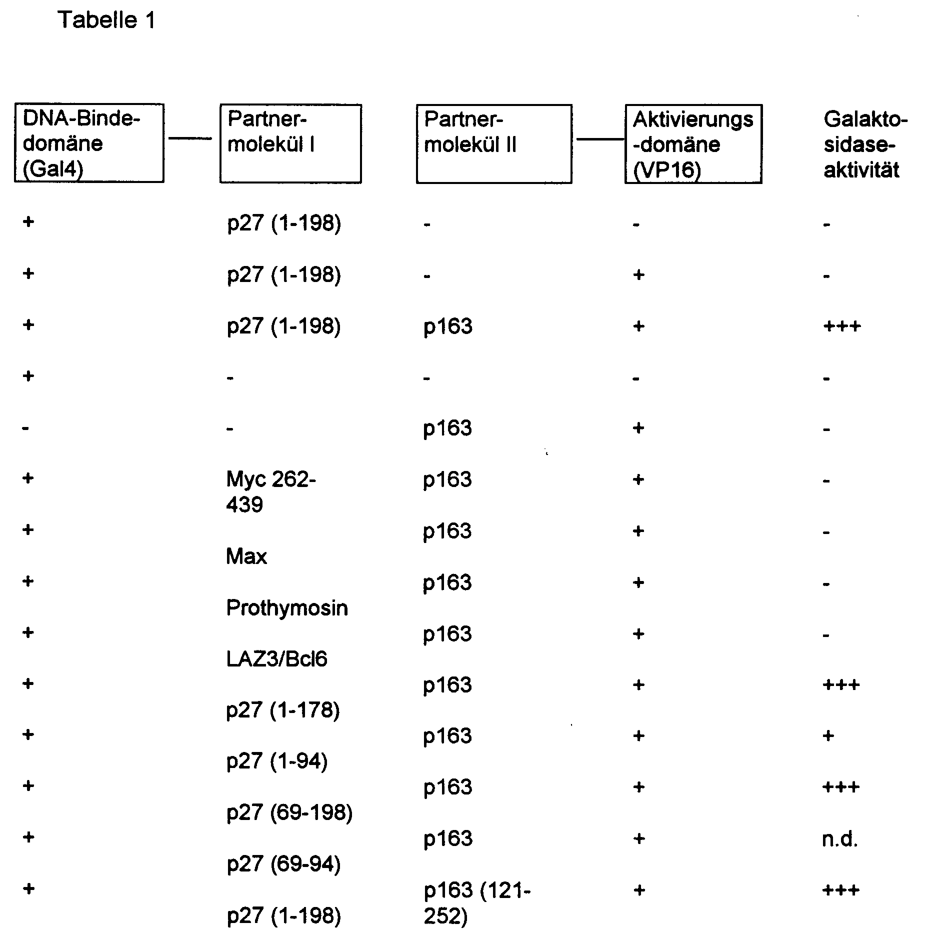

**Tabelle 2**

| Säulenauftrag | Bindung an Affinitätschromatographiesäule beschichtet mit | |
|---|---|---|
| | Fusionsprotein Glutathion-transferase - p163 | Glutathion-transferase |
| rekombinantes (³⁵S-markiertes) p27 | +++ | + |
| Zellextrakt (RAT1-Myc ER), enthaltend p27 | (+) | - |
| Zellextrakt, RAT1-Myc ER), enthaltend p27 (erhitzt 95°C, 2 min) | +++ | - |
| ckd-2 (Zellextrakt) | - | - |
| cdk-4 (Zellextrakt) | - | - |

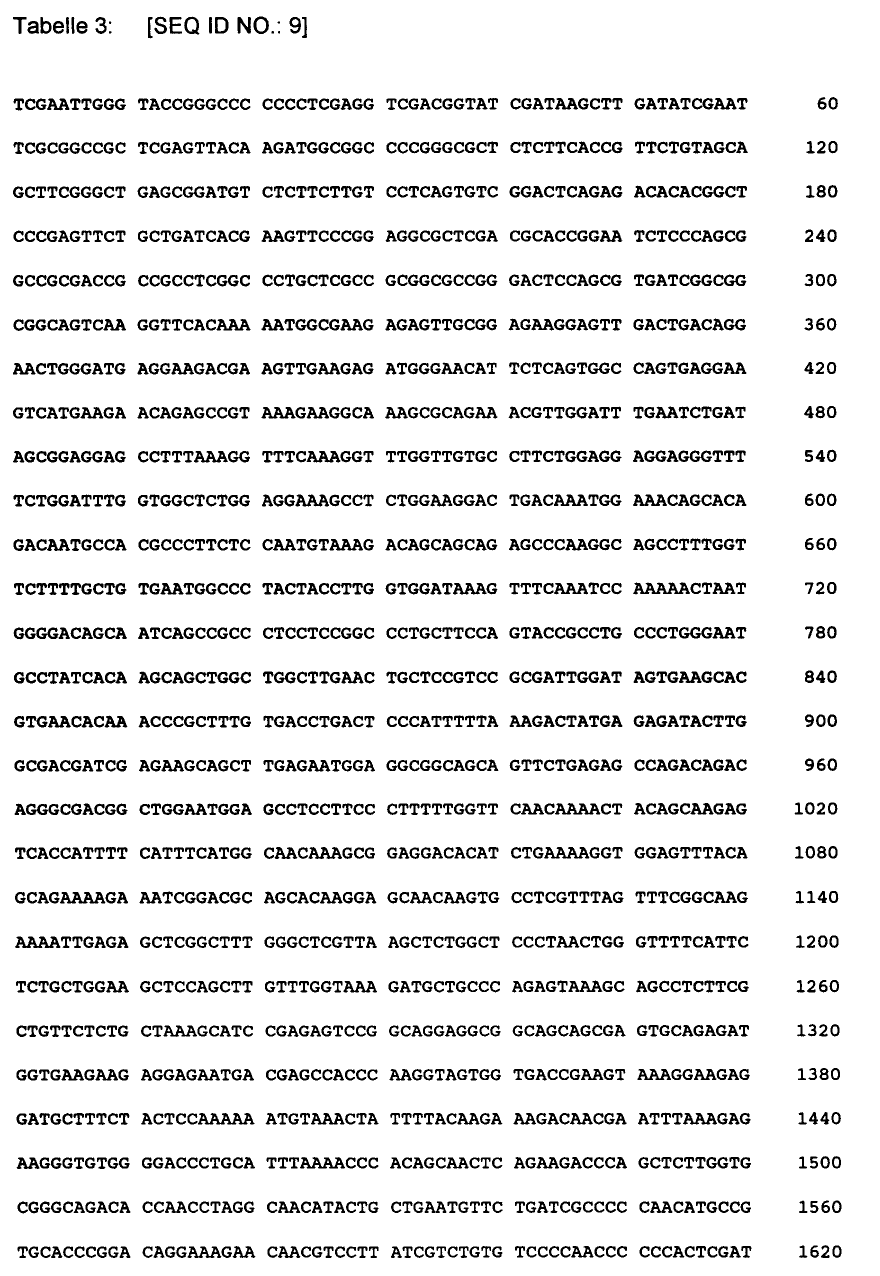

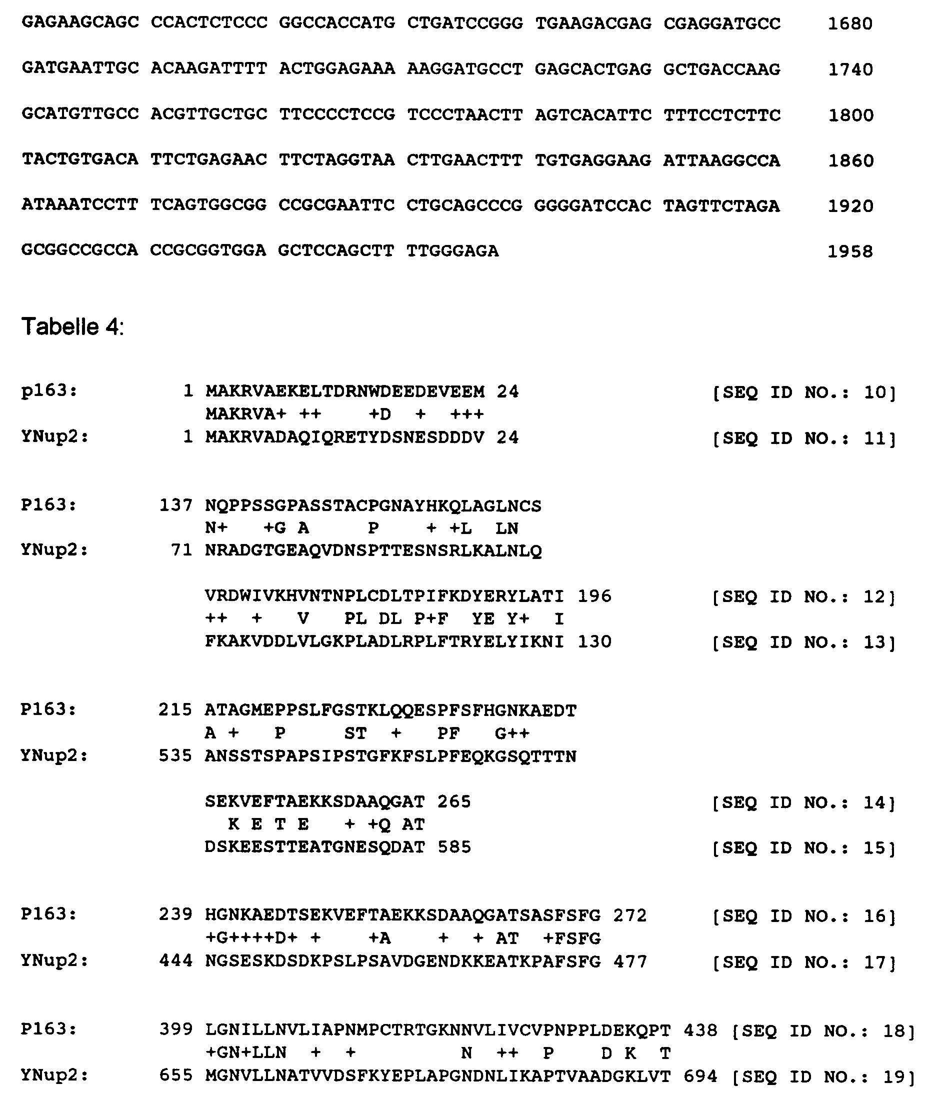

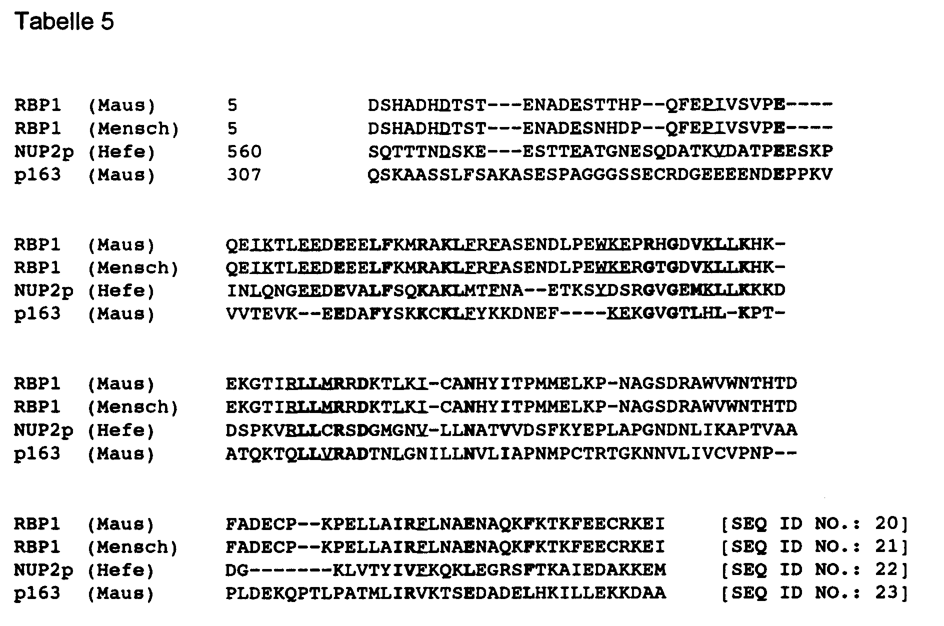

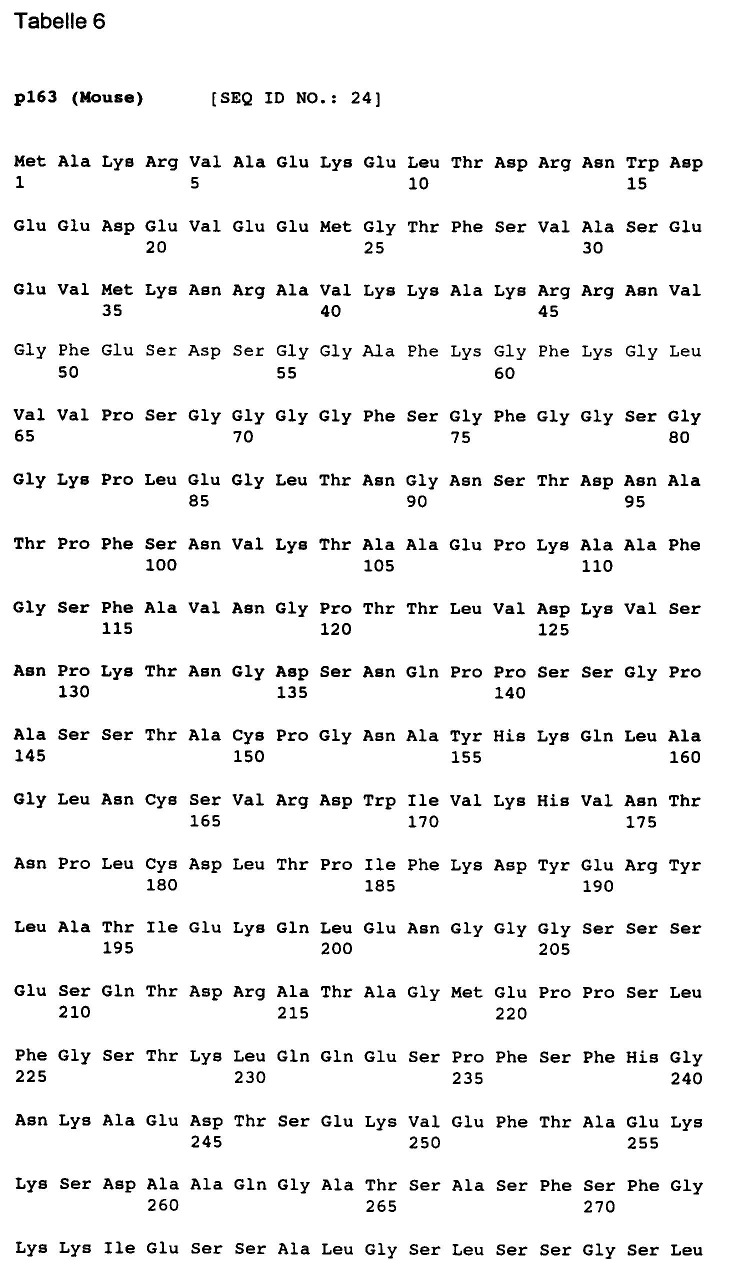

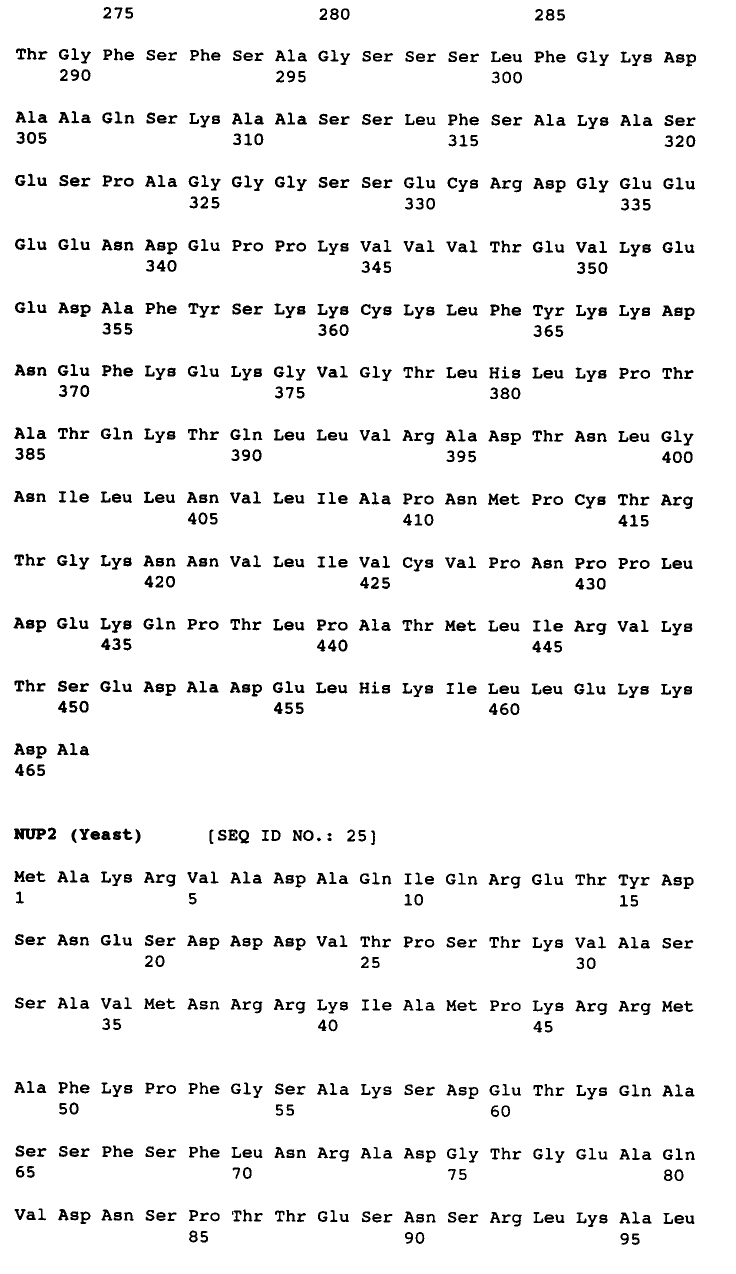

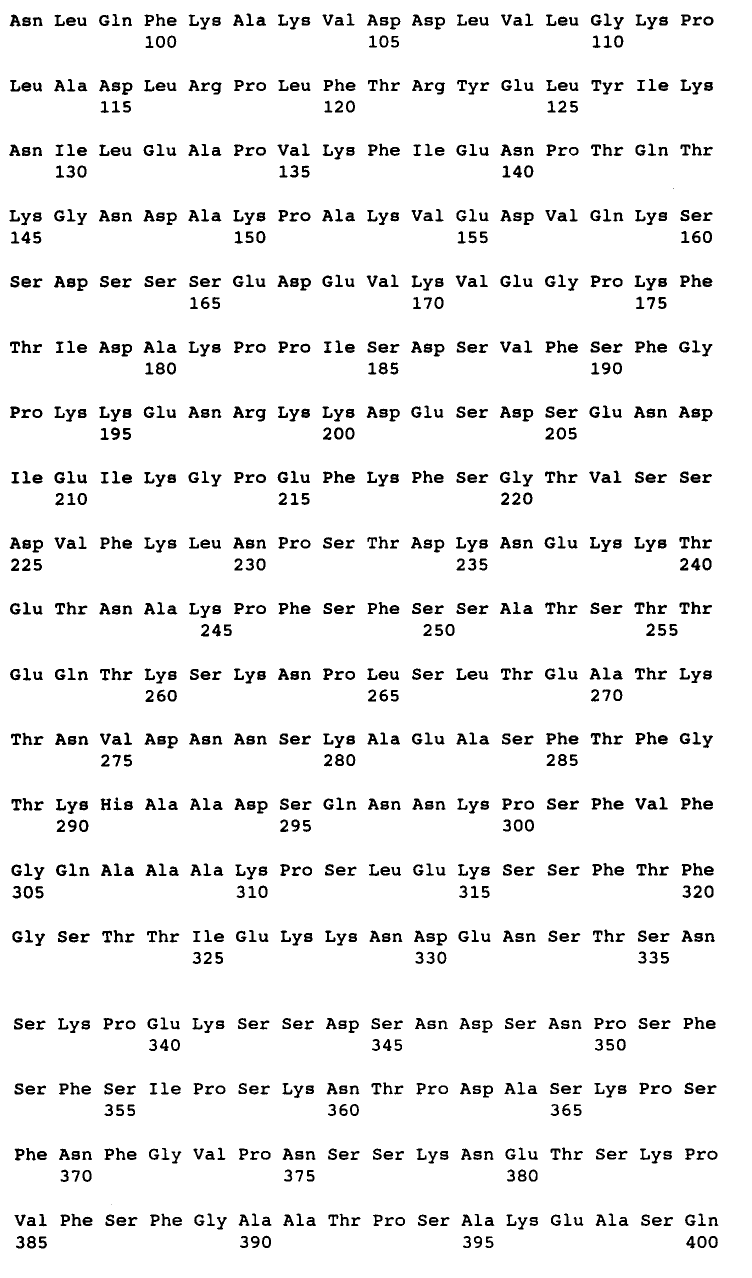

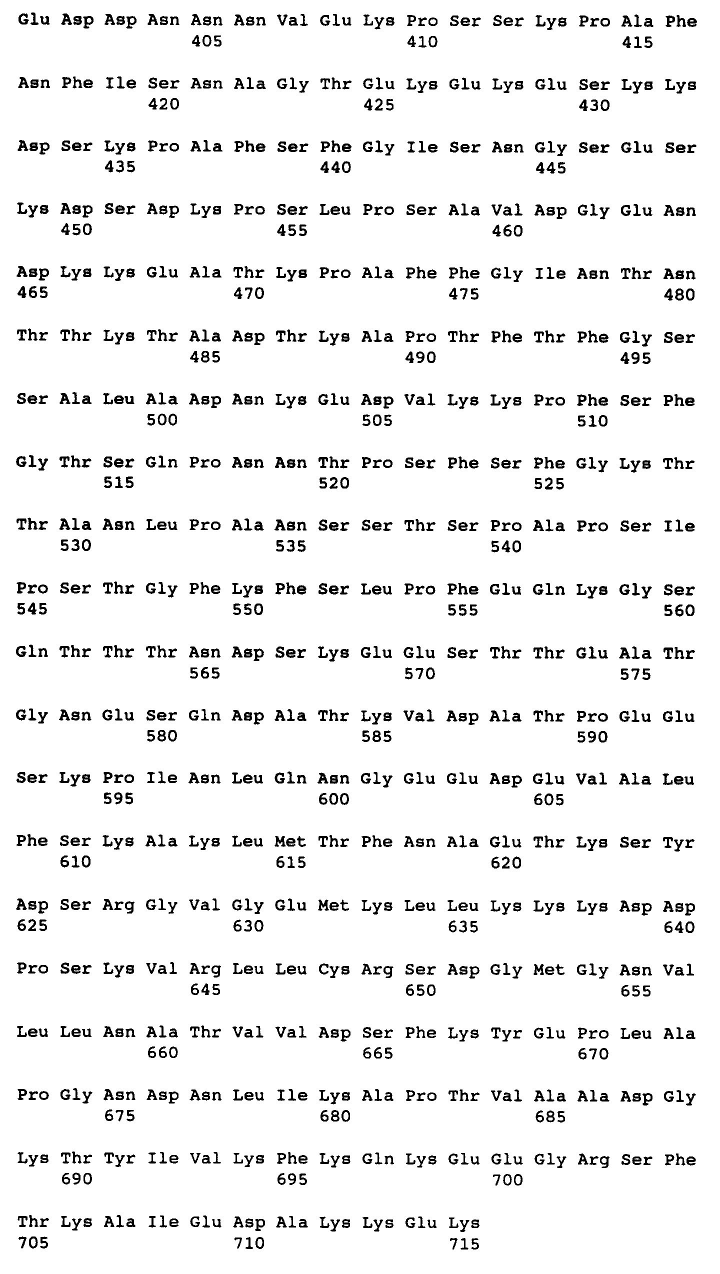

**Tabelle 7**

| Nachweis von Assoziationen zwischen p27 und p163 in HeLa-Zellen | | | | |
|---|---|---|---|---|
| | Präzipitatoin des Gesamtproteins von HeLa-Zellen transfiziert mit | | | |
| Präzipitation mit | CMV-p27 | CMV-p27 | - | - |
| | | + | | |
| | | CMV-Nap | CMV-Nap2 | - |
| Antikörper spezifisch für Nap2 | - | + | + | - |
| Antikörper spezifisch für p27 | + | ++ | - | - |

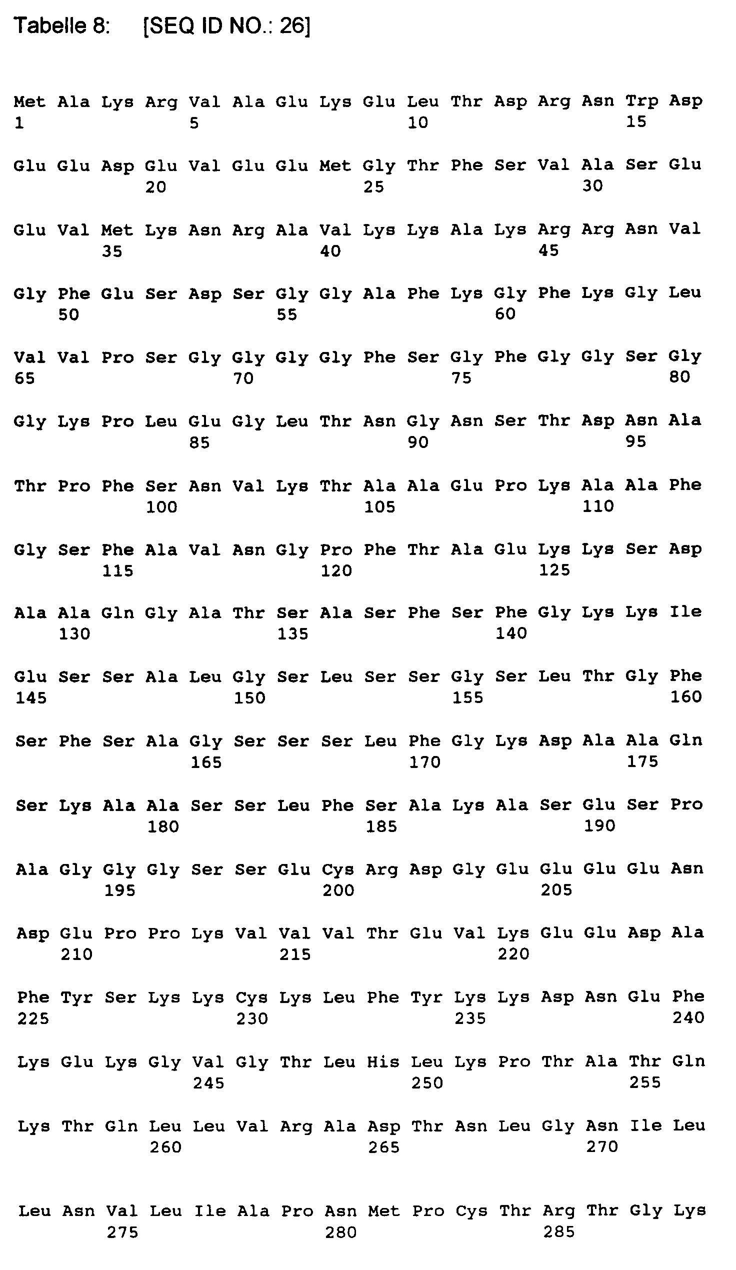

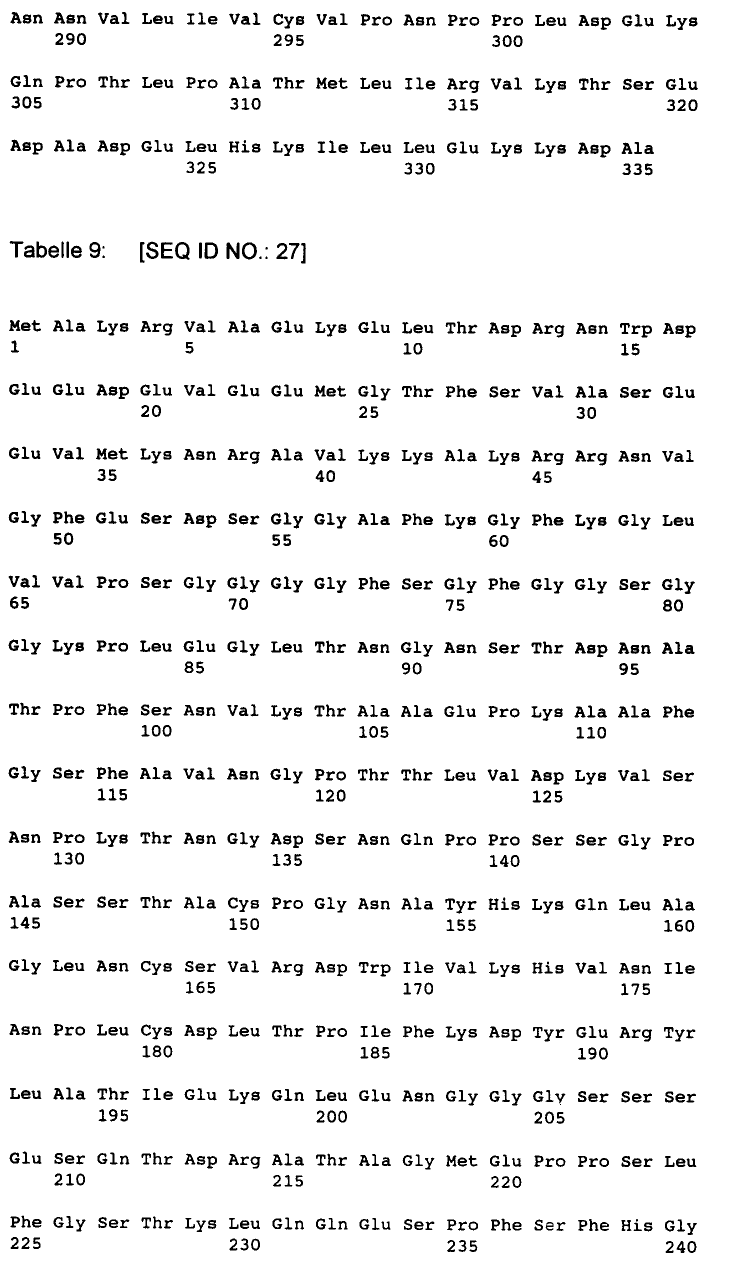

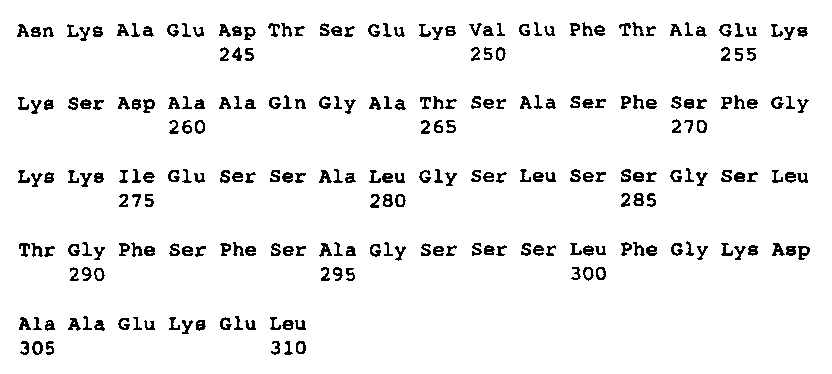

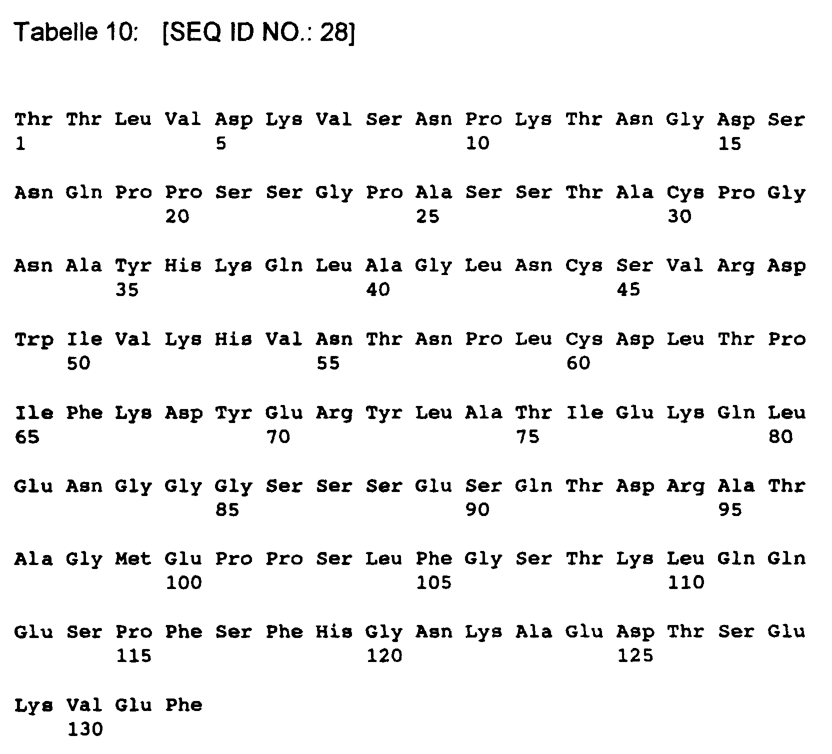

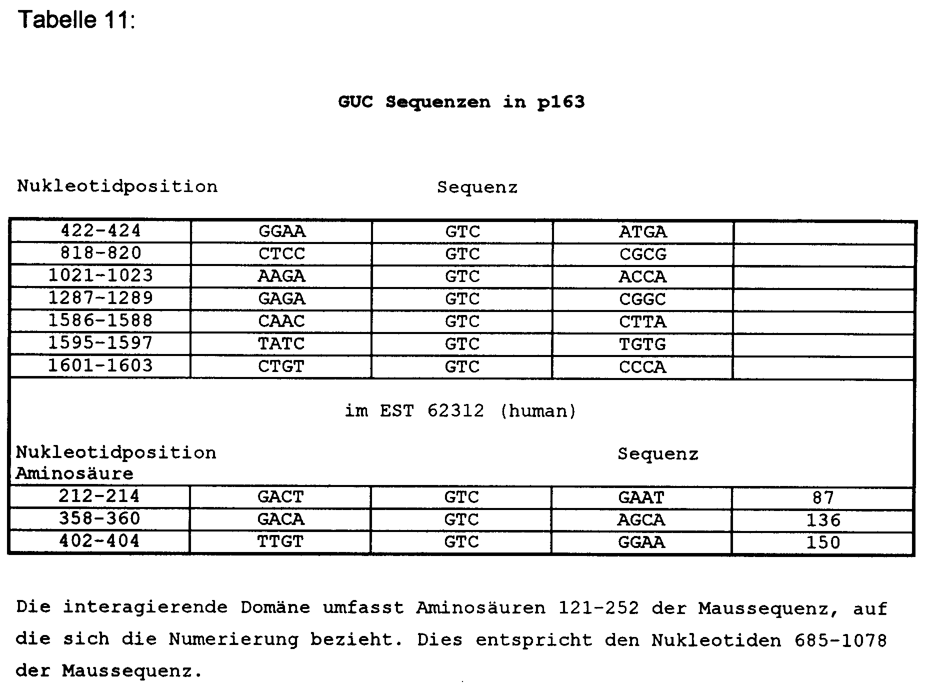

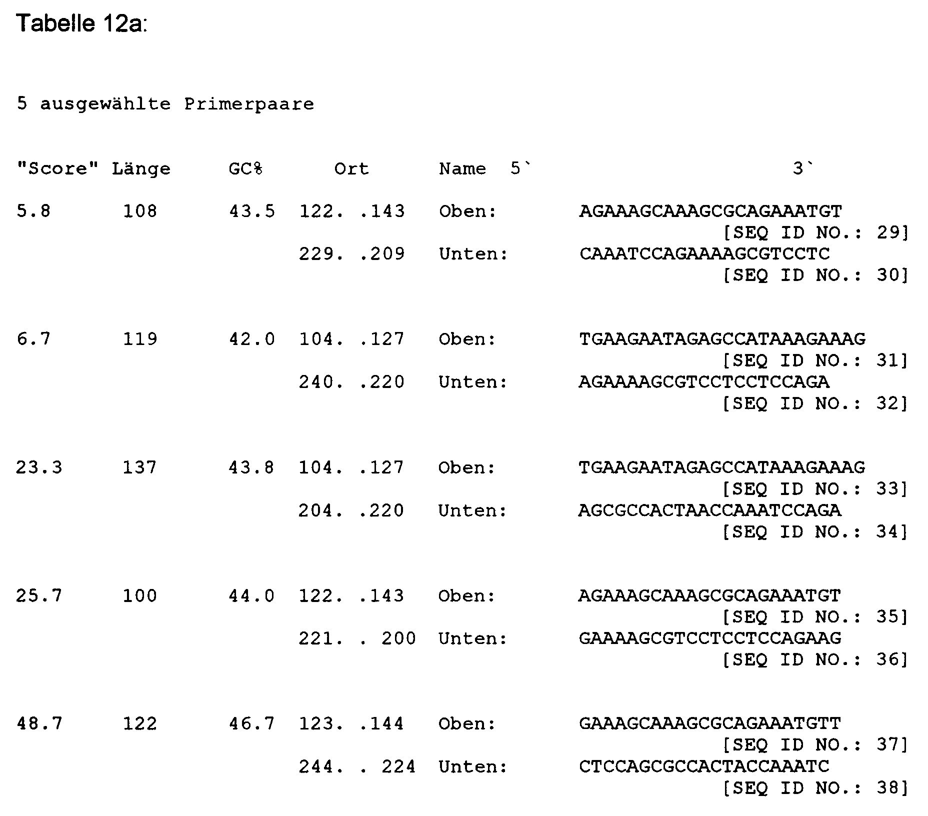

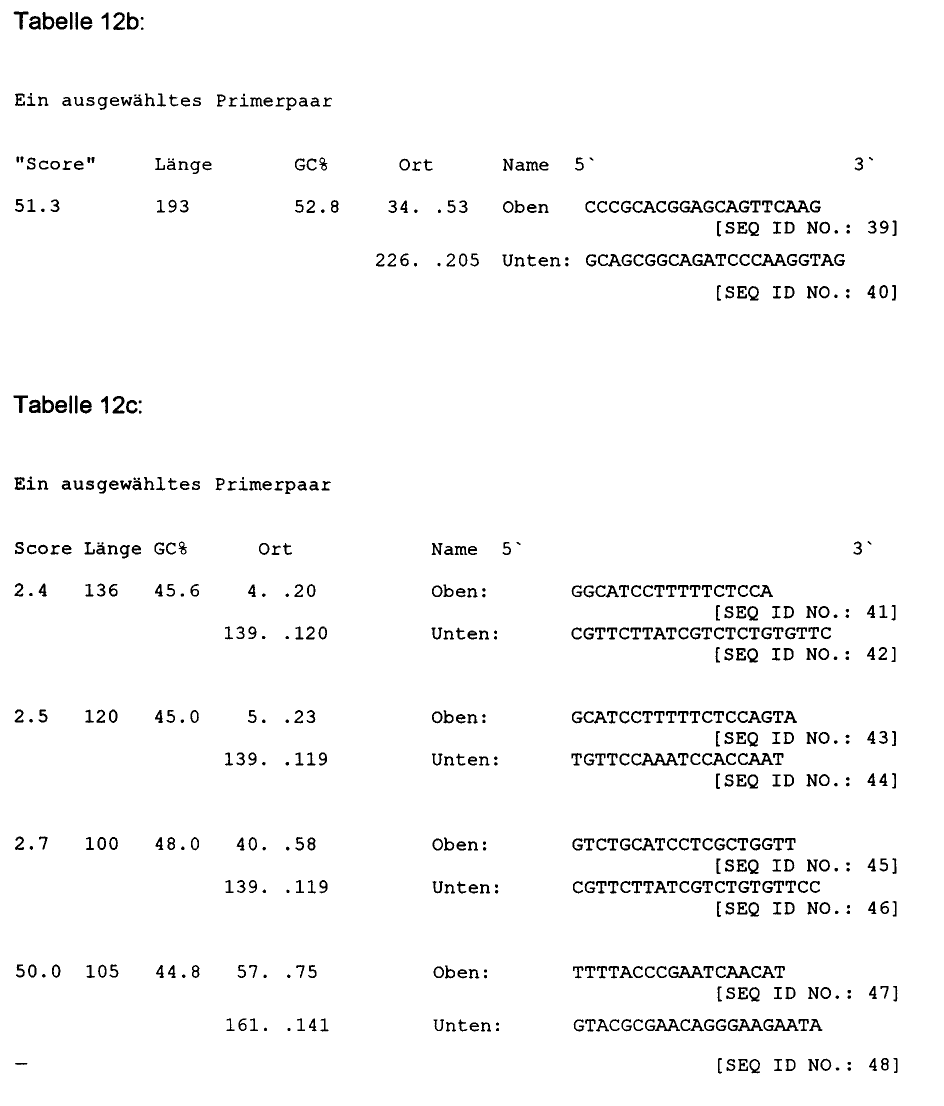

**Tabelle 13**

| Zusammenfassung der Wachstumsversuche | | | | |
|---|---|---|---|---|
| | Cyclin D1 | | Nup2 | |
| | nicht selektiv | selektiv | nicht selektiv | selektiv |
| wt p27 | +++ | +++ | +++ | +++ |
| Mut 106 | +++ | +++ | +++ | +++ |
| Mut 152 | +++ | + | +++ | + |
| Mut 294 | +++ | +++ | +++ | - |
| Mut 660 | +++ | +++ | +++ | - |
| Mut 687 | +++ | - | +++ | - |
| Mut 826 | +++ | +++ | +++ | +++ |
| Mut 850 | +++ | +++ | +++ | - |

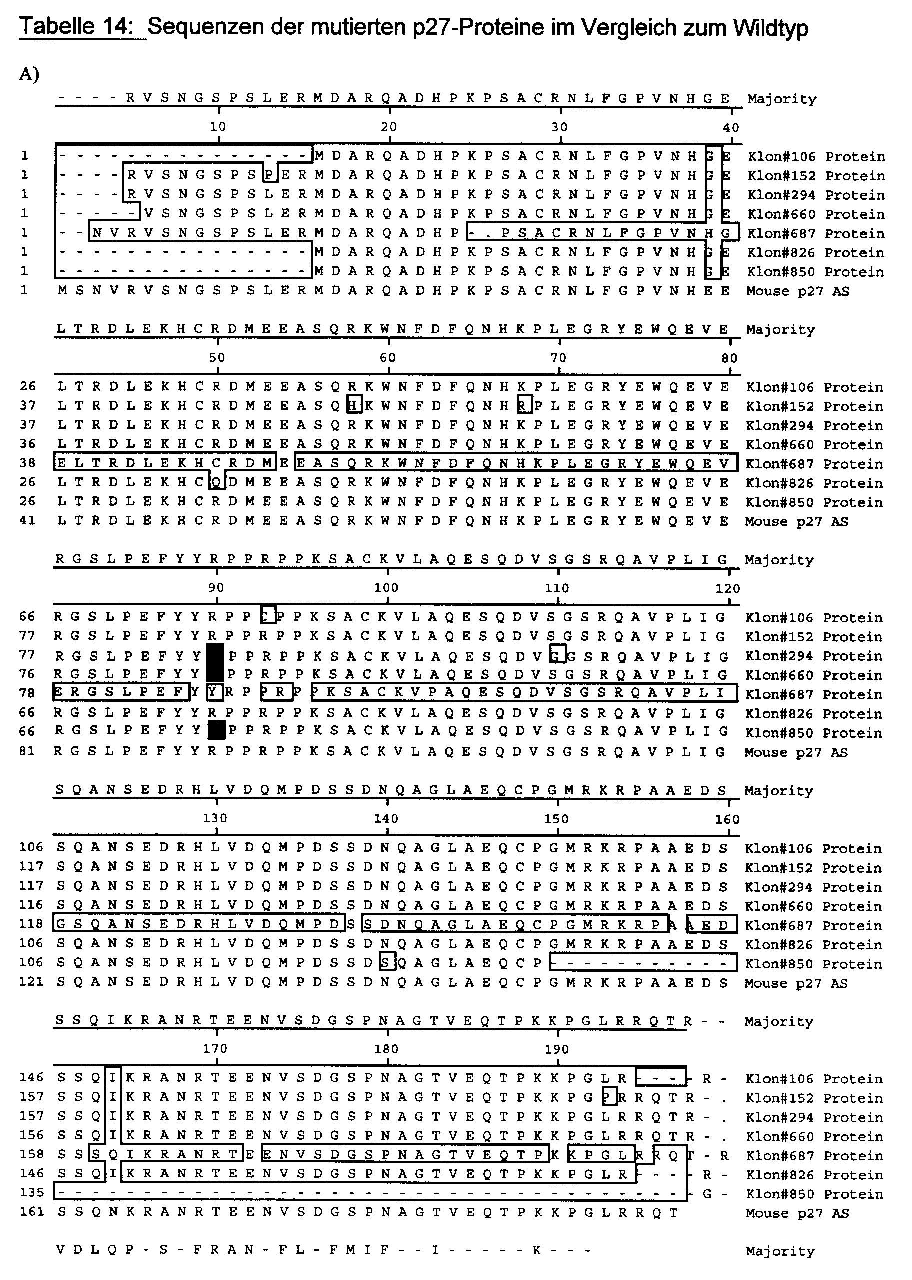

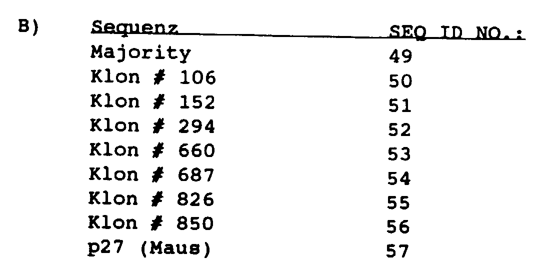

## Patentansprüche

1. Nukleinsäurekonstrukt enthaltend folgende Bestandteile:
Komponente a): eine Aktivierungssequenz für die Transkription der Komponente b)
Komponente b): ein Gen für einen Transkriptionsfaktor enthaltend
b₁) eine Aktivierungsdomäne
b₂) eine Bindesequenz für einen Inhibitor
b₃) eine DNA-Bindedomäne
Komponente c): eine Aktivierungsequenz, welche durch Bindung des Expressionsproduktes der Komponente b) aktiviert wird und die die Transkription der Komponente d) induziert
Komponente d): ein Effektorgen.

2. Nukleinsäurekonstrukt nach Anspruch 1) dadurch gekennzeichnet, daß die Komponente a) gleich ist der Komponente c).

3. Nukleinsäurekonstrukt nach Anspruch 1) oder 2) dadurch gekennzeichnet, daß die Komponente a) eine unspezifisch, zellspezifisch, metabolisch spezifisch, virusspezifisch und/oder zellzyklusspezifisch aktivierbare Promotorsequenz darstellt.

4. Nukleinsäurekonstrukt nach Anspruch 3) dadurch gekennzeichnet, daß die Komponente a) ausgewählt ist aus der Gruppe enthaltend
- Promotoren aktiviert in Endothelzellen, Peritonealzellen, Pleuralzellen, Epithelzellen der Haut, der Lunge, des Gastrointestinaltraktes, der Niere und der harnableitenden Wege, in Muskelzellen, in Bindegewebszellen, in blutbildenden Zellen in Makrophagen, in Lymphozyten, in Leukämiezellen, in Tumorzellen oder in Gliazellen,
- Promotorsequenzen von Viren, wie HBV, HCV, HSV, HPV, EBV, HTLV, CMV oder HIV
- Promotor- oder Enhancersequenzen aktiviert durch Hypoxie oder zellzyklusspezifische Aktivierungssequenzen der Gene für cdc25C, Cyclin A, cdc2, E2F-1, B-myb, DHFR und
- Bindesequenzen für zellproliferationsabhängig auftretende oder aktivierte Transkriptionsfaktoren wie Monomere oder Multimere der Myc E-Box.

5. Nukleinsäurekonstrukt nach einem der Ansprüche 1) bis 4) dadurch charakterisiert, daß die Aktivierungsdomäne [Komponente b1) der Komponente b)] ausgewählt ist aus den Aktivierungsdomänen der Transkriptionsfaktoren umfassend Oct-2, Sp1, NFY, ITF-2, VP-16, c-Myc und CTF.

6. Nukleinsäurekonstrukt nach einem der Ansprüche 1) bis 5) dadurch charakterisiert, daß die Bindesequenz für einen Inhibitor (b₂) ausgewählt ist aus einer Gruppe umfassend Proteine welche p27 inhibieren und hierdurch die durch das Protein p27 bewirkte Hemmung der Zellproliferation aufhebt.

7. Nukleinsäurekonstrukt nach einem der Ansprüche 1) bis 5) dadurch charakterisiert, daß die Bindesequenz für einen Inhibitor (b₂) die Aminosäuresequenz von p163 gemäß Tabelle 6 [SEQ ID NO.: 24] oder Teile davon enthält.

8. Nukleinsäurekonstrukt gemäß Anspruch 7, dadurch gekennzeichnet, daß die Teile der Aminosäuresequenz von p 163 ausgewählt werden aus der Gruppe enthaltend Peptide mit den Aminosäuresequenzen von Position 1 bis 24 [SEQ ID No.: 10], von Position 137 bis 196 [SEQ ID NO.: 12], von Position 215 bis 265 [SEQ ID NO.: 14], von Position 239 bis 272 [SEQ ID NO.: 16], von Position 399 bis 438 [SEQ ID NO.: 18] und von Position 307 bis 469 [SEQ ID NO.: 20] bezogen auf die Numerierung gemäß Tabelle 6.

9. Nukleinsäurekonstrukt nach einem der Ansprüche 1) bis 5) dadurch charakterisiert, daß die Bindesequenz für einen Inhibitor (b₂) durch Deletion funktioneller Domänen von dem Protein gemäß Anspruch 7 abgeleitet ist, wobei von der [SEQ ID NO. 24] der Tabelle 6 die p27-bindende Domäne oder die Ran-bindende Domäne deletiert ist.

10. Nukleinsäurekonstrukt gemäß Anspruch 9, dadurch gekennzeichnet, daß nach Deletion der p27-bindenden Domäne ein Protein der Aminosäuresequenz gemäß Tabelle 8 [SEQ ID NO.: 26] und bei Deletion der Ran-bindenden Domäne ein Protein der Aminosäuresequenz gemäß Tabelle 9 [SEQ ID NO.: 27] vorliegt.

11. Nukleinsäurekonstrukt nach einem der Ansprüche 1) bis 5) dadurch charakterisiert, daß die Bindesequenz für einen Inhibitor (b₂) durch Deletion aller Aminosäuresequenzen außer dem Bereich der p27-bindenden Domäne von dem Protein gemäß Anspruch 2 abgeleitet werden kann.

12. Nukleinsäurekonstrukt gemäß Anspruch 11, dadurch gekennzeichnet, daß die Bindesequenz für einen Inhibitor die Aminosäuresequenz gemäß Tabelle 10 [SEQ ID NO.: 28] enthält.

13. Nukleinsäurekonstrukt nach einem der Ansprüche 1) bis 5), dadurch charakterisiert, daß die Bindesequenz für einen Inhibitor (b₂) ausgewählt ist aus der Gruppe enthaltend das entsprechende homologe Protein des Inhibitors oder der entsprechenden Teile davon einer anderen Säugetierspezies oder des Menschen gemäß einem der Ansprüche 6 bis 12.

14. Nukleinsäurekonstrukt gemäß Anspruch 13, dadurch gekennzeichnet, daß die Aminosäuresequenz des Inhibitors das menschliche Homolog des p163 ist.

15. Nukleinsäurekonstrukt nach einem der Ansprüche 1) bis 14), dadurch charakterisiert, daß die Komponente c) mindestens eine DNA-(Binde)sequenz zur Bindung der Komponente b) enthält und durch diese Bindung aktiviert wird.

16. Nukleinsäurekonstrukt nach Anspruch 15), bei welchem die DNA-Bindesequenz ausgewählt ist aus der Gruppe enthaltend die Bindesequenz (5'-CGGACAACTGTTGACCCG-3', SEQ ID NO.: 1) für das Gal4-Protein; die Bindesequenz (5'-TACTGTATGTACATACAGTA-3', SEQ ID NO.: 2) für das LexA-Protein; die Bindesequenz (5'-GAATTGTGAGCGCGCACAATTC-3, SEQ ID NO.: 3) für das Lac I-Repressorprotein; die Bindesequenz (5'-TCGAGTTTACCACTCCC TATCAGTGATAGA-GAAAAGTGAAAG-3', SEQ ID NO.: 4) für das Tetracyclin-Repressorprotein und die Bindesequenz (5'-TAATGATGGGCG-3', SEQ ID NO.: 5) für das ZFHD-1 Protein.

17. Nukleinsäurekonstrukt nach einem der Ansprüche 1) bis 16) dadurch gekennzeichnet, daß es sich bei dem Effektorgen (Komponente d) um ein Gen handelt, das für einen Wirkstoff kodiert, der ausgewählt ist aus der Gruppe enthaltend Cytokine, Chemokine, Wachstumsfaktoren, Rezeptoren für Cytokine, Chemokine oder Wachstumsfaktoren, antiproliferativ oder zytostatisch oder apoptotisch wirkende Proteine, Antikörper, Antikörperfragmente, Angiogeneseinhibitoren, Peptidhormone, Gerinnungsfaktoren, Gerinnungshemmer, fibrinolytische Proteine, auf den Blutkreislauf wirksame Peptide oder Proteine, Blutplasmaproteine und Antigene von Infektionserregern oder von Zellen oder von Tumoren, wobei das ausgewählte Antigen eine Immunreaktion bewirkt.

18. Nukleinsäurekonstrukt nach einem der Ansprüche 1) bis 14), dadurch gekennzeichnet, daß es sich bei dem Effektorgen um ein Gen handelt, das für ein Enzym kodiert, welches eine Vorstufe eines Pharmakons in ein Pharmakon überführt.

19. Nukleinsäurekonstrukt nach einem der Ansprüche 1 bis 14), dadurch gekennzeichnet, daß es sich bei dem Effektorgen um ein Gen handelt, das für ein Ligand-Wirkstoff-Fusionsprotein oder ein Ligand-Enzym-Fusionsprotein kodiert, wobei der Ligand ausgewählt ist aus einer Gruppe umfassend Cytokine, Wachstumsfaktoren, Antikörper, Antikörperfragmente, Peptidhormone, Mediatoren und Zelladhäsionsmoleküle.

20. Nukleinsäurekonstrukt nach einem der vorhergehenden Ansprüche dadurch gekennzeichnet, daß es sich bei der Nukleinsäure um DNS handelt.

21. Nukleinsäurekonstrukt nach einem der vorhergehenden Ansprüche dadurch gekennzeichnet, daß das Nukleinsäurekonstrukt eingefügt ist in einen Vektor.

22. Nukleinsäurekonstrukt nach Anspruch 21) dadurch gekennzeichnet, daß es sich um einen Plasmidvektor handelt.

23. Nukleinsäurekonstrukt nach Anspruch 21) dadurch gekennzeichnet, daß es sich um einen viralen Vektor handelt.

24. Nukleinsäurekonstrukt nach einem der Ansprüche 1) bis 23) dadurch gekennzeichnet, daß es äußerlich, peroral, intravesikal, nasal, intrabronchial oder in den Magen-Darm-Trakt verabreicht oder in ein Organ, in eine Körperhöhle, in die Muskulatur, subkutan oder in den Blutkreislauf injiziert wird zur Prophylaxe oder Therapie einer Erkrankung.

25. Isolierte Zelle dadurch gekennzeichnet, daß sie ein Nukleinsäurekonstrukt gemäß einem der Ansprüche 1) bis 24) enthält.

26. Verwendung eines Nukleinsäurekonstruktes nach einem der Ansprüche 1) bis 24) oder einer Zelle nach Anspruch 25) zur Herstellung eines Heilmittels zur Behandlung einer Erkrankung ausgewählt aus der Gruppe enthaltend Infektionen, Tumore, Leukämien, Autoimmunerkrankungen, Allergien, Arthritiden, Entzündungen, Organabstoßungen, Transplantat gegen Wirt-Reaktionen, Blutgerinnungserkrankungen, Kreislauferkrankungen, Blutarmut, Hormonerkrankungen und ZNS-Schäden.

27. Verfahren zur Herstellung der Nukleinsäurekonstrukte nach einem der Ansprüche 1) bis 24), bei dem die einzelnen Elemente schrittweise zusammenligiert werden.

28. Verwendung einer Zelle nach Anspruch 25) zur Herstellung eines Heilmittels zur Therapie von Erkrankungen gemäß Anspruch 24) dadurch gekennzeichnet, daß mindestens eine Zelle äußerlich, intravesikal, nasal, intrabronchial, oral oder in den Magen-Darm-Trakt verabreicht oder in ein Organ, in eine Körperhöhle, in die Muskulatur, subkutan oder in den Blutkreislauf injiziert wird zur Prophylaxe oder Therapie einer Erkrankung.
